(19) Europäisches Patentamt
European Patent Office
Office européen des brevets

(11) **EP 4 606 822 A1**

(12) **EUROPEAN PATENT APPLICATION**
published in accordance with Art. 153(4) EPC

(43) Date of publication:
**27.08.2025 Bulletin 2025/35**

(21) Application number: **23879154.5**

(22) Date of filing: **18.10.2023**

(51) International Patent Classification (IPC):
***C07K 16/32*** *(2006.01)* ***C07K 16/30*** *(2006.01)*
***C07K 16/28*** *(2006.01)* ***A61K 47/68*** *(2017.01)*
***A61K 39/395*** *(2006.01)* ***A61P 35/00*** *(2006.01)*

(52) Cooperative Patent Classification (CPC):
**A61K 39/395; A61K 47/68; A61P 35/00; C07K 16/28; C07K 16/30; C07K 16/32**

(86) International application number:
**PCT/CN2023/125269**

(87) International publication number:
**WO 2024/083166 (25.04.2024 Gazette 2024/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **19.10.2022 PCT/CN2022/126269**

(71) Applicant: **MULTITUDE THERAPEUTICS INC.**
**Shanghai 200233 (CN)**

(72) Inventors:
- **MENG, Xun**
  **Shanghai 200233 (CN)**
- **WENG, Weining**
  **Shanghai 200233 (CN)**

(74) Representative: **Ostertag & Partner Patentanwälte mbB**
**Azenbergstraße 35**
**70174 Stuttgart (DE)**

Remarks:
The complete document including Reference Table(s) and the Sequence Listing(s) can be downloaded from the EPO website

# (54) ANTIBODY-DRUG CONJUGATE, PREPARATION METHOD THEREFOR AND USE THEREOF IN RESISTING TUMORS

(57) An antibody-drug conjugate, a preparation method therefor and the use thereof in resisting tumors, which specifically relates to an anti-HER3 antibody or an antigen-binding fragment thereof, and an antibody-drug conjugate linked to the antibody. The antibody-drug conjugate has a structure as shown in Ab-(L-D)n, and is covalently linked with an effective load with cytotoxicity via a linker. The anti-HER3 antibody or the antigen-binding fragment thereof and the antibody-drug conjugate have a use in the preparation of a therapeutic agent for diagnosing, preventing and treating tumor diseases.

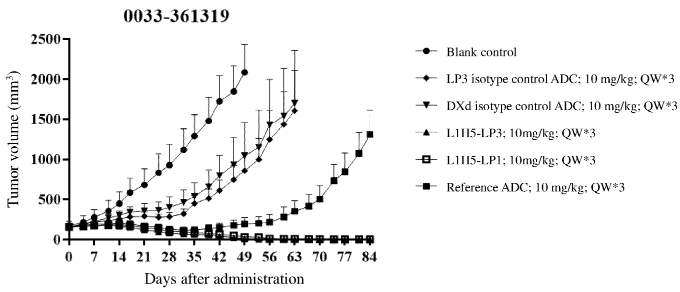


FIG. 21

Reference antibody

L1H5-LP3



EP 4 606 822 A1

## Description

### TECHNICAL FIELD

The present disclosure relates to an anti-HER3 antibody and an antibody-drug conjugate, which are linked to specifically targeted antibodies and anti-tumor drugs or toxins via linkers with improved hydrophobicity, exerting anti-tumor effects by targeting tumor cells expressing HER3.

### BACKGROUND

Antibody-drug conjugate (ADC) is a vectorized chemotherapy that selectively delivers potent cytotoxic drugs to tumor/cancer cells (Antibody-Drug Conjugates: The Last Decade, Nicolas Joubert et al., Pharmaceuticals (Basel) 2020 Sep 14; 13 (9): 245.). The marketed ADC drugs, Enhertu and Sacituzumab govitecan, have superior effects in the treatment of tumors, especially malignant tumors, both of which take as cytotoxic drugs DNA topoisomerase inhibitors camptothecin derivatives, which are more hydrophobic than tubulin inhibitors (e.g., MMAE and MMAF). Sacituzumab govitecan takes MCC-triazole spacer-PEG7-lysine-PABC as a linker, and decomposes in a lysosome of cells to release camptothecin SN38 (US 13/948,732); Enhertu developed by AstraZeneca and Daiichi Sankyo takes cathepsin B activated GGFG (amino acid sequence consisting of glycine-glycine-phenylalanine-glycine linked by peptide bonds) tetrapeptide as a linker and introduces a self-cleaving structure, releasing an Exatecan derivative Dxd (Yusuke Ogitani et al., Clin Cancer Res (2016) 22 (20): 5097-5108.). However, due to the physicochemical characteristics of the DNA topoisomerase inhibitor, such as rigid structure and poor hydrophilicity, the ADC molecules described above generate a large number of aggregates during preparation, directly affecting the safety and effectiveness of ADC therapy.

HER3 (NCBI, Gene ID: 2065) is a member of the HER (EGFR/ErbB) receptor family, and the family consists of four closely related type 1 transmembrane receptors (EGFR, HER2, HER3, and HER4). Monoclonal antibodies and small molecule inhibitors targeting the tyrosine kinase activity of EGFR and HER2 show clinical benefits in the treatment of several types of cancer, but their clinical efficacy is limited by drug resistance. HER3 is special in the EGFR family. It has little intracellular tyrosine kinase activity and is unable to form homodimers, so that it cannot function alone. Its kinase activity is dependent on the formation of heterodimers with other EGFR family members such as EGFR or HER2. When HER3 protein and HER2 form a heterodimer, its downstream pathway is activated, and inhibition of activation of HER3 downstream pathway can indirectly inhibit growth, proliferation and metastasis of cancer cells. HER3 therefore plays an important role in drug resistance to EGFR and HER2 targeted therapies (Clin Cancer Res; 20 (6) March 15, 2014).

Currently, Patritumab Deruxtecan (HER3-DXd, U3-1402) by Daiichi Sankyo is the only ADC drug targeting HER3 under development. The ADC structure is formed by splicing HER3 monoclonal antibody Patritumab and topoisomerase 1 inhibitor exatecan derivative (Deruxtecan) through a cleavable linker GGFG, which aims to meet the unmet clinical requirements of a wide group of EGFR-TKI-resistant and chemotherapy-failure non-small lung cancer patients.

Thus, there remains a need for new antibodies and antibody-drug conjugates that target HER3.

### SUMMARY

The present disclosure provides an anti-HER3 antibody or an antigen-binding fragment thereof comprising at least one VL and VH, wherein CDR1 of the VL comprises the amino acid sequence set forth in SEQ ID NO: 1, CDR2 of the VL comprises the amino acid sequence set forth in SEQ ID NO: 2, and CDR3 of the VL comprises the amino acid sequence set forth in SEQ ID NO: 3; CDR1 of the VH comprises the amino acid sequence set forth in SEQ ID NO: 4, CDR2 of the VH comprises an amino acid sequence having no more than 5, 4, 3, 2, 1, or 0 mutations compared to the amino acid sequence set forth in SEQ ID NO: 5, and CDR3 of the VH comprises the amino acid sequence set forth in SEQ ID NO: 6.

In some embodiments, in the anti-HER3 antibody or the antigen-binding fragment thereof, the CDR2 of the VH comprises one or more mutations selected from (a) and (b):

(a) C52N, T62N, T66K, and G67S;
(b) C52Y, T62N, T66K, and G67S.

In some embodiments, in the anti-HER3 antibody or the antigen-binding fragment thereof, the CDR2 of the VH comprises mutations of (a) or (b):

(a) C52N, T62N, T66K, and G67S;
(b) C52Y, T62N, T66K, and G67S.

The present disclosure provides a nucleic acid encoding the anti-HER3 antibody or the antigen-binding fragment

thereof described above.

The present disclosure provides a vector comprising a nucleic acid encoding the anti-HER3 antibody or the antigen-binding fragment thereof described above.

The present disclosure provides a host cell comprising the nucleic acid and/or vector described above.

The present disclosure provides an antibody-drug conjugate having the structure of formula I,

Ab-(L-D)n (I)

or an isomer, an isotopic variant, a pharmaceutically acceptable salt, a prodrug, or a solvate thereof, or a combination thereof,

wherein Ab is the aforementioned anti-HER3 antibody or the antigen-binding fragment thereof;

L is a linker covalently linked to Ab and D;

D is a payload;

n is a number between 1 and 10.

The present disclosure provides a preparation method for an antibody-drug conjugate, which comprises the following steps: reducing at least part of interchain disulfide bonds of the antibody or the antigen-binding fragment thereof through reduction treatment, and reacting with a reactive group of the linker in the linker-payload to obtain the antibody-drug conjugate having the structure of formula I,

Ab-(L-D)n (I)

or an isomer, an isotopic variant, a pharmaceutically acceptable salt, a prodrug, or a solvate thereof, or a combination thereof,

wherein Ab is the anti-HER3 antibody or the antigen-binding fragment thereof described above;

L is a linker covalently linked to Ab and D;

D is a payload;

n is a number between 1 and 10.

The present disclosure provides a pharmaceutical composition comprising the anti-HER3 antibody or the antigen-binding fragment thereof or the antibody-drug conjugate described above, or an isomer, an isotopic variant, a pharmaceutically acceptable salt, a prodrug, or a solvate thereof, or a combination thereof, and a pharmaceutically acceptable excipient.

The present disclosure provides a kit comprising the anti-HER3 antibody or the antigen-binding fragment thereof or the antibody-drug conjugate described above, or an isomer, an isotopic variant, a pharmaceutically acceptable salt, a prodrug, or a solvate thereof, or a combination thereof.

The present disclosure provides use of the anti-HER3 antibody or the antigen-binding fragment thereof described above, the antibody-drug conjugate, an antibody-drug conjugate prepared by the method described above, the pharmaceutical composition, or the kit described above in preparing a therapeutic agent for diagnosing, preventing and treating tumor diseases.

In some embodiments, the use comprises use in preparing a medicament targeting HER3.

In some embodiments, the tumor comprises a solid tumor expressing HER3.

The present disclosure provides a method for diagnosing, preventing and treating tumor diseases, which comprises administering to a subject a therapeutic dose of a therapeutic agent comprising the anti-HER3 antibody or the antigen-binding fragment thereof described above, the antibody-drug conjugate, an antibody-drug conjugate prepared by the method described above, and the pharmaceutical composition or the kit described above.

## BRIEF DESCRIPTION OF THE DRAWINGS

A and B in FIG. 1 respectively show chromatograms of size exclusion chromatography and hydrophobic chromatography of the L1H5 antibody (naked antibody) prepared in Example 4;

A and B in FIG. 2 respectively show chromatograms of size exclusion chromatography and hydrophobic chromatography detection of aggregates in the antibody-drug conjugate L1H5-LP3 prepared in Example 6;

A and B in FIG. 3 respectively show chromatograms of size exclusion chromatography and hydrophobic chromatography detection of aggregates in the antibody-drug conjugate L1H5-LP1 prepared in Example 7;

A and B in FIG. 4 respectively show chromatograms of size exclusion chromatography and hydrophobic chromatography detection of aggregates in the antibody-drug conjugate L1H5-LP2 prepared in Example 8;

FIG. 5 shows a statistical plot of flow cytometry of the Mu4O3 antibody prepared in Example 4 in cells expressing different amounts of HER3;

FIG. 6 shows immunofluorescence images of the Mu4O3 antibody prepared in Example 4 in HER3-GFP Tag overexpressing 293T cells;

FIG. 7 shows cell survival curves of the Mu4O3 antibody prepared in Example 4 in the assay of SW620 cell killing;

FIG. 8 shows the affinity curves of the L1H4, L1H5, L2H4, L2H5, L4H4, L4H5, and L4H7 antibodies prepared in Example 4 in the MDA-MB-453 cell line;

FIG. 9 shows the affinity curves of the L1H4, L1H5, L2H4, L2H5, L4H4, L4H5, and L4H7 antibodies prepared in Example 4 in the SW620 cell line;

FIG. 10 shows the cell proliferation inhibition rate of the L1H5 antibody, the reference antibody, and the blank control prepared in Example 4 in the SK-BR-3 cell line;

FIG. 11 shows curves of changes of tumor volumes over time of the L1H5, the reference antibody, and the blank control PBS prepared in Example 4 in a BT-474 breast cancer mouse model;

FIG. 12 shows cell survival curves of the antibody-drug conjugates L1H4-LP1, L1H5-LP1, and L4H4-LP1 prepared in Example 7 and the reference ADC (positive control) prepared in Comparative Example 1 in the SW620 cell line;

FIG. 13 shows cell survival curves in a killing assay for the SK-BR-3 cell line by the L1H5 antibody prepared in Example 4, the L1H5-LP3 prepared in Example 6, the reference antibody, and the reference ADC prepared in Comparative Example 1;

FIG. 14 shows cell survival curves in a killing assay for the BXPC-3 cell line by the L1H5 antibody prepared in Example 4, the L1H5-LP3 prepared in Example 6, the reference antibody, and the reference ADC prepared in Comparative Example 1;

FIG. 15 shows cell survival curves of the antibody-drug conjugate L1H5-LP1 prepared in Example 7, the L1H5-LP2 prepared in Example 8, and the reference ADC prepared in Comparative Example 1 in the HCT-15 cell line;

FIG. 16 shows curves of changes of tumor volumes over time of the antibody-drug conjugate L1H5-LP prepared in Example 7, the L1H5-LP2 prepared in Example 8, the reference ADC prepared in Comparative Example 1, and a blank control group in a HCT-15 colon cancer mouse model;

FIG. 17 shows curves of changes of tumor volumes over time after a mouse colon cancer PDX model is subjected to the treatment of the reference ADC prepared in Comparative Example 1, the L1H5-LP3 prepared in Example 6, the L1H5-LP1 prepared in Example 7, the Dxd isotype control ADC prepared in Comparative Example 4, and the LP3 isotype control ADC prepared in Comparative Example 5;

FIG. 18 shows curves of changes of tumor volumes over time after a mouse EGFR-TKI-resistant lung adenocarcinoma PDX model is subjected to the treatment of the reference ADC prepared in Comparative Example 1, the L1H5-LP3 prepared in Example 6, the L1H5-LP1 prepared in Example 7, the Dxd isotype control ADC prepared in Comparative Example 4, and the LP3 isotype control ADC prepared in Comparative Example 5;

FIG. 19 shows curves of changes of tumor volumes over time after a mouse colon cancer CDX model is subjected to the treatment of the L1H5-DXd prepared in Comparative Example 3, the reference antibody-LP3 prepared in Comparative Example 2, the L1H5-LP3 prepared in Example 6, and the reference ADC prepared in Comparative Example 1, and a negative control PBS group is set;

FIG. 20 is a partially enlarged view of FIG. 19 with the negative control PBS group taken away;

FIG. 21 shows curves of changes of tumor volumes over time after a mouse pancreatic cancer CDX model is subjected to the treatment of the reference ADC prepared in Comparative Example 1, the L1H5-LP3 prepared in Example 6, the L1H5-LP1 prepared in Example 7, the Dxd isotype control ADC prepared in Comparative Example 4, and the LP3 isotype control ADC prepared in Comparative Example 5;

FIG. 22 shows a comparison of hydrophobicity measurements for the L1H5 antibody prepared in Example 4, the antibody-drug conjugate L1H5-LP3 prepared in Example 6, the reference antibody, and the reference ADC prepared in Comparative Example 1;

A and B in FIG. 23 respectively show chromatograms of size exclusion chromatography and hydrophobic chromatography detection of aggregates in the reference ADC prepared in Comparative Example 1;

A and B in FIG. 24 respectively show chromatograms of size exclusion chromatography and hydrophobic chromatography detection of aggregates in the antibody-drug conjugate reference antibody-LP3 prepared in Comparative Example 2;

A and B in FIG. 25 respectively show chromatograms of size exclusion chromatography and hydrophobic chromatography detection of aggregates in the antibody-drug conjugate L1H5-DXd prepared in Comparative Example 3;

A and B in FIG. 26 respectively show chromatograms of size exclusion chromatography and hydrophobic chromatography detection of aggregates in the Dxd isotype control ADC prepared in Comparative Example 4;

A and B in FIG. 27 respectively show chromatograms of size exclusion chromatography and hydrophobic chromatography detection of aggregates in the LP3 isotype control ADC prepared in Comparative Example 5.

## DETAILED DESCRIPTION

Unless otherwise stated, all numbers expressing the content, concentration, ratio, mass, volume, time, temperature, thickness, technical effects, and the like used in the specification and claims are to be understood as being modified in any instances by the term "about" or "approximately". Accordingly, unless indicated to the contrary, the numerical parameters set forth in the following specification and appended claims are approximations. It should be understood by those skilled in the art that the numerical parameters can vary depending on the desired properties and effects sought and obtained by the present disclosure, and each numerical parameter should be interpreted according to the number of significant digits and conventional rounding rules or as understood by those skilled in the art.

Although the numerical ranges and parameters setting forth the broad scope of the present disclosure are approximations, the numerical values set forth in the specific examples are provided as precisely as possible. However, any numerical value inherently contains certain errors necessarily resulting from the standard deviation found in their respective tests or measurements. Each numerical range given in this specification will include each narrower numerical range that falls within this broader numerical range, as if such narrower numerical ranges are all explicitly written herein.

[Antibody]

The present disclosure provides an anti-HER3 antibody or an antigen-binding fragment thereof comprising at least one light chain variable region (VL) and one heavy chain variable region (VH), in which the VL and VH can be paired.

As used herein, the term "antibody" means any antigen-binding molecule or molecular complex comprising at least one complementarity determining region that specifically binds to or interacts with a particular antigen (e.g., HER3). The term "antibody" comprises an immunoglobulin molecule and a multimer thereof (e.g., IgM), and the immunoglobulin molecule comprises four polypeptide chains, i.e., two heavy (H) chains and two light (L) chains interconnected via disulfide bonds. Each heavy chain comprises a heavy chain variable region and a heavy chain constant region. The heavy chain constant region comprises three domains: CH1, CH2, and CH3. Each light chain comprises a light chain variable region and a light chain constant region. The light chain constant region comprises one domain (CL1). The VH and VL regions can be further subdivided into hypervariable regions, termed complementarity determining regions (CDRs), with more conserved regions termed framework regions (FRs) inserted therebetween. Each VH and VL is composed of three CDRs and four FRs arranged from the amino terminus to the carboxyl terminus in the following order: FR1, CDR1, FR2, CDR2, FR3, CDR3, and FR4.

As used herein, the term "antibody" also includes an antigen-binding fragment of an intact antibody molecule. The term "antigen-binding fragment" includes any natural, synthesized or genetically-engineered polypeptide or glycoprotein that can be obtained enzymatically and specifically binds to an antigen to form a complex. An antigen-binding fragment of an antibody can be obtained, for example, from an intact antibody molecule using any suitable standard technique. Non-limiting examples of antigen-binding fragments include: Fab fragments, $F(ab')_2$ fragments, Fd fragments, Fv fragments, single-chain Fv (scFv) molecules, dAb fragments, and minimal recognition units consisting of the amino acid residues that mimic the hypervariable region of an antibody (e.g., an isolated complementarity determining region (CDR) such as a CDR3 peptide), or a constrained FR3-CDR3-FR4 peptide. Other engineered molecules, such as domain-specific antibodies, single-domain antibodies, domain-deleted antibodies, chimeric antibodies, CDR-grafted antibodies, bifunctional antibodies, trifunctional antibodies, tetrafunctional antibodies, minibodies, and nanobodies (e.g., monovalent nanobodies, bivalent nanobodies, etc.), are also encompassed within the "antigen-binding fragment" as used herein. An antigen-binding fragment of an antibody will typically comprise at least one variable domain. The variable domain may be of any size or amino acid composition and will typically comprise at least one CDR that is adjacent to or in the frame of one or more framework sequences. In antigen-binding fragments having a VH domain associated with a VL domain, the VH and VL domains may be positioned relative to each other in any suitable configuration. For example, the variable region may be a dimer and contain a VH-VH, VH-VL, or VL-VL dimer. Alternatively, an antigen-binding fragment of an antibody may contain a monomer VH or VL domain.

In the anti-HER3 antibody or the antigen-binding fragment thereof, the CDR1-3 of the VL respectively comprise the amino acid sequences set forth in SEQ ID NOs: 1-3; the CDR1 of the VH comprises the amino acid sequence set forth in SEQ ID NO: 4; the CDR2 of the VH comprises an amino acid sequence having no more than 5, 4, 3, 2, 1, or 0 mutations compared to the amino acid sequence set forth in SEQ ID NO: 5; the CDR3 of the VH comprises the amino acid sequence set forth in SEQ ID NO: 6. The CDRs of the anti-HER3 antibody or the antigen-binding fragment thereof are defined and numbered using the Kabat system.

In some embodiments, the CDR2 of the VH comprises an amino acid sequence having no more than 4, 3, 2, 1, or 0 mutations compared to the amino acid sequence set forth in SEQ ID NO: 5. In some embodiments, the CDR2 of the VH comprises an amino acid sequence having no more than 4 mutations compared to the amino acid sequence set forth in SEQ ID NO: 5. In some embodiments, the CDR2 of the VH comprises the amino acid sequence set forth in SEQ ID NO: 5.

The CDR2 of the VH comprises a mutation selected from any one or a combination of C52N/Y, T62N, T66K, and

G67S. C52N/Y indicates that the 52th amino acid "C" is mutated into "N" or "Y". As used herein, "C", "N", "Y", "T", "K", "G", and "S" are standard amino acids expressed in one letter and respectively represent cysteine, asparagine, tyrosine, threonine, lysine, glycine, and serine. The 1st amino acid refers to the first amino acid from the amino terminus to the carboxyl terminus of VH, and so on.

In some embodiments, in the anti-HER3 antibody or the antigen-binding fragment thereof, the CDR2 of the VH comprises mutations C52N, T62N, T66K, and G67S. In some embodiments, in the anti-HER3 antibody or the antigen-binding fragment thereof, the CDR2 of the VH comprises mutations C52Y, T62N, T66K, and G67S.

Also included in the variable region of the anti-HER3 antibody or the antigen-binding fragment thereof is a framework region (FR), which is a region that has relatively little change in amino acid composition and arrangement order, other than the CDRs.

In some embodiments, there is no mutation in the framework region or a mutation that does not affect the binding of the antibody variable region to the antigen, which may increase the binding affinity of the antibody to the antigen, or remain substantially unchanged. In some embodiments, the anti-HER3 antibody or the antigen-binding fragment thereof further comprises a conservatively modified variant, including a single substitution, deletion, or addition to the polypeptide sequence, which results in the substitution of an amino acid with a chemically similar amino acid. Conservative replacement tables providing functionally similar amino acids are well known in the art. Such conservatively modified variants are in addition to, and do not exclude, polymorphic variants, interspecies homologs, and alleles. The following 8 groups comprise amino acids that are conservatively substituted with each other: 1) alanine (A) and glycine (G); 2) aspartic acid (D) and glutamic acid (E); 3) asparagine (N) and glutamine (Q); 4) arginine (R) and lysine (K); 5) isoleucine (I), leucine (L), methionine (M) and valine (V); 6) phenylalanine (F), tyrosine (Y), and tryptophan (W); 7) serine (S) and threonine (T); and 8) cysteine (C) and methionine (M) (see, for example, Creighton, Proteins (1984)). In some aspects, the term "conservative sequence modification" is used to refer to an amino acid modification that does not significantly affect or alter the binding characteristics of an antibody comprising the amino acid sequence.

In some embodiments, in the anti-HER3 antibody or the antigen-binding fragment thereof, the VL comprises an amino acid sequence having at least 70%, 75%, 77%, 78%, 80%, 82%, 84%, 85%, 90%, 95%, 96%, 97%, 98%, or 99% identity to the framework region sequences of the amino acid sequences set forth in SEQ ID NOs: 9, 11, 12, and 13; the VH comprises an amino acid sequence having at least 70%, 75%, 77%, 78%, 80%, 82%, 84%, 85%, 90%, 95%, 96%, 97%, 98%, or 99% identity to the framework region sequences of the amino acid sequences set forth in SEQ ID NOs: 10, 14, 15, and 16.

In some embodiments, in the anti-HER3 antibody or the antigen-binding fragment thereof, the VL comprises an amino acid sequence having at least 70%, 75%, 77%, 78%, 80%, 82%, 84%, 85%, 90%, 95%, 96%, 97%, 98%, or 99% identity to the amino acid sequences set forth in SEQ ID NOs: 9, 11, 12, and 13; the VH comprises an amino acid sequence having at least 70%, 75%, 77%, 78%, 80%, 82%, 84%, 85%, 90%, 95%, 96%, 97%, 98%, or 99% identity to the amino acid sequences set forth in SEQ ID NOs: 10, 14, 15, and 16.

In the present disclosure, the term "%identity" in two or more polypeptide sequences refers to the degree to which two or more sequences or subsequences are identical. Two sequences are "identical" if they have the same amino acid sequence over the regions being compared. If two sequences have a specified percentage of identical amino acid residues (e.g., 60% identity, optionally 65%, 70%, 75%, 80%, 85%, 90%, 95%, or 99% identity over a specified region, or, when not specified, over the entire sequence), when compared and aligned over a comparison window or specified region to obtain maximum correspondence, or the specified region as measured using one of the following sequence comparison algorithms or by manual alignment and visual inspection. Optionally, identity exists over a region of at least about 10 amino acids in length, or more preferably over a length of 20, 50, 200, or more amino acids. Two examples of algorithms suitable for determining percent sequence identity and sequence similarity are the BLAST and BLAST 2.0 algorithms (Altschul et al., Nuc. Acids Res. 25: 3389-3402, 1997, and Altschul et al., J. Mol. Biol. 215: 403-410, 1990).

In addition to the sequence identity percentages described above, another meaning that two polypeptides are substantially identical refers to a polypeptide that is immunologically cross-reactive with the second polypeptide, and thus, one polypeptide is typically substantially identical to the second polypeptide, e.g., where the two peptides differ only by conservative substitutions.

In some embodiments, in the anti-HER3 antibody or the antigen-binding fragment thereof, the VL comprises the amino acid sequence set forth in SEQ ID NO: 9, 11, 12, or 13, and the VH comprises the amino acid sequence set forth in SEQ ID NO: 10, 14, 15, or 16.

In some embodiments, in the anti-HER3 antibody or the antigen-binding fragment thereof, the VL and VH respectively comprise amino acid sequences having at least 85%, 90%, 95%, 96%, 97%, 98%, or 99% identity to the amino acid sequences set forth in SEQ ID NO: 9 and SEQ ID NO: 10; the VL and VH respectively comprise amino acid sequences having at least 85%, 90%, 95%, 96%, 97%, 98%, or 99% identity to the amino acid sequences set forth in SEQ ID NO: 11 and SEQ ID NO: 14; the VL and VH respectively comprise amino acid sequences having at least 85%, 90%, 95%, 96%, 97%, 98%, or 99% identity to the amino acid sequences set forth in SEQ ID NO: 11 and SEQ ID NO: 15; the VL and VH respectively comprise amino acid sequences having at least 85%, 90%, 95%, 96%, 97%, 98%, or 99% identity to the amino

acid sequences set forth in SEQ ID NO: 12 and SEQ ID NO: 14; the VL and VH respectively comprise amino acid sequences having at least 85%, 90%, 95%, 96%, 97%, 98%, or 99% identity to the amino acid sequences set forth in SEQ ID NO: 12 and SEQ ID NO: 15; the VL and VH respectively comprise amino acid sequences having at least 85%, 90%, 95%, 96%, 97%, 98%, or 99% identity to the amino acid sequences set forth in SEQ ID NO: 13 and SEQ ID NO: 14; the VL and VH respectively comprise amino acid sequences having at least 85%, 90%, 95%, 96%, 97%, 98%, or 99% identity to the amino acid sequences set forth in SEQ ID NO: 13 and SEQ ID NO: 15; or the VL and VH respectively comprise amino acid sequences having at least 85%, 90%, 95%, 96%, 97%, 98%, or 99% identity to the amino acid sequences set forth in SEQ ID NO: 13 and SEQ ID NO: 16.

In some embodiments, the anti-HER3 antibody or the antigen-binding fragment thereof comprises or has a VH and a VL respectively being an amino acid sequence or conservatively substituted variants thereof set forth in SEQ ID NO: 9 and 10, 11 and 14, 11 and 15, 11 and 16, 12 and 14, 12 and 15, 12 and 16, 13 and 14, 13 and 15, or 13 and 16; optionally, the framework regions have conservative substitutions.

In some embodiments, the anti-HER3 antibody or the antigen-binding fragment thereof comprises or has a VH and a VL respectively being an amino acid sequence or conservatively substituted variants thereof set forth in SEQ ID NO: 9 and 10, 11 and 14, 11 and 15, 12 and 14, 12 and 15, 13 and 14, 13 and 15, or 13 and 16; optionally, the framework regions have conservative substitutions.

The HER3 antibody or the antigen-binding fragment thereof provided by the present disclosure can bind to mammalian (e.g., human or murine) HER3 protein. In some embodiments, the anti-HER3 antibody or the antigen-binding fragment thereof specifically binds to human HER3. In some embodiments, the anti-HER3 antibody or the antigen-binding fragment thereof specifically binds to murine HER3.

The HER3 antibody or the antigen-binding fragment thereof can be of the IgM, IgG, IgA, IgD, or IgE class. In some embodiments, the HER3 antibody or the antigen-binding fragment thereof is an IgG molecule. In some embodiments, the HER3 antibody or the antigen-binding fragment thereof is an IgG1, IgG2, IgG3, or IgG4 subtype. In some embodiments, the HER3 antibody or the antigen-binding fragment thereof is human IgG1. The class and subclass of antibodies are identified according to methods common in the art, e.g., based on the differences in heavy chain $\alpha$, $\delta$, $\varepsilon$, $\gamma$, and $\mu$, and the constructed antibodies are referred to as IgA, IgD, IgE, IgG, and IgM. Among them, human $\gamma$ can be further subdivided into $\gamma$1, $\gamma$2, $\gamma$3, and $\gamma$4, which correspond to four subtypes, i.e., IgG1, IgG2, IgG3, and IgG4, respectively.

The HER3 antibody or the antigen-binding fragment thereof may be a human antibody, humanized antibody, or chimeric antibody defined using means commonly understood in the art. For example, a human antibody refers to an antibody that is encoded entirely by a human antibody gene; a humanized antibody refers to an antibody in which at least a portion of the constant region is encoded by a human gene; a chimeric antibody is expressed by transforming mammalian cells by inserting light and heavy chain variable region genes of a heterologous monoclonal antibody into an expression vector containing a human antibody constant region by using a DNA recombination technology.

The anti-HER3 antibody or the antigen-binding fragment thereof provided by the present disclosure has improved hydrophobicity, which is beneficial to improving stability *in vitro* and *in vivo.*

The anti-HER3 antibody or the antigen-binding fragment thereof provided by the present disclosure has good targeting property, and can anchor tumor cells expressing HER3 to play a role in tumor killing. Optionally, the anti-HER3 antibody or the antigen-binding fragment thereof can play an anti-tumor role *in vivo* and *in vitro.*

Also, the present disclosure provides a nucleic acid encoding the anti-HER3 antibody or the antigen-binding fragment thereof described above, a vector comprising a nucleic acid encoding the anti-HER3 antibody or the antigen-binding fragment thereof described above, and a host cell comprising the nucleic acid and/or the vector.

The anti-HER3 antibody or the antigen-binding fragment thereof can be obtained by immunizing an animal with HER3 or any polypeptide selected from the amino acid sequence of HER3 according to methods commonly practiced in the art, and collecting and purifying the antibodies produced *in vivo.* In this case, antibodies applicable to human diseases can be selected by investigating the cross-reactivity of the antibody binding to the obtained xenogenic HER3 with human HER3. Alternatively, monoclonal antibodies can be obtained from hybridomas established by fusing antibody-producing cells producing antibodies against HER3 with myeloma cells according to known methods (e.g., Kohler and Milstein, Nature (1975) 256, pages 495-497; Kennet, R., eds., Monoclonal Antibodies, pages 365-367, Plenum Press, N. Y. (1980); Goding J.W., Monoclonal Antibodies: Principles and Practice 3rd edition (1986) Academic Press, San Diego, CA). HER3 for use as an antigen can be obtained by expressing HER3 gene in a host cell using genetic engineering.

The chimeric antibody can be obtained by engineering a hybridoma, for example, a chimeric antibody in which a mouse-or rat-derived antibody variable region is linked to a human-derived constant region (see Proc. Natl. Acad. Sci. U.S.A., 81, 6851-6855, (1984)).

The humanized antibody can be produced by substituting most or all of the structural parts of a non-human monoclonal antibody with the corresponding human antibody sequence. For example, an antibody obtained by incorporating only complementarity determining regions (CDRs) into a human-derived antibody (see Nature (1986) 321, pages 522-525) and an antibody obtained by grafting amino acid residues of a part of the framework in addition to the sequences of the CDRs to a human antibody by the CDR-grafting method (WO 90/07861). The human antibody can be obtained by

using a method involving mice engineered to produce human antibodies, which carry human chromosomal fragments containing heavy chain and light chain genes of human antibodies (see Tomizuka, K. et al., Nature Genetics (1997) 16, pages 133-143; Kuroiwa, Y. et al., Nucl. Acids Res. (1998) 26, pages 3447-3448; Yoshida, H. et al., Animal Cell Technology: Basic and Applied Aspects, Vol. 10, pages 69-73 (Kitagawa, Y., Matsuda, T., and Iijima, S. eds.), Kluwer Academic Publishers, 1999; Tomizuka, K. eds., Proc. Natl. Acad. Sci. USA (2000) 97, pages 722-727, etc.). Methods for obtaining humanized antibodies also include those described, for example, in the following: Winter and Milstein, Nature, 1991, 349: 293-299; Rader et al., Proc. Nat. Acad. Sci. U.S.A., 1998, 95: 8910-8915; Steinberger et al., J. Biol. Chem., 2000, 275: 36073-36078; Queen et al., Proc. Natl. Acad. Sci. U.S.A., 1989, 86: 10029-10033;

[Antibody-drug conjugate]

The present disclosure provides an antibody-drug conjugate (ADC), which comprises an antibody-drug conjugate having the structure of formula I,

Ab-(L-D)n (I)

or an isomer, an isotopic variant, a pharmaceutically acceptable salt, a prodrug, or a solvate thereof, or a combination thereof,
wherein Ab is the anti-HER3 antibody or the antigen-binding fragment thereof described above;
L is a linker covalently linked to Ab and D;
D is a payload;
n is a number between 1 and 10.

The term "antibody-drug conjugate" or "ADC" refers to a conjugate of the anti-HER3 antibody or the antigen-binding fragment thereof described herein covalently linked to a payload. In general, the antibody-drug conjugate may comprise an antibody, a payload, and optionally a linker between the antibody and the payload. The ADC can provide a therapeutic effect by delivering a payload to HER3 cells, particularly HER3 tumor cells, targeted by the antibody. Antibody-drug conjugates may be prepared by various methods known in the art for preparing antibody-drug conjugates.

In ADC molecules, a linker, as a connecting structure for connecting an antibody and a payload, is a key factor for successfully constructing the ADC molecules. The molecular design and properties of a linker are key determinants of the efficacy of the ADC in terms of pharmacokinetics (PK)/pharmacodynamics (PD) and therapeutic window. For optimal efficacy, the ideal linker should have the following properties: (1) The linker is sufficiently stable in plasma that the ADC molecules can circulate in the bloodstream and localize to the tumor site without premature lysis. Unstable linkers may lead to premature release of the cytotoxic payload and damage non-target healthy cells, resulting in systemic toxicity and adverse reactions. (2) The linker needs to have the ability to be cleaved rapidly, releasing the free cytotoxic payload rapidly once the ADC is internalized into the target tumor cell. (3) Linker design also requires consideration of hydrophobicity. The binding of hydrophobic linkers to hydrophobic cytotoxic payloads generally promotes aggregation of the ADC molecule, which is not only detrimental to ADC efficacy, but may also lead to hepatotoxicity or elicit undesirable immune responses (Kyoji Tsuchikama et al., Antibody-drug conjugates: recent advances in conjugation and linker chemistrie, Protein Cell. 2018 Jan; 9(1): 33-46).

The term "isomer" refers to compounds with the same molecular formula but different structures, also known as isomers or structural isomers, which generally include constitutional isomers and stereoisomers. The constitutional isomers refer to isomers caused by different connection orders or different bonding properties of atoms in a molecule, and preferably include tautomers. The tautomer refers to functional isomers resulting from the rapid movement of an atom in a molecule between two positions. The stereoisomer refers to isomers caused by the same order of connection of atoms or groups of atoms and bonding substances in molecules but different spatial arrangement, and preferably include optical isomers. The optical isomer refers to stereoisomers having different optical rotation properties due to the absence of axial symmetry in the molecule, such as enantiomers, diastereomers, racemates, and mesomers.

The term "prodrug" refers to compounds that are chemically modified from a drug that are inactive or less active *in vitro* and that are converted *in vivo,* enzymatically or non-enzymatically, to release the active drug and to exert the efficacy. In the present disclosure, a prodrug may be an ADC molecule, but also a payload.

In some embodiments, the linker is a cleavable linker and a non-cleavable linker.

In some embodiments, the linker comprises a cleavable peptide; optionally, the cleavable peptide is cleavable by an enzyme. In some embodiments, the enzyme comprises cathepsin B.

In some embodiments, the cleavable peptide or L comprises an amino acid unit comprising a dipeptide, tripeptide, tetrapeptide, or pentapeptide.

In some embodiments, the amino acid unit is selected from: any or a combination of Val-Cit, Val-Ala, Glu-Val-Cit, Ala-Ala-Asn, Gly-Val-Cit, Gly-Gly-Gly, and Gly-Gly-Phe-Gly. Val represents valine, Cit represents citrulline, A1a represents alanine, Glu represents glutamic acid, Asn represents asparagine, Gly represents glycine, and Phe represents phenylalanine.

In some embodiments, the linker comprises a self-immolative spacer; optionally, the self-immolative spacer comprises p-aminobenzyloxycarbonyl (PABC) or *p*-aminobenzyl (PAB).

In some embodiments, the cleavable peptide is spliced directly to the self-immolative spacer.

In some embodiments, the linker comprises the structure of -$L_1$-$L_2$-$L_3$-, in which $L_1$ represents - (succinimid-3-yl-N)-$(CH_2)m^1$-C(=O)-, -$CH_2$-C(=O)-NH-$(CH_2)m^2$-C(=O)-, or -C(=O)-$(CH_2)m^3$-C(=O)-, wherein $m^1$ represents an integer of 2-8, $m^2$ represents an integer of 1-8, and $m^3$ represents an integer of 1-8; $L_2$ represents an amino acid unit; $L_3$ represents a self-immolative spacer.

In some embodiments, $m^1$ represents 2, 3, 4, 5, 6, 7, or 8. In some embodiments, $m^2$ represents 1, 2, 3, 4, 5, 6, 7, or 8. In some embodiments, $m^3$ represents 1, 2, 3, 4, 5, 6, 7, or 8.

In some embodiments, L is selected from one or more of the following groups:

- (succinimid-3-yl-N)-$CH_2CH_2$-C(=O)-GGFG-PABC-;
- (succinimid-3-yl-N)-$CH_2CH_2CH_2CH_2CH_2$-C(=O)-GGFG-PABC-;
- (succinimid-3-yl-N)-$CH_2CH_2CH_2CH_2CH_2$-C(=O)-GGFG-NH-PABC-;
- (succinimid-3-yl-N)-$CH_2CH_2$-C(=O)-NH-$CH_2CH_2O$-$CH_2CH_2O$-$CH_2CH_2$-C(=O)-GGFG-PABC-;
- (succinimid-3-yl-N)-$CH_2CH_2$-C(=O)-NH-$CH_2CH_20$-$CH_2CH_20$-$CH_2CH_20$-$CH_2CH_2O$-$CH_2CH_2$-C(=O)-GGFG-PABC-;
- $CH_2$-C(=O)-NH-$CH_2CH_2$-C(=O)-GGFG-PABC-;
- C(=O)-$CH_2CH_2CH_2CH_2CH_2CH_2$-C(=O)-GGFG-PABC-;
- (succinimid-3-yl-N)-$CH_2CH_2$-C(=O)-GGFG-NH-$CH_2CH_2$-C(=O)-;
- (succinimid-3-yl-N)-$CH_2CH_2$-C(=O)-GGFG-NH-$CH_2CH_2CH_2$-C(=O)-;
- (succinimid-3-yl-N)-$CH_2CH_2CH_2CH_2CH_2$-C(=O)-GGFG-NH-$CH_2CH_2$-C(=O)-;
- (succinimid-3-yl-N)-$CH_2CH_2CH_2CH_2CH_2$-C(=O)-GGFG-NH-$CH_2CH_2CH_2$-C(=O)-;
- (succinimid-3-yl-N)-$CH_2CH_2CH_2CH_2CH_2$-C(=O)-GGFG-NH-$CH_2CH_2CH_2CH_2CH_2$-C(=O)-;
- (succinimid-3-yl-N)-$CH_2CH_2CH_2CH_2CH_2$-C(=O)-GGFG-NH-$CH_2$-O-$CH_2$-C(=O)-;
- (succinimid-3-yl-N)-$CH_2CH_2CH_2CH_2CH_2$-C(=O)-GGFG-NH-$CH_2CH_2$-O-$CH_2$-C(=O)-;
- (succinimid-3-yl-N)-$CH_2CH_2$-C(=O)-NH-$CH_2CH_20$-$CH_2CH_20$-$CH_2CH_2$-C(=O)-GGFG-NH-$CH_2CH_2CH_2$-C(=O)-;
- (succinimid-3-yl-N)-$CH_2CH_2$-C(=O)-NH-$CH_2CH_2O$-$CH_2CH_2O$-$CH_2CH_2$-C(=O)-GGFG-NH-$CH_2CH_2$-C(=O)-;
- (succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-GGFG-NH-$CH_2CH_2CH_2$-C(=O)-;
- (succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-GGFG-NH-$CH_2CH_2$-C(=O)-;
- $CH_2$-C(=O)-NH-$CH_2CH_2$-C(=O)-GGFG-NH-$CH_2CH_2CH_2$-C(=O)-;
- C(=O)-$CH_2CH_2CH_2CH_2CH_2CH_2$-C(=O)-GGFG-NH-$CH_2CH_2CH_2$-C(=O)-;
- (succinimid-3-yl-N)-$CH_2CH_2$-C(=O)-VA-PABC-;
- (succinimid-3-yl-N)-CH2CH2CH2CH2CH2-C(=O)-VA-PABC-;
- (succinimid-3-yl-N)-$CH_2CH_2CH_2CH_2CH_2$-C(=O)-VA-NH-PABC-;
- (succinimid-3-yl-N)-$CH_2CH_2$-C(=O)-NH-$CH_2CH_2O$-$CH_2CH_2O$-$CH_2CH_2$-C(=O)-VA-PABC-;
- (succinimid-3-yl-N)-CH2CH2-C(=O)-NH-$CH_2CH_2O$-$CH_2CH_2O$-$CH_2CH_2O$-$CH_2CH_2O$-$CH_2CH_2$-C(=O)-VA-PABC-;
- $CH_2$-C(=O)-NH-$CH_2CH_2$-C(=O)-VA-PABC-;
- C(=O)-$CH_2CH_2CH_2CH_2CH_2CH_2$-C(=O)-VA-PABC-;
- (succinimid-3-yl-N)-$CH_2CH_2$-C(=O)-VA-NH-$CH_2CH_2$-C(=O)-;
- (succinimid-3-yl-N)-$CH_2CH_2$-C(=O)-VA-NH-$CH_2CH_2CH_2$-C(=O)-;
- (succinimid-3-yl-N)-$CH_2CH_2CH_2CH_2CH_2$-C(=O)-VA-NH-$CH_2CH_2$-C(=O)-;
- (succinimid-3-yl-N)-$CH_2CH_2CH_2CH_2CH_2$-C(=O)-VA-NH-$CH_2CH_2CH_2$-C(=O)-;
- (succinimid-3-yl-N)-$CH_2CH_2CH_2CH_2CH_2$-C(=O)-VA-NH-$CH_2CH_2CH_2CH_2CH_2$-C(=O)-;
- (succinimid-3-yl-N)-$CH_2CH_2CH_2CH_2CH_2$-C(=O)-VA-NH-$CH_2$-O-$CH_2$-C(=O)-;
- (succinimid-3-yl-N)-$CH_2CH_2CH_2CH_2CH_2$-C(=O)-VA-NH-$CH_2CH_2$-O-$CH_2$-C(=O)-;
- (succinimid-3-yl-N)-$CH_2CH_2$-C(=O)-NH-$CH_2CH_2O$-$CH_2OH_2O$-$CH_2CH_2$-C(=O)-VA-NH-$CH_2CH_2CH_2$-C(=O)-;
- (succinimid-3-yl-N)-$CH_2CH_2$-C(=O)-NH-$CH_2CH_2O$-$CH_2CH_2O$-$CH_2CH_2$-C(=O)-VA-NH-$CH_2CH_2$-C(=O)-;
- (succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-VA-NH-$CH_2CH_2CH_2$-C(=O)-;
- (succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-VA-NH-$CH_2CH_2$-C(=O)-;
- $CH_2$-C(=O)-NH-$CH_2CH_2$-C(=O)-VA-NH-$CH_2CH_2CH_2$-C(=O)-; and
- C(=O)-$CH_2CH_2CH_2CH_2CH_2CH_2$-C(=O)-VA-NH-$CH_2CH_2CH_2$-C(=O)-.

In some embodiments, the p-aminobenzyloxycarbonyl (PABC) or *p*-aminobenzyl (PAB) is linked to a poly-sarcosine (poly-N-methylglycine) residue or a methylamino group.

In some embodiments, the antibody-drug conjugate comprises an antibody, a payload, and a linker of formula II,

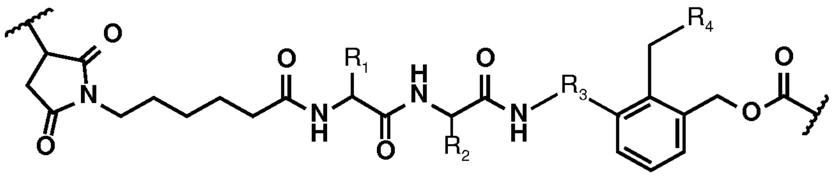

formula II

wherein the linker of formula II is covalently bonded with a sulfhydryl part reduced from an interchain disulfide chain of the antibody through a thioether bond via a succinimidyl group;

the carbonyl in the ester group of the linker of formula II is linked to the amino group in the payload;

in formula II, $R_1$ and $R_2$ are independently selected from hydrogen, methyl, and isopropyl groups;

$R_3$ represents $-(CR_5HCONH)n^1-(CH_2CONH)n^2-$ or a single bond, $R_5$ is hydrogen or benzyl, $n^1$ represents an integer of 0-2, and $n^2$ represents an integer of 0-2;

$R_4$ represents a methylamino group or $-(NCH_3COCH_2)n^3-NCH_3COCH_3$, and $n^3$ represents an integer of 1-20.

The term "payload" includes compounds that are cytotoxic or capable of killing cells upon release from an antibody-drug conjugate, compounds labeled with a radioactive marker, fluorophore, chromophore, imaging agent, and/or metal ion for use as a detection marker or in cell-killing applications, radionuclides or polypeptides, and compounds, nucleic acids, polypeptides or proteins, enzymes, hormones, or nucleic acids that can modulate immune activity (including activation or inhibition) of the body.

In some ideal cases, the payload of the conjugated form in the antibody-drug conjugate is either almost non-cytotoxic or cytotoxic to as little as an effective therapeutic dose of the ADC administered without inducing a systemic toxic response in the subject due to the payload of the conjugated form. The payload may be a drug that has been clinically validated for the treatment of a particular disease, or a compound, radionuclide, nucleic acid, protein, or polypeptide that has an acceptable pharmacological activity under conditions of clinical use.

In the present disclosure, in formula I, the linker is connected with the antibody through a thioether bond formed from sulfhydryl reduced from an interchain disulfide chain of the antibody via a succinimidyl group at the end. The succinimidyl group is

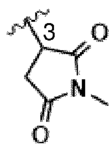

,

which forms a thioether bond through the carbon atom at the 3-position and a sulfhydryl group reduced from an interchain disulfide chain of the antibody. In the present disclosure, the bond with 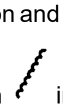 in the structural formula represents a chemical bond connected to other groups.

In the present disclosure, the disulfide bond of the antibody includes an interchain disulfide bond and an intrachain disulfide bond; preferably, the interchain disulfide bond is bonded to a linker after being treated, for example, activated (or reduced) to a sulfhydryl group. The amino acids of the antibody that chemically bond to the succinimidyl group of the linker include one or a combination of lysine, histidine, tyrosine, and cysteine, preferably through cysteine.

In some embodiments, the linker in formula I may be linked to the hinge region, variable region and/or constant region of the antibody.

In some embodiments of the present disclosure, in the linker of formula II, $R_4$ represents - $(NCH_3COCH_2)n^3-NCH_3COCH_3$, and $n^3$ represents an integer of 1-20. $n^3$ may be selected from, for example, any integer of 1, 2, 3, 4, 5, 6, 7, 8, 9, 10, 11, 12, 13, 14, 15, 16, 17, 18, 19, and 20.

In some embodiments, in the linker of formula II, $R_4$ represents $-(NCH_3COCH_2)n^3-NCH_3COCH_3$, and $n^3$

represents an integer of 8-15. In some embodiments, in the antibody-drug conjugate, in the linker of formula II, $R_4$ represents $-(NCH_3COCH_2)n^3-NCH_3COCH_3$, and $n^3$ represents an integer of 10-12.

In some embodiments, in the antibody-drug conjugate, in the linker of formula II, $R_4$ represents a methylamino group.

In the linker of formula II of the present disclosure, $R_4$ comprises a hydrophilic poly-sarcosine group or a methylamino group, so that the hydrophilicity of the antibody-drug conjugate is increased, and particularly when the hydrophobic payload is conjugated in the antibody-drug conjugate, the improvement of the hydrophilicity of ADC molecules is beneficial to reducing the aggregation of the ADC molecules in the preparation process, thereby improving the stability, uniformity, and purity of the antibody-drug conjugate.

In some embodiments, in the antibody-drug conjugate, in the linker of formula II, $R_3$ represents a single bond.

In some embodiments, in the antibody-drug conjugate, in the linker of formula II, $R_3$ represents $-(CR_5HCONH)n^1-(CH_2CONH)n^{2-}$, $R_5$ is benzyl, $n^1$ represents an integer of 1-2, and $n^2$ represents an integer of 1-2.

In some embodiments, in the antibody-drug conjugate, in the linker of formula II, $R_3$ represents $-CR_5HCONH-$, $-CH_2CONH-$, or $-CRsHCONH-CH_2CONH-$; $-(CRsHCONH)_2-CH_2CONH-$;$-CR_5HCONH-(CH_2CONH)_2-$; or $-(CRsHCONH)_2-(CH_2CONH)_2-$; $R_5$ is benzyl.

In some embodiments, in the antibody-drug conjugate, in the linker of formula II, $R_1$ is hydrogen. In some embodiments, in the linker of formula II, $R_1$ is isopropyl.

In some embodiments, in the antibody-drug conjugate, in the linker of formula II, $R_2$ is hydrogen. In some embodiments, in the linker of formula II, $R_2$ is methyl.

In some embodiments, the linker in the antibody-drug conjugate is selected from one or more of the following groups,

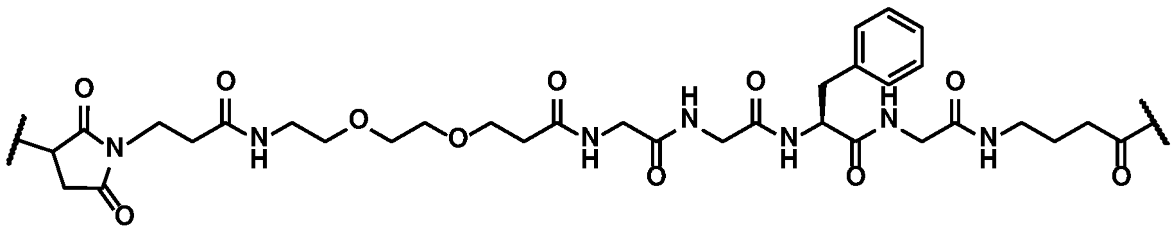
;

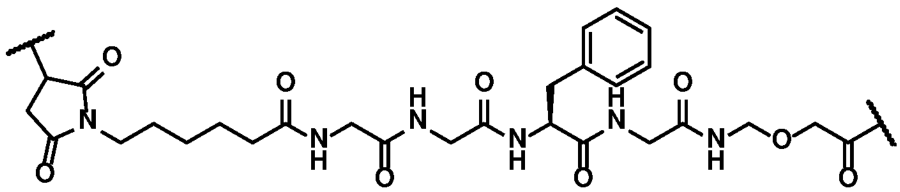
;

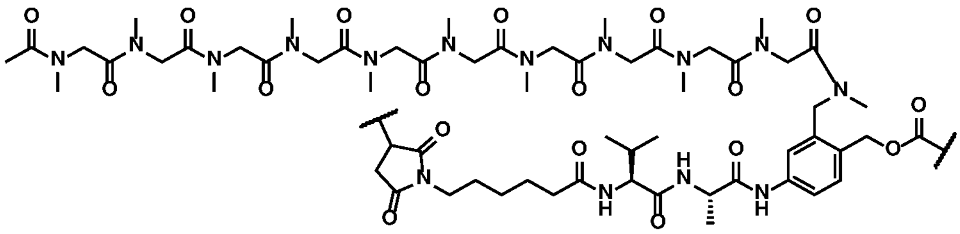
;

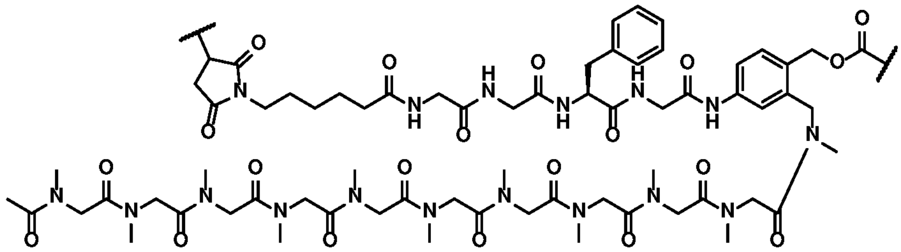
;

and

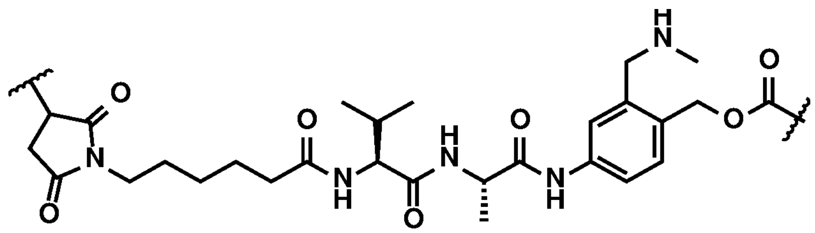

.

In some embodiments, the payload in the antibody-drug conjugate is a marker containing a radioactive marker, fluorophore, chromophore, imaging agent, and/or metal ion as a detection marker, including, but not limited to, a chemically synthesized organic compound, radionuclide, metal complex, or polypeptide. The radioactive marker refers to a labeled compound which is capable of being identified and used as a tracer by replacing one or more atoms of a compound molecule with radioactive nuclides, including amino acids, polypeptides, proteins, saccharides, nucleotides, nucleosides, purines, pyrimidines, steroids, lipids, and tumor antigens, hormones, receptors, vitamins, and medicaments for medical research, and the like. Radioactive nuclides are typically nuclides that spontaneously emit radiation, including but not limited to tritium, iodine 125, iodine 131, sulfur 35, phosphorus 32, and carbon 14. Fluorophores are typically groups comprising conjugated double bonds that fluoresce when the molecule returns from an excited state to a ground state. Chromophore refers to unsaturated groups and their associated chemical bonds contained in a molecule that can absorb light radiation and undergo electronic transitions. Imaging agents generally refer to radiopharmaceuticals that, when introduced into the body, can be used to image organs, tissues or molecules in nuclear medicine.

In some embodiments, the payload in the antibody-drug conjugate is a nucleic acid, which may be a ribonucleic acid and/or a deoxyribonucleic acid.

In some embodiments, the payload in the antibody-drug conjugate is a hormone, a growth factor, a coagulation factor, a plasminase (e.g., a prodrug-converting enzyme capable of converting a prodrug to an active drug, and a ribonuclease).

In some embodiments, the payload in the antibody-drug conjugate is an immunomodulator (including cytokines and chemokines with immune affecting effects), an agonistic antibody with biological activity, or an antagonistic antibody.

In some embodiments, the payload in the antibody-drug conjugate is a cytotoxic compound. In some embodiments, the payload in the antibody-drug conjugate has anti-tumor activity or is an anti-tumor drug, and the payload is selected from a DNA topoisomerase inhibitor or selected from a tubulin inhibitor. The DNA topoisomerase inhibitor may be a topoisomerase I inhibitor or a topoisomerase II inhibitor.

In the present disclosure, the term "topoisomerase inhibitor" generally refers to compounds that inhibit the activity of topoisomerases. Compounds that are inhibitors of topoisomerase I are active on topoisomerase I; a topoisomerase II inhibitor has activity on topoisomerase II; some compounds are active on both topoisomerase I and topoisomerase II and are known as topoisomerase I/II inhibitors.

The term "tubulin inhibitor" generally refers to compounds that inhibit the microtubule system of eukaryotic cells, interfere with cell division, and inhibit cell proliferation.

In some embodiments, the payload is camptothecin or a derivative thereof having a topoisomerase inhibition effect. The term "derivative": a compound formed by substituting atoms or atom groups in the molecule of the parent compound with other atoms or atom groups is referred to as a derivative of the parent compound. The term "camptothecin and a derivative thereof" generally include camptothecin and camptothecin derivatives. Camptothecin exerts its pharmacological effects by irreversibly inhibiting topoisomerase I. The camptothecin derivative includes exatecan, irinotecan, topotecan, lurtotecan, silatecan, etirinotecan pegol, TAS 103, 9-aminocamptothecin, 7-ethylcamptothecin, 10-hydroxycamptothecin, 9-nitrocamptothecin, 10,11-methylenedioxycamptothecin, 9-amino-10,11-methylenedioxycamptothecin, 9-chloro-10,11-methylenedioxycamptothecin, (7-(4-methylpiperazinomethylene)-10,11-ethylenedioxy-20(S)-camptothecin), (7-(4-methylpiperazinomethylene)-10,11-methylenedioxy-20(S)-camptothecin), 7-(2-(N-isopropylamino) ethyl)-(20S)-camptothecin and the like and stereoisomers, salts, and esters thereof. Methods for the synthesis of camptothecin and camptothecin analogs or derivatives are known and are summarized and described in U.S. Pat. No. 5,244,903, which is incorporated herein by reference in its entirety.

In some embodiments, the payload is auristatin or a derivative thereof, or maytansine or a derivative thereof having a tubulin inhibition effect. The term "auristatin and a derivative thereof" generally include auristatin F and a derivative of auristatin F including monomethyl auristatin E (MMAE) and monomethyl auristatin F (MMAF). The term "maytansine and a derivative thereof" generally include maytansine and a derivative of maytansine including maytansine DM1, maytansine DM2, and maytansine DM4.

In some embodiments, the payload is the camptothecin derivative, exatecan, which is a topoisomerase inhibitor that acts on the entire cell cycle with strong penetration and good therapeutic effect on slow growing solid tumors. Moreover, the number of targets in the cell is far lower than that of targets of the tubulin inhibitor, and the ADC molecule can

play a better killing effect when carrying the same number of payloads to enter the cell. The exatecan molecule is not a substrate of P-gp and is beneficial to reducing or alleviating the problem of drug resistance.

In some embodiments, the payload is exatecan of formula III, which is linked to the linker through the nitrogen atom of the amino group on its cyclohexane ring,

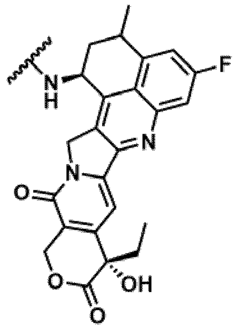

formula III.

The structure of the exatecan molecule is rigid and poorly hydrophilic. When conjugated with the GGFG tetrapeptide linker commonly used in the prior art to prepare ADCs, it tends to cause aggregation between ADC molecules, which does not meet the development requirements for ADC drugs (Bioorg. Med. Chem. Lett. 26 (2016) 1542-1545). Therefore, the selection and compatibility of the linker and payload have an impact on the safety and stability of ADC drugs.

Without being bound by any theory, the antibody-drug conjugates provided in the present disclosure improve the hydrophilicity of the linker-payload structure due to the presence of multiple hydrophilic groups in the linker. This improvement can, to some extent, reduce phenomena such as aggregation and precipitation of ADC molecules caused by hydrophobic payloads.

After endocytosis of the ADC molecule into the cell, depending on whether the linker is degraded, the payload or a compound of the linker (or part of the linker)-payload structure is released. In some embodiments, the amino group on the cyclohexane ring of exatecan of formula III and the carbonyl group in the ester group of the linker of formula II form a linker-payload structure containing carbamate. Without being bound by any theory, in the linker-payload structure provided in the present disclosure, after endocytosis of the ADC molecule into the cell, the linker is enzymatically cleaved by cathepsin (e.g., Cathepsin B), forming an intermediate or active metabolite of formula V,

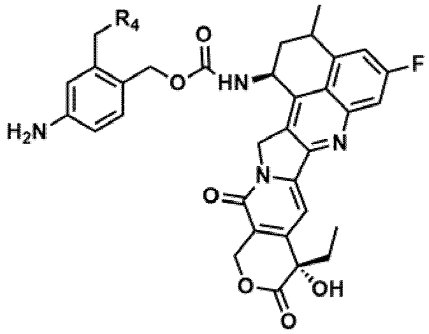

formula V.

$R_4$ represents a methylamino group or $-(NCH_3COCH_2)n^3-NCH_3COCH_3$, and $n^3$ represents an integer of 1-20.

The PABC group in the intermediate or active metabolite of formula V is then 1,6-eliminated to release exatecan. The mechanism of 1,6-elimination of PABC is described in detail in the literature Angew. Chem. Int. Ed. 2015, 54, 7492-7509. Therefore, the linker-payload structures in the ADC molecules provided by the present disclosure have good *in vivo* stability and bioactivity.

Without being bound by any theory, the enzyme cutting site in the linker-payload structure may be an amide bond in the linker, for example, the amide bond between the carbon atom where the substituent represented by $R_2$ is located and the group represented by $R_3$, or the amide bond in the group represented by $R_3$.

In some embodiments, in the antibody-drug conjugate, n is the ratio of the number of conjugated payload molecules to each molecule of antibody (drug to antibody ratio, DAR). In some embodiments, in the antibody-drug conjugate, n is 1-10, 1-2, 2-4, 4-6, 2-8, 4-8, 4-10, 6-10, 7-10, or 8-10, and exemplary DAR values are 4, 6, 7.78, 8.038, or 9.92.

DAR represents the average number of conjugated payload or drug molecules per antibody molecule, i.e., the average number of conjugated drug molecules. In the antibody-drug conjugate, the number of conjugated payload

molecules per antibody molecule is a key factor affecting their efficacy and safety. The production of antibody-drug conjugates is carried out by specifying reaction conditions, such as the amounts of starting materials and reagents used in the reaction, in order to achieve a constant number of conjugated payload molecules. Typically, the preparation of antibody-drug conjugates results in a mixture containing different numbers of conjugated payload molecules. Unless otherwise indicated, the number of conjugated payload or drug molecule per antibody molecule is defined in the present disclosure as the average value, i.e., the average number of conjugated payloads or drug molecules.

In some embodiments, the antibody-drug conjugate comprises any one having the following structures:

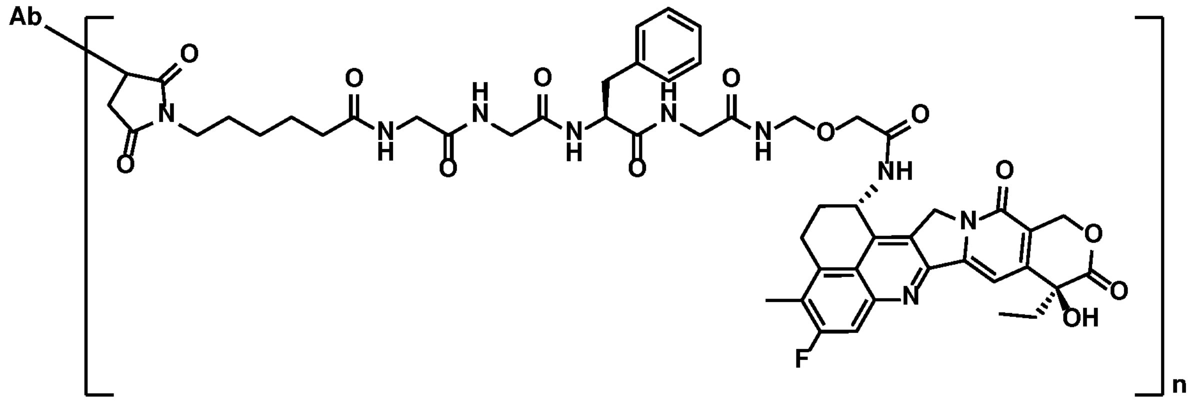

,

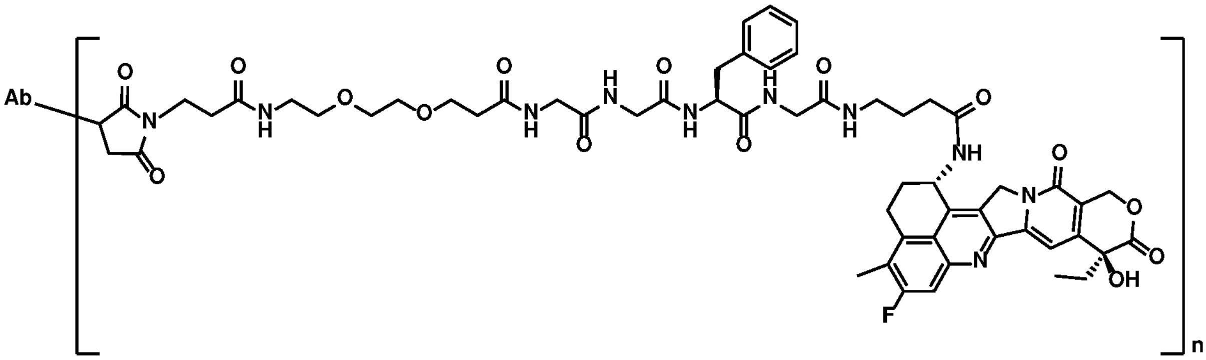

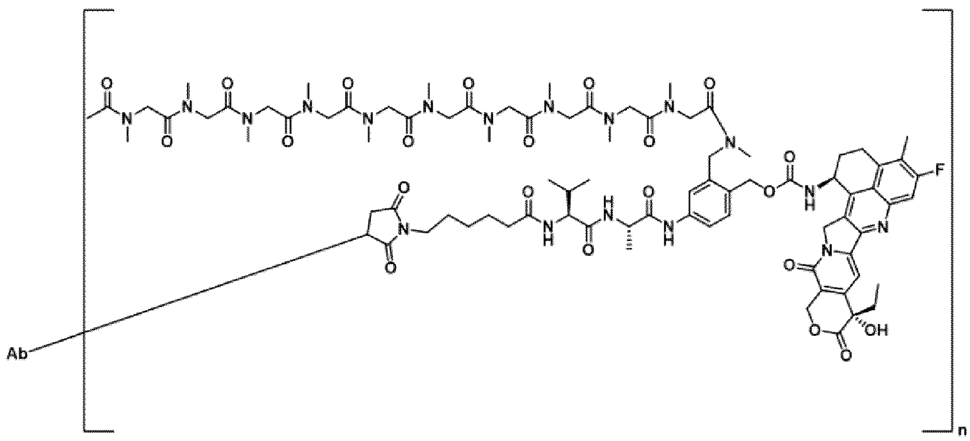

;

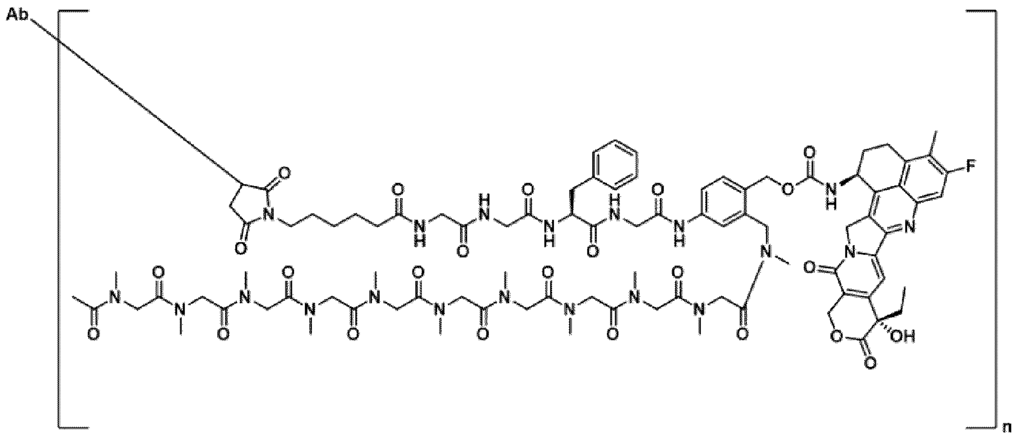

and

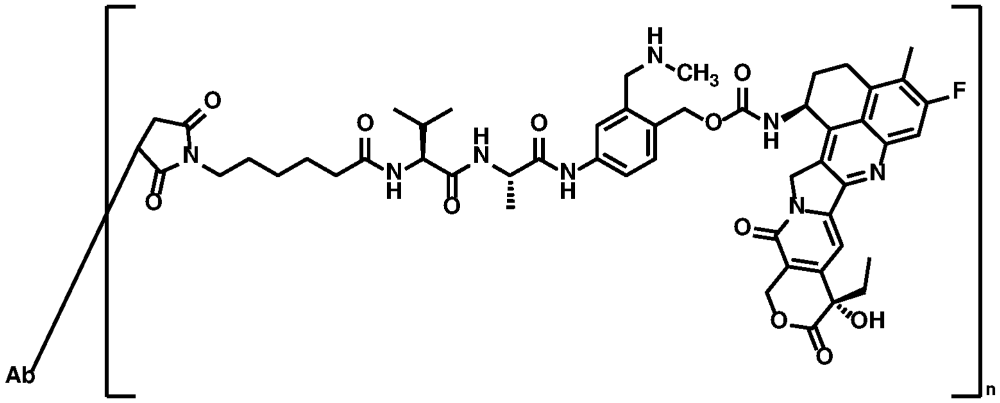

,

Ab represents an anti-HER3 antibody or an antigen-binding fragment; n is 1-10 or 4-10.

The antibody in the antibody-drug conjugate is a HER3 target-specific antibody or an antigen-binding fragment thereof, and is connected with a linker after forming active sulfhydryl through a disulfide bond. In some embodiments, a disulfide bond of the hinge region of the antibody forms active sulfhydryl and is then linked to a linker.

In some embodiments, the antibody in the antibody-drug conjugate is the anti-HER3 antibody or the antigen-binding fragment thereof provided in the [Antibody] section described above of the present disclosure. In some embodiments, the anti-HER3 antibody or the antigen-binding fragment thereof in the antibody-drug conjugate comprises the VL sequence set forth in SEQ ID NO: 11 and the VH sequence set forth in SEQ ID NO: 15.

In the antibody-drug conjugate provided by the present disclosure, due to the use of the anti-HER3 antibody or the antigen-binding fragment thereof and the linker with improved hydrophobicity, the hydrophilicity and the uniformity of the prepared product are both significantly improved, which is beneficial to improving the killing effect of the target cells, and the pharmaceutical properties such as biological activity and safety are improved or maintained, for example, improving the *in vitro*/*in vivo* stability and the *in vivo* drug metabolism properties (longer half-life, lower free small molecule toxin, etc.).

The antibody drug provided by the present disclosure has excellent *in vivo* antibody tumor effect, and particularly has HER3-dependent cell killing or anti-tumor activity, which has killing effect on tumor cells to different degrees according to the expression level of the tumor cells HER3. In some embodiments, the antibody-drug conjugates provided by the present disclosure have very high killing activity on HER3-highly expressed tumor cells, and the level of HER3 expression can be determined according to methods known in the art, such as H-Score scoring: low expression (H-Score = 10-99), medium expression (H-Score = 100-199), and high expression (H-Score = 200-300).

It should be noted that the antibody-drug conjugate of the present disclosure may absorb water, retain adsorbed water, or become a hydrate when left in the atmosphere or recrystallized, and such water-containing compounds and salts are also included in the present disclosure. In addition, isotopic variant compounds labeled with various radioactive or non-radioactive isotopes are also included in the present disclosure. The antibody-drug conjugates of the present disclosure may include one or more atoms that are isotopes in non-natural proportions. Examples of the atomic isotopes include deuterium (2H), tritium (3H), iodine-125 (125I), and carbon-14 (14C). In addition, the compounds of the present disclosure may be radiolabeled with radioactive isotopes, such as tritium (3H), iodine-125 (125I), or carbon-14 (14C). The

radiolabeled compounds are useful as therapeutic or prophylactic agents, research reagents such as test reagents, and diagnostic agents such as *in vivo* imaging diagnostic agents. All isotopic variations for the antibody-drug conjugates of the present disclosure, whether radioactive or not, are intended to be encompassed within the scope of the present disclosure.

[Method for preparing antibody-drug conjugate]

The present disclosure provides a preparation method for an antibody-drug conjugate, which comprises the following steps:

reducing at least part of interchain disulfide bonds of the antibody or the antigen-binding fragment thereof through reduction treatment, reacting with the linker-payload, and particularly reacting with a reactive group of the linker in the linker-payload to obtain the antibody-drug conjugate having the structure of formula I,

Ab-(L-D)n (I)

or an isomer, an isotopic variant, a pharmaceutically acceptable salt, a prodrug, or a solvate thereof, or a combination thereof,
wherein Ab is the anti-HER3 antibody or the antigen-binding fragment thereof described above;
L is a linker covalently linked to Ab and D;
D is a payload;
n is a number between 1 and 10.

In some embodiments, the carbon atom at position 3 of the maleimide-N-yl in L-D is reacted with the reduced antibody and covalently linked to provide an ADC.

In some embodiments, the preparation method comprises the following steps: reducing at least part of interchain disulfide bonds of the antibody through reduction treatment, and conjugating with the linker-payload, wherein the sulfhydryl after disulfide bond reduction is reacted with the carbon atom at position 3 of the maleimide-N-yl of the linker of formula IV in the linker-payload,

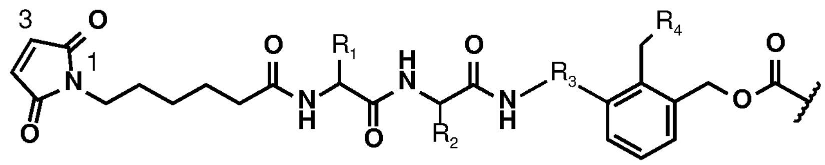

formula IV

the carbonyl in the ester group of the linker of formula IV is linked to the amino group of the payload in the antibody-drug conjugate;
in formula IV, $R_1$ and $R_2$ are independently selected from hydrogen, methyl, and isopropyl groups;
$R_3$ represents $-(CR_5HCONH)n^1-(CH_2CONH)n^2-$ or a single bond, $R_5$ is selected from hydrogen and benzyl, $n^1$ represents an integer of 0-2, and $n^2$ represents an integer of 0-2;
$R_4$ represents a methylamino group or $-(NCH_3COCH_2)n^3-NCH_3COCH_3$, and $n^3$ represents an integer of 1-20.

In many practical cases, in the antibody-drug conjugate, the linker carrying a payload of the structure of formula IV described above is linked to the same antibody molecule having reactive sulfhydryl. In some embodiments, the antibody is reacted with a reducing agent such as dithiothreitol (DTT), 2-mercaptoethanol, or tris(2-carboxyethyl)phosphine hydrochloride (TCEP) to form reactive sulfhydryl from a disulfide bond on the antibody chain. The amount of the reducing agent may be 0.3-10-fold equivalents of the molar quantity of the antibody, for example, 1-10, 3-10, 5-10, and 7-10-fold molar equivalents.

In some embodiments, the method further comprises: reacting the antibody with the reducing agent in a buffer solution containing a chelating agent, and then adding a linker-payload solution to carry out a reaction. The linker-payload is specifically a compound formed by bonding the linker of formula IV and the payload, wherein the amino group (primary amino group) in the payload is linked to the carbonyl group in the ester group of the linker of IV. The payload is selected from the payloads described in [Antibody-drug conjugates]. In some embodiments, the payload is exatecan. The term "chelating agent" is capable of binding to a metal atom or ion via a coordinate bond to form a complex having a cyclic structure.

In some embodiments, the reducing agent is reacted with the antibody in a buffer solution containing a chelating

agent to give an antibody in which the interchain disulfide bonds are partially or completely reduced. The chelating agents include, but are not limited to, ethylenediaminetetraacetic acid (EDTA) and diethylenetriaminepentaacetic acid (DTPA). The chelating agent is used in a concentration of 1 mM-20 mM, for example, 2 mM-20 mM, 5 mM-20 mM, 8 mM-20 mM, 1 mM-15 mM, or 1 mM-10 mM. The ingredients of the buffer solution may be buffer salts commonly used in the art, such as sodium phosphate, sodium borate, sodium acetate, or similar buffer salts.

The reaction of the antibody with the reducing agent is carried out under adjusted pH conditions. In some embodiments, the antibody is reacted with the reducing agent at a pH of 5-9, optionally pH 6-8, pH 6-7, pH 6.5-7.5 or pH 7-8, for example at about pH 7. The pH of the solution may be adjusted using chemicals having acidity or basicity, and exemplary chemicals having acidity or basicity include acetic acid, hydrochloric acid, phosphoric acid, sulfuric acid, sodium bicarbonate, sodium carbonate, sodium hydroxide, and triethylamine.

The reaction of the antibody with the reducing agent is carried out at an adjusted temperature, for example, at -10 to 40 °C, -5 to 40 °C, 0 to 40 °C, 5 to 40 °C, 10 to 40 °C, 15 to 40 °C, 20 to 40 °C, 25 to 40 °C, 30 to 40 °C, and 35 to 38 °C, e.g., about 37 °C.

The linker-payload may be dissolved in an organic solvent selected from any one or a combination of dimethyl sulfoxide (DMSO), dimethylformamide (DMF), dimethylacetamide (DMA), and N-methyl-2-pyrrolidone (NMP).

In some embodiments, the solution of the linker-payload is added to the buffer solution that has been subjected to reduction treatment or contains an antibody having active sulfhydryl in an amount of 1-20% by volume based on the volume of the antibody buffer solution. In some embodiments, the solution of the linker-payload is added in a volume ratio of 1-20%, 2-20%, 5-20%, 10-20%, 15-20%, 1-18%, 1-15%, 1-13%, 1-10%, or 5-15% based on the volume of the antibody buffer solution.

In some embodiments, the ratio of the molar amount of the linker-payload to the antibody is 4-20, optionally 8-20. In some embodiments, the ratio of the molar amount of the linker-payload to the antibody is 10-20, 14-20, 16-20, or 18-20.

In some embodiments, the temperature at which the antibody and linker-payload are reacted is 0-37 °C. In some embodiments, the reaction temperature is -10 to 40 °C, -5 to 40 °C, 0 to 40 °C, 5 to 40 °C, 5 to 37 °C, 10 to 37 °C, 10 to 25 °C, or 15 to 30 °C.

In some embodiments, the antibody is reacted with the linker-payload for a period of 0.5-2 h. In some embodiments, the antibody is reacted with the linker-payload for a period of 0.5-1.75 h, 0.5-1.5 h, 0.5-1.25 h, 0.75-2 h, or 1-2 h.

The reaction can be terminated by inactivating the reactivity of the unreacted linker-payload using a thiol-containing reagent. The thiol-containing reagent includes, but is not limited to, cysteine or N-acetyl-(L)-cysteine (NAC). More specifically, adding a thiol-containing reagent having a 1-2-fold molar equivalents of the linker-payload is added to the reaction solution, and the reaction solution is incubated at room temperature (10 to 25 °C) for 10-30 min to terminate the reaction.

In the case of antibodies having sulfhydryl, the antibody-drug conjugate can also be obtained by reacting the compound using known methods (e.g., obtainable by the method described in patent publication US2016/297890 (e.g., obtainable by the method described in paragraphs [0336] to [0374])). The antibody having sulfhydryl can be obtained by methods well known to those skilled in the art (Hermanson, G. T, Bioconjugate Techniques, pp. 56-136, pp. 456-493, Academic Press (1996)).

In some embodiments, the antibody in the antibody-drug conjugate is selected from the anti-HER3 antibody or the antigen-binding fragment thereof disclosed in the [Antibody] section. In some embodiments, the anti-HER3 antibody or the antigen-binding fragment thereof in the antibody-drug conjugate comprises the VL sequence set forth in SEQ ID NO: 11 and the VH sequence set forth in SEQ ID NO: 15.

The antibody-drug conjugate provided by the present disclosure can be obtained by the preparation method described above. In some embodiments, the prepared antibody-drug conjugate is subjected to a purification process including, but not limited to, gel filtration, e.g., purification using a gel column.

In the antibody-drug conjugate prepared by the method disclosed by the present disclosure, the linker and the payload exatecan are conjugated with the antibody targeting HER3 by a simple chemical method. Compared with the traditional random conjugation mode, the anti-HER3 antibody-drug conjugate obtained by applying the linker has a higher DAR value (such as DAR 8).

[Pharmaceutical composition]

The present disclosure provides a pharmaceutical composition comprising the anti-HER3 antibody or the antigen-binding fragment thereof or the antibody-drug conjugate described above, or an isomer, an isotopic variant, a pharmaceutically acceptable salt, a prodrug, or a solvate thereof, or a combination thereof, and a pharmaceutically acceptable excipient.

A proper administration mode may be selected for the pharmaceutical composition of the present disclosure according to the specific application form, physicochemical characteristics and the like of the pharmaceutically acceptable

excipient. In some embodiments, the pharmaceutical composition may be formulated in the form of a lyophilized formulation or a liquid formulation, which may contain an appropriate formulation additive known in the art. For example, the pharmaceutical composition described above typically comprises one or more pharmaceutical carriers, e.g., sterile liquids such as water and oils (including those of petroleum, animal, vegetable, or synthetic origin (e.g., peanut oil, soybean oil, mineral oil, sesame oil, etc.)). In the case of intravenous administration of the pharmaceutical composition described above, water is a more typical carrier. In addition, saline, and solutions of glucose and glycerol in water can also be used as a liquid carrier, particularly for injection. Suitable pharmaceutical excipients are known in the art. The composition described above may further contain, if desired, a small amount of a wetting agent, an emulsifier, or a pH buffering agent. The pharmaceutical composition is usually administered parenterally, and may be administered by intradermal, intramuscular, intraperitoneal, intravenous or subcutaneous injection, but is not limited thereto, for example, by infusion or bolus injection. In general, the active ingredients, e.g., an anti-HER3 antibody or an antigen-binding fragment thereof, or an antibody-drug conjugate, can be prepared as a pharmaceutical composition using techniques and methods well known in the art (see, e.g., Ansel Introduction to Pharmaceutical Dosage Forms, seventh edition 1999).

The pharmaceutical composition of the present disclosure may be a pharmaceutical composition comprising only the anti-HER3 antibody or the antigen-binding fragment thereof, or the antibody-drug conjugate of the present disclosure, or may be a pharmaceutical composition comprising the anti-HER3 antibody or the antigen-binding fragment thereof, or the antibody-drug conjugate, and at least one second therapeutic agent (e.g., a cancer therapeutic agent). In some embodiments, the antibody-drug conjugate of the present disclosure may also be administered with other cancer therapeutic agents to enhance the anti-cancer effect. Other anti-cancer agents used for this purpose may be administered to the individual simultaneously, separately or sequentially with the antibody-drug conjugate, or may be administered with varying intervals of administration. Exemplary other cancer therapeutic agents such as paclitaxel, cisplatin, and vinblastine are not limited as long as they have anti-tumor activity.

[Kit]

The present disclosure also provides a kit comprising the anti-HER3 antibody or the antigen-binding fragment thereof, or the antibody-drug conjugate described above. The kit may further comprise, if desired, a container, a buffer, and controls such as a positive control and a negative control. One skilled in the art may make a corresponding selection if desired. Accordingly, the kit may further comprise instructions for use to facilitate the operation and use of the kit by those skilled in the art.

[Use]

The present disclosure provides use of the anti-HER3 antibody or the antigen-binding fragment thereof described above, the antibody-drug conjugate, the pharmaceutical composition, an antibody-drug conjugate prepared by the method described above, and the kit in preparing a therapeutic agent for diagnosing, preventing and treating tumor diseases.

In some embodiments, provided is use of the HER3 antibody or the antigen-binding fragment thereof, the antibody-drug conjugate, the pharmaceutical composition, and the antibody-drug conjugate prepared by the method described above, and the kit in preparing an anti-tumor drug, or in preparing a drug targeting HER3.

The anti-HER3 antibody or the antigen-binding fragment thereof and the antibody-drug conjugate provided by the present disclosure have the function of killing cells expressing HER3, which can be used for killing cells expressing HER3 *in vivo* or *in vitro.*

Such tumor diseases include benign tumors and malignant tumors (e.g. cancers), and in particular, the antibody-drug conjugate is suitable for use in tumors or cancers where HER3 expression is observed. In some embodiments, the tumors or cancers are solid tumors expressing HER3. In some embodiments, the tumor or cancer includes, but is not limited to, lung cancer, kidney cancer, urothelial cancer, colorectal cancer, prostate cancer, glioblastoma multiforme, ovarian cancer, pancreatic cancer, breast cancer, metastatic breast cancer, luminal breast cancer, melanoma, liver cancer, bladder cancer, gastric cancer (stomach cancer), gastrointestinal stromal tumor, cervical cancer, head and neck cancer, esophageal cancer, epidermoid cancer, peritoneal cancer, adult glioblastoma multiforme, hepatic cancer, hepatocellular cancer, colon cancer, rectal cancer, colon and rectal cancer, endometrial cancer, uterine cancer, salivary gland cancer, renal cancer, vulval cancer, thyroid cancer, hepatic carcinoma, anal cancer, penile cancer, and lung adenocarcinoma (including EGFR-TKI-resistant lung adenocarcinoma).

In some embodiments, the class of cancer is breast cancer, lung adenocarcinoma, pancreatic cancer, and colon cancer. In some embodiments, the class of cancer is breast cancer, EGFR-TKI-resistant lung adenocarcinoma, pancreatic cancer, and colon cancer.

When a subject has or is suspected of having a solid tumor expressing HER3, a therapeutic dose of a therapeutic agent comprising the anti-HER3 antibody or the antigen-binding fragment thereof described above, the antibody-drug

conjugate, an antibody-drug conjugate prepared by the method described above, and the pharmaceutical composition described above is administered to the subject, which can be used for diagnosing, preventing and treating tumor diseases.

The present disclosure also provides a method for diagnosing, preventing and treating tumor diseases, which comprises administering to a subject in need a therapeutic dose of a therapeutic agent comprising the anti-HER3 antibody or the antigen-binding fragment thereof described above, the antibody-drug conjugate, an antibody-drug conjugate prepared by the method described above, and the pharmaceutical composition or the kit described above.

The subject is a mammal, including a human, a non-human primate, a dog, a pig, and a mouse; preferably, the subject is a human, e.g. a patient with a HER3-expressing tumor.

The therapeutic dose of the therapeutic agent will vary depending upon factors such as the particular condition being treated, the severity of the condition, the individual patient parameters (including age, physical condition, size, gender, and weight), the duration of treatment, the nature of concurrent therapy (if any), the specific route of administration, and the knowledge of the medical practitioner. In some embodiments, the dose of an active ingredient, such as the anti-HER3 antibody or the antigen-binding fragment thereof, or the antibody-drug conjugate, may be determined empirically in an individual who has been given one or more antibody administrations.

In some embodiments, the therapeutically acceptable dose of the anti-HER3 antibody or the antigen-binding fragment thereof, or the antibody-drug conjugate is 0.1-30 mg/kg, 0.5-30 mg/kg, 1-30 mg/kg, 1-25 mg/kg, 0.1-25 mg/kg, 0.1-20 mg/kg, 1-20 mg/kg, or 0.5-20 mg/kg. In some embodiments, the frequency of administration is once every 12 h, daily, weekly, every 2 weeks, every 4 weeks, every 5 weeks, every 6 weeks, every 7 weeks, every 8 weeks, every 9 weeks, or every 10 weeks; or once a month, every 2 months, or every 3 months or longer. The therapeutic dose and frequency of administration may vary with the treatment regimen.

The anti-HER3 antibody or the antigen-binding fragment thereof, the antibody-drug conjugate, or the pharmaceutical composition of the present disclosure may also be used in combination with one or more second therapeutic agents, which when combined may occur simultaneously or sequentially by the same or different routes of administration. This may vary depending on the metabolic characteristics of the therapeutic agent itself and the disease being treated.

The various embodiments and preferences of the present disclosure may be combined with each other (provided they are not inherently contradictory to each other), and the various embodiments formed thereby are considered part of the present disclosure.

The technical solutions of the present disclosure will be more clearly and explicitly illustrated by way of examples in the following. It should be understood that the examples are for illustrative purposes only and are not intended to limit the scope of the present disclosure in any way. The scope of the present disclosure is limited only by the claims.

## EXAMPLES

The present disclosure is specifically illustrated by the examples shown below, but the present disclosure is not limited thereto. In addition, the examples are not to be construed as limiting in any manner. In addition, in this specification, reagents, solvents, and starting materials not specifically described can be easily obtained from commercially available sources.

### Example 1: Preparation of Compound LP-1

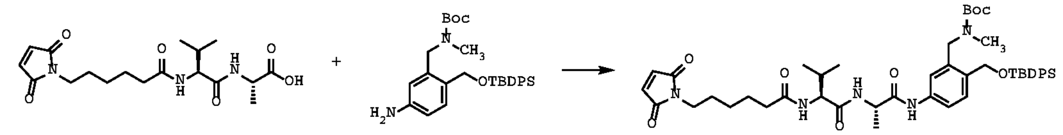

11-1A 11-1B 11-1

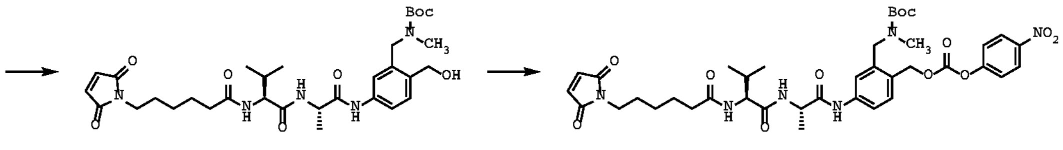

11-2 11-3

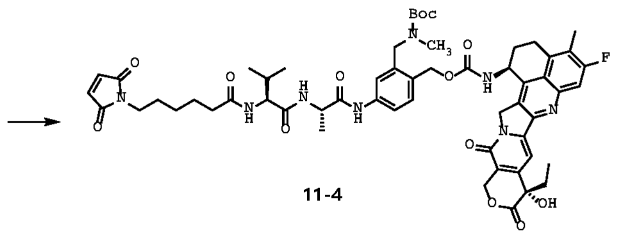

11-4

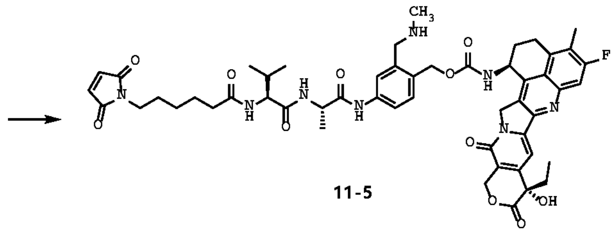

11-5

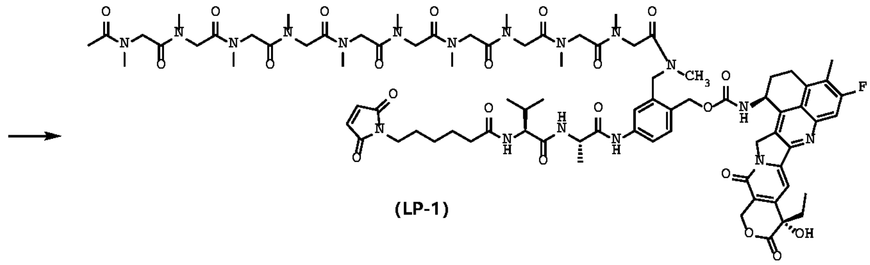

(LP-1)

Step 1: synthesis of intermediate 11-1

A mixed solution of dichloromethane (DCM) and methanol (MeOH) (v:v = 2:1, 90 mL) and 2-ethoxy-1-ethoxycarbonyl-1,2-dihydroquinoline (EEDQ, 1.86 g, 7.55 mmol) were added to a mixed solution of compound 11-1A (Mc-Val-Ala-OH, purchased from Shanghai Haoyuan Chemexpress Co. Ltd., 2.4 g, 6.29 mmol) and 11-1B (3.18 g, 6.29 mmol) at room temperature (25 °C-30 °C). The mixture was reacted at room temperature for 24 h, and the solvent was removed under vacuum. The crude residue was purified by flash chromatography to give compound 11-1 (3.9 g, 71%). LC-MS (ESI, m/z): 868.49(M+H).

Step 2: synthesis of intermediate 11-2

Compound 11-1 (2 g, 2.3 mmol) was dissolved in anhydrous tetrahydrofuran (THF, 50 mL). Hydrogen fluoride-pyridine (4.6 g, 46 mmol) was added at 0 °C under argon atmosphere. The mixture was reacted for 2 h under stirring, and water was added to quench the reaction. The mixture was extracted with DCM, and the organic phase was dried and concentrated. The residue was purified by silica gel chromatography to give compound 11-2 (1.1 g, 76%). LC-MS (ESI, m/z): 630.31 (M+H).

Step 3: synthesis of intermediate 11-3

Compound 11-2 (700 mg, 1.11 mmol) was dissolved in anhydrous N,N-dimethylformamide (DMF, 4 mL), and N,N-diisopropylethylamine (DIPEA, 0.39 mL, 2.23 mmol) and 4,4'-dinitrodiphenyl carbonate (406 mg, 1.33 mmol) were added under argon atmosphere at room temperature. The reaction mixture was stirred at ambient temperature overnight. The mixture was concentrated to remove the solvent, and methyl *tert*-butyl ether (MTBE) was added to precipitate a product. The mixture was filtered to collect a yellow solid, which was washed with diethyl ether and dried to give compound 11-3. LC-MS (ESI, m/z):795.41 (M+H).

Step 4: synthesis of intermediate 11-4

Compound 11-3 (300 mg, 0.44 mmol) was dissolved in 4 mL of anhydrous DMF, 1 mL of dried pyridine was added, and then exatecan mesylate (purchased from Shanghai Haoyuan Chemexpress Co. Ltd., 234 mg, 0.44 mmol) and 1-hydroxybenzotriazole (HOBt, 60 mg, 0.44 mmol) were added. The reaction mixture was stirred at room temperature under argon atmosphere overnight. The product was purified by preparative high performance liquid chromatography (pre-HPLC) to give intermediate 11-4 (230 mg, 48%). LC-MS (ESI, m/z):1091.53 (M+H).

Step 5: synthesis of intermediate 11-5

Compound 11-4 (200 mg, 0.183 mmol) was dissolved in 1 mL of anhydrous DCM, and 300 μL of TFA was added at 0 °C. The mixture was stirred at room temperature for 30 min and concentrated to remove the solvent to give the TFA salt of intermediate 11-5. LC-MS (ESI, m/z):991.47 (M+H).

Step 6: Synthesis of compound LP-1

Compound 11-5 (120 mg, 0.109 mmol) was dissolved in 1 mL anhydrous DMF, N-acetyldecasarcosine (Ac-Sar10-COOH, 84 mg, 0.109 mmol) was added, and 2-(7-azabenzotriazole)-N,N,N',N'-tetramethyluronium hexafluorophosphate (HATU, 50 mg, 0.130 mmol) and DIPEA (38 μL, 0.22 mmol) were then added. The reaction mixture was stirred overnight at room temperature and concentrated to remove the solvent. The crude product was purified by pre-HPLC to give compound LP-1 (74 mg, 38%). LC-MS (ESI, m/z):1743.85 (M+H).

**Example 2: Preparation of Compound LP-2**

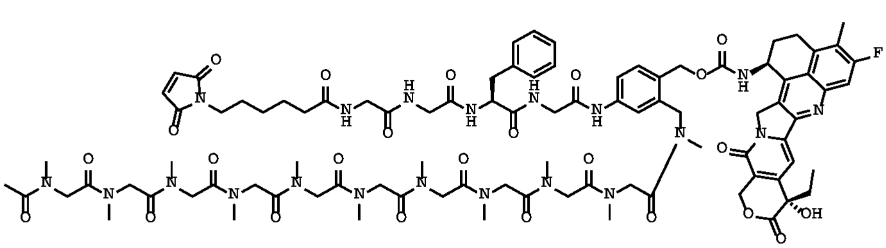

LP-2

Compound LP2 was synthesized according to the procedure for compound LP-1, in which the starting material 11-1A was replaced with Mc-GGFG-OH (purchased from Shanghai Haoyuan Chemexpress Co. Ltd., GGFG represents an amino acid sequence consisting of glycine-glycine-phenylalanine-glycine linked through peptide bonds) to give compound LP-2 as a beige amorphous solid. LC-MS (ESI, m/z): 1891.90 (M+H).

**Example 3: Preparation of Compound LP-3**

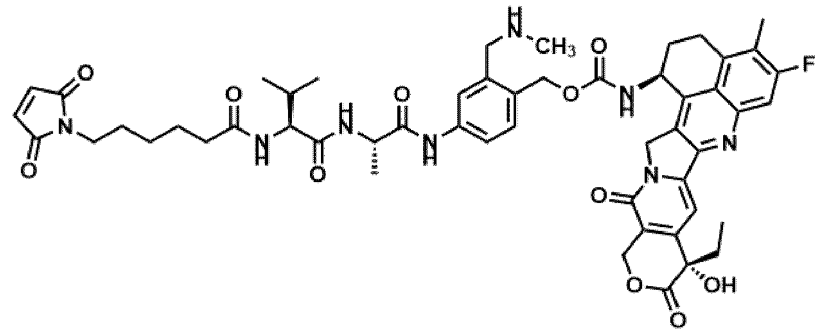

LP-3

Compound LP-3 is an intermediate of LP-1. The step 6 was removed, and intermediate 11-5 was LP-3.

### Example 4: Preparation of Monoclonal Antibodies

Balb/c mice (8-12 weeks old) were immunized with human HER3 protein extracellular domain fragment antigen (NCBI reference sequence: NM_001982.4, amino acid sequence positions 20-643) and their serum titers were monitored to determine the number of immunizations. After the initial immunization, 3 to 4 booster immunizations were performed. The mouse sera were taken and the titer was determined according to the routine method in the art. After one intensive immunization of mice qualified by titer detection, the whole spleen and 1/2 lymph node were taken and PEG-fused with myeloma SP2/0 cell line. The fused cells were plated and cultured, supernatants from all wells were collected, and antigens were screened by ELISA. Positive wells containing cells identified under the microscope were transferred to a 96-well plate for further culture. After 7 days of growth, supernatants from all wells were collected and tested for reaction with antigen using ELISA. Positive wells were further detected for different dilutions of antigen-binding for affinity ranking. 20 parental clones with the highest immunogenic affinity were selected for subcloning. Subcloning was performed by a limiting dilution method and ELISA screening to obtain a hybridoma cell strain. The hybridoma cell strain was used to prepare mouse ascites, and a monoclonal antibody named as Mu4O3 was obtained after collection and purification, the amino acid sequences of the CDR and the variable region of which were determined as shown in Table 1 below, wherein the CDR (Kabat system definition and numbering) is shown by underlining.

Table 1 Hybridoma variable region amino acid sequence (one-letter abbreviation)

| | |
|---|---|
| VL | DIVMTQSPATLSVTPGDRVSLSCRASQSISDYLHWYQQKSHESPRLLIKYASQSISGIPSRFSGSGSGSDFTLNINSVEPEDVGLYYCQSGRSFPLTFGAGTKVELK (SEQ ID NO:9) |
| VH | DVQLQESGPGLVKPSQTVSLTCTVTGISITTGNYRWSWIRQFPGNKLEWIGCIYYSGAITYTPSLTGRTTITRDTSRNQFFLEMNSLTAEDTATYYCARDGGTVVEGWYFDVWGAGTTVTVSS (SEQ ID NO:10) |

The hybridoma sequences were subjected to humanization modification to obtain humanized 4O3 antibodies (abbreviated as "Hu4O3") L1H4, L1H5, L2H4, L2H5, L4H4, L4H5, and L4H7. L1, L2, and L4 represent the numbers of the antibody light chain variable region sequences, and H4, H5, and H7 represent the numbers of the antibody heavy chain variable region sequences, that is, the L1H4 antibody comprises the light chain variable region numbered L1 and the heavy chain variable region numbered H4; the L1H5 antibody comprises the light chain variable region numbered L1 and the heavy chain variable region numbered H5; the L2H4 antibody comprises the light chain variable region numbered L2 and the heavy chain variable region numbered H4; the L2H5 antibody comprises the light chain variable region numbered L2 and the heavy chain variable region numbered H5; the L4H4 antibody comprises the light chain variable region numbered L4 and the heavy chain variable region numbered H4; the L4H5 antibody comprises the light chain variable region numbered L4 and the heavy chain variable region numbered H5; the L4H7 antibody comprises the light chain variable region numbered L4 and the heavy chain variable region numbered H7.

The CDR sequences (underlined) and variable region sequences of Hu4O3 are shown in Table 2 below, wherein the mutated amino acids in CDR2 of VH are shown in bold italics. CDRs are defined and numbered using the Kabat system.

Table 2 Variable region sequence of humanized 4O3 antibody (one-letter abbreviation)

| Hu4O3 | No. | Amino acid sequence |
|---|---|---|
| VL | L1 | EIVLTQSPDTQSVTPKEKVTITCRASQSISDYLHWYQQKPDQSPKLLIKYASQSISGVPSRFSGSGSGTDFTLTINSLEAEDAATYYCQSGRSFPLTFGGGTKVEIK (SEQ ID NO:11) |
| | L2 | EIVLTQSPDTQSVTPKEKVTITCRASQSISDYLHWYQQKPDQSPKLLIKYASQSISGVPSRFSGSGSGTDFTLTINSLEAEDVGLYYCQSGRSFPLTFGGGTKVEIK (SEQ ID NO:12) |
| | L4 | EIVLTQSPDTQSVTPKEKVTITCRASQSISDYLHWYQQKPGQAPKLLIKYASQSISGVPSRFSGSGSGTDFTLTINSLEAEDFGLYYCQSGRSFPLTFGGGTKVEIK (SEQ ID NO:13) |
| VH | H4 | QVQLQESGPGLVKPSQTLSLTCTVSGISITTGNYRWSWIRQHPGKKLEWIG***N***IYYSGAITY***N***PSL***KS***RVTISRDTSKNQFFLKLSSVTAADTAVYYCARDGGTVVEGWYFDVWGKGTTVTVSS (SEQ ID NO:14) |
| | H5 | QVQLQESGPGLVKPSQTVSLTCTVSGISITTGNYRWSWIRQHPGKKLEWIG***N***IYYSGAITY***N***PSL***KS***RVTISRDTSKNQFFLKLSSVTAADTAVYYCARDGGTVVEGWYFDVWGKGTTVTVSS (SEQ ID NO:15) |
| | H7 | QVQLQESGPGLVKPSQTLSLTCTVSGISITTGNYRWSWIRQPPGKGLEWIG***Y***IYYSGAITY***N***PSL***KS***RVTISRDTSKNQFSLQLRSVTAADTAVYYCARDGGTVVEGWYFDVWGQGTTVTVSS (SEQ ID NO:16) |

For the purpose of example preparation and/or detection, the constant region of the above antibody is selected from the constant region of human IgG1, wherein the heavy chain constant region sequence is selected from the amino acid sequence set forth in SEQ ID NO: 17, and the light chain constant region sequence is selected from the amino acid sequence set forth in SEQ ID NO: 18.

**Example 5: Method for Detecting Antibody-Drug Conjugate**

The antibody-drug conjugates were identified by concentration, medium exchange, purification, measurement of the antibody concentration, and calculation of the average molecular number of the drug molecules carried by each antibody according to the following methods.

**Operation A: Concentration of antibody or antibody-drug conjugate**

A solution of the antibody or antibody-drug conjugate to be concentrated was added to an ultrafiltration tube (Amicon Ultra, 50000 MWCO, Millipore), centrifuged to a desired volume, and taken out.

**Operation B: Measurement of antibody concentration**

The absorbance of the antibody was measured using a microplate reader (Multiskan GO, Thermo Fisher) according to the method defined by the manufacturer, and the ratio of the absorbance to the absorption coefficient of the antibody at that wavelength was the concentration of the antibody.

**Operation C: Medium exchange of antibody**

The zeba desalting column (5 mL, 40K MWCO) was equilibrated beforehand with a phosphate buffer containing sodium chloride (50 mM) and EDTA (2 mM) (abbreviated as "PBS 7.0/EDTA", 50 mM, pH 7.0) according to the instructions provided by the manufacturer (Thermo Fisher). 2 mL of sample was loaded on each zeba desalting column and centrifuged (1000 g, 4 min). The flow-through fractions were collected and concentrated by Operation A. The antibody concentration was determined by Operation B and adjusted with PBS 7.0/EDTA.

**Operation D: Purification of antibody-drug conjugate**

The zeba desalting column (5 mL, 40K MWCO) was equilibrated beforehand with a storage buffer according to the instructions provided by the manufacturer (Thermo Fisher). A histidine-acetic acid buffer (20 mM histidine, pH 5.5) containing 150 mM sodium chloride or a phosphoric acid buffer (50 mM, pH 7.0) containing 50 mM sodium chloride was used as the storage buffer. The reaction solution containing the antibody-drug conjugate (about 2 mL) was added to the zeba desalting column and centrifuged (1000 g, 4 min), and the flow-through fractions (about 2 mL) were collected. The elution process was repeated twice to remove unbound linker-payload and low molecular weight compounds comprising a reducing agent.

**Operation E: Measurement of antibody concentration in antibody-drug conjugate and average number of drug molecule linked per antibody (DAR value)**

The concentration of the conjugated drug on the antibody-drug conjugate can be obtained by measuring the ultraviolet absorbance at 280 nm and 370 nm of an aqueous solution of the antibody-drug conjugate and calculating by the following formulas.

At any given wavelength, the total absorbance of a system is equal to the sum of the absorbances of all light-absorbing chemicals present in the system (additivity of absorbance). Therefore, assuming that the molar absorption coefficients of the antibody and the drug are unchanged before and after the antibody and the drug are conjugated, the antibody concentration and the drug concentration in the antibody-drug conjugate can be expressed by the following formulas.

$$A_{280} = A_{D,280} + A_{A,280} = \varepsilon_{D,280}C_D + \varepsilon_{A,280}C_A \qquad \text{formula (1)}$$

$$A_{370} = A_{D,370} + A_{A,370} = \varepsilon_{D,370}C_D + \varepsilon_{A,370}C_A \qquad \text{formula (2)}$$

$A_{280}$ represents the total absorbance at 280 nm of the aqueous solution of the antibody-drug conjugate, and $A_{370}$ represents the total absorbance at 370 nm of the aqueous solution of the antibody-drug conjugate. $A_{A,280}$ represents the absorbance of the antibody at 280 nm, $A_{A,370}$ represents the absorbance of the antibody at 370 nm, $A_{D,280}$ represents the absorbance of the drug molecule at 280 nm, $A_{D,370}$ represents the absorbance of the drug molecule at 370 nm, $\varepsilon_{A,280}$ represents the molar extinction coefficient of the antibody at 280 nm, $\varepsilon_{A,370}$ represents the molar extinction coefficient of the antibody at 370 nm, $\varepsilon_{D,280}$ represents the molar extinction coefficient of the drug molecule at 280 nm, $\varepsilon_{D,370}$ represents

the molar extinction coefficient of the drug molecule at 370 nm, $C_A$ represents the concentration of the antibody in the antibody-drug conjugate, and $C_D$ represents the concentration of the drug molecule in the antibody-drug conjugate.

$\varepsilon_{A,280}$, $\varepsilon_{A,370}$, $\varepsilon_{D,280}$, and $\varepsilon_{D,370}$ are known values (calculated from the sequence of the antibody or measured by UV absorption of the compound). For example, $\varepsilon_{A,280}$ can be calculated from the amino acid sequence of an antibody by a known method (Protein Science, 1995, Vol.4, pages 2411-2423). Antibodies typically do not absorb at 370 nm, so $\varepsilon_{A,370}$ is typically 0. The values of $\varepsilon_{0,280}$ and $\varepsilon_{D,370}$ can be calculated by measuring the changes in absorbance of the drug molecule at 280 nm and 370 nm with concentration, respectively, using the Lambert-Beer law (absorbance = molar concentration × molar extinction coefficient × optical path length). $C_A$ and $C_D$ can be obtained by measuring the absorbance $A_{280}$ and $A_{370}$ of the antibody-drug conjugate at 280 nm and 370 nm and then solving the system of linear equations in two variables of formulas (1) and (2). In addition, the number of drug molecules linked to each antibody (DAR value) can be obtained by dividing $C_D$ by $C_A$.

**Operation F: Measurement of aggregate in antibody-drug conjugate**

Aggregates in the antibody-drug conjugate were detected using size exclusion chromatography in high performance liquid chromatography as follows:

High performance liquid chromatography system: Agilent 1260 Infinity II HPLC system
Detector: Ultraviolet absorption spectrometer (detection wavelength: 280 nm)
Chromatographic column model: TOSOH TSKgel G3000SWXL (7.8 × 300 mm, 5 μm)
Mobile phase: 200 mmol/L $KHPO_4$, 150 mmol/L NaCl, 15 % (v/v) isopropanol, pH 7.0
Flow rate: 0.75 mL/min
Analysis time: 18 min
Column temperature: room temperature
Sample injection amount: 50 μg
Data analysis:
A size exclusion chromatogram of a quality control (QC, L1H5 naked antibody, i.e., antibody not conjugated with linker-payload and numbered L1H5) is shown in FIG. 1A; the retention time of the main peak (single peak) of the 150 kDa quality control is between 9.5 and 10.5 min. The retention time of the aggregates should be earlier than the retention time of the above monomers.

**Operation G: Hydrophobicity measurement of antibody-drug conjugate**

The hydrophobicity of the antibody-drug conjugate was analyzed using high performance liquid chromatography hydrophobic interaction chromatography (HIC) as follows:

High performance liquid chromatography system: Agilent 1260 Infinity II HPLC system
Detector: Ultraviolet absorption spectrometer (detection wavelength: 280 nm)
Chromatographic column model: TOSOH TSKgel Butyl-NPR (4.6 mm I.D. × 3.5 cm, 2.5 μm)
Mobile phase A: $(NH_4)_2SO_4$, 50 mmol/L $KHPO_4$, pH 7.0
Mobile phase B: 50 mmol/L $KHPO_4$, 25 % (v/v) isopropanol, pH 7.0
Analysis time: 25 min
Column temperature: room temperature
Elution procedure (B%): 0%-25% (0-1 min), 25% (1-3 min), 25%-80% (3-13 min), 80% (13-17 min), 80%-0% (17-17.10 min), 0% (17.10-25 min)
Sample injection volume: 10 μL

Data analysis: The hydrophobic chromatography chromatogram of the quality control (QC, L1H5 naked antibody) is shown in FIG. 1B and FIG. 22; the hydrophobic chromatography chromatogram of the quality control reference antibody is shown in FIG. 22. Generally, samples with shorter retention time will be less hydrophobic and the antibody-drug conjugate will be more hydrophobic than the naked antibody to which the drug molecule is not conjugated, and thus should have longer retention time.

**Example 6: Preparation of Antibody-Drug Conjugate L1H5-LP3**

Reduction of antibody: The antibody L1H5 prepared in Example 4 was subjected to medium exchange using PBS7.0/EDTA according to Operation B (1.49 $mLmg^{-1}$ $cm^{-1}$ was used as the extinction coefficient of the antibody at 280 nm) and Operation C of Example 5, and the antibody concentration after the medium exchange was 20.76 mg/mL. To

481.70 μL of an aqueous solution of L1H5 antibody, 93.33 μL of a 5 mM TCEP solution (equivalent to 7 times the antibody content) was added, and 200 μL of 50 mM phosphate buffer (pH 7.0, PBS 7.0) and 224.97 μL of ultrapure water were added. The mixture was reacted at 37 °C for 2 h.

Conjugation of antibody to linker-payload: The mixture described above was incubated at 4 °C for 10 min. The linker-payload LP3 prepared in Example 3 was dissolved in N,N-dimethylacetamide (DMA) and added to the mixture in a volume of 93.33 μL (equivalent to 14 times the antibody content), and the mixture was reacted at 22 °C for another 30 min.

Purification of antibody-drug conjugate: The reaction solution described above was purified by the method of Operation D in Example 5 to obtain the antibody-drug conjugate L1H5-LP3.

Characterization of antibody-drug conjugate: The obtained antibody-drug conjugate was characterized using Operation E ($\varepsilon_{D,280}$ = 5186 and $\varepsilon_{D,370}$ = 13688), Operation F, and Operation G in Example 5.

The concentration of the antibody-drug conjugate was calculated to be 5.73 mg/mL by the Operation E measurement, and the number of payloads conjugated on average per antibody was calculated to be 7.78 by the Operation E measurement. FIG. 2A shows a detection profile of aggregates, with an aggregate content of 2.20% for antibody-drug conjugate L1H5-LP3, as measured by Operation F. FIG. 2B shows a hydrophobic chromatography detection profile of antibody-drug conjugate L1H5-LP3, with a retention time of 6.169 min for the antibody-drug conjugate, as measured by Operation G.

**Example 7: Preparation of Antibody-Drug Conjugate L1H5-LP1**

Reduction of antibody: The antibody was subjected to medium exchange using PBS7.0/EDTA according to Operation B (1.49 $mLmg^{-1}$ $cm^{-1}$ was used as the extinction coefficient of the antibody at 280 nm) and Operation C of Example 5, and the antibody concentration after the medium exchange was 11.44 mg/mL. To 1048.95 μL of an aqueous solution of the L1H5 antibody prepared in Example 4, 160 μL of a 5 mM TCEP solution (equivalent to 10 times the antibody content) was added, and 320 μL of 50 mM PBS 7.0 and 71.05 μL of ultrapure water were added thereto at the same time. The mixture was reacted at 37 °C for 2 h.

Conjugation of antibody to linker-payload: The mixture described above was incubated at 4 °C for 10 min. The linker-payload LP1 prepared in Example 1 was dissolved in DMA and added to the mixture in a volume of 120 μL (equivalent to 15 times the antibody content), and the mixture was reacted at 22 °C for another 30 min.

Purification of antibody-drug conjugate: The reaction solution described above was purified by the method of Operation D in Example 5 to obtain the antibody-drug conjugate L1H5-LP1.

Characterization of antibody-drug conjugate: The obtained antibody-drug conjugate was characterized using Operation E ($\varepsilon_{D,280}$ = 6384 and $\varepsilon_{D,370}$ = 16180), Operation F, and Operation G in Example 5.

The concentration of the antibody-drug conjugate was calculated to be 6.12 mg/mL by the Operation E measurement, and the number of payloads conjugated on average per antibody was calculated to be 8.03 by the Operation E measurement. FIG. 3A shows a detection profile of aggregates, with an aggregate content of 1.60% for antibody-drug conjugate L1H5-LP1, as measured by Operation F. FIG. 3B shows a hydrophobic chromatography detection profile of antibody-drug conjugate L1H5-LP1, with a retention time of 6.234 min for the antibody-drug conjugate L1H5-LP1, as measured by Operation G.

Drug conjugates of other antibodies, such as L1H4 and L4H4, to LP1 were prepared as described in Example 7.

**Example 8: Preparation of Antibody-Drug Conjugate L1H5-LP2**

Reduction of antibody: The antibody was subjected to medium exchange using PBS7.0/EDTA according to Operation B (1.49 $mLmg^{-1}$ $cm^{-1}$ was used as the extinction coefficient of the antibody at 280 nm) and Operation C of Example 5, and the antibody concentration after the medium exchange was 11.44 mg/mL. To 1048.95 μL of an aqueous solution of the L1H5 antibody prepared in Example 4, 160 μL of a 5 mM TCEP solution (equivalent to 10 times the antibody content) was added, and 320 μL of 50 mM PBS 7.0 and 71.05 μL of ultrapure water were added thereto at the same time. The mixture was reacted at 37 °C for 2 h.

Conjugation of antibody to linker-payload: The mixture described above was incubated at 4 °C for 10 min. The linker-payload LP2 prepared in Example 2 was dissolved in DMA and added to the mixture in a volume of 120 μL (equivalent to 15 times the antibody content), and the mixture was reacted at 22 °C for another 30 min.

Purification of antibody-drug conjugate: The reaction solution described above was purified by the method of Operation D in Example 5 to obtain the antibody-drug conjugate L1H5-LP2.

Characterization of antibody-drug conjugate: The obtained antibody-drug conjugate was characterized using Operation E ($\varepsilon_{D,280}$ = 5814 and $\varepsilon_{D,370}$ = 14742), Operation F, and Operation G in Example 5.

The concentration of the antibody-drug conjugate was calculated to be 6.28 mg/mL by the Operation E measurement, and the number of payloads conjugated on average per antibody was calculated to be 8.89 by the Operation E measurement. FIG. 4A shows a detection profile of aggregates, with an aggregate content of 1.89% for

antibody-drug conjugate L1H5-LP2, as measured by Operation F. FIG. 4B shows a hydrophobic chromatography detection profile of antibody-drug conjugate L1H5-LP2, with a retention time of 6.599 min for the antibody-drug conjugate L1H5-LP2, as measured by Operation G.

**Comparative Example 1: Preparation of Antibody-Drug Conjugate Reference ADC**

Reduction of antibody: The antibody was subjected to medium exchange using PBS7.0/EDTA according to Operation B (1.77 $mLmg^{-1}$ $cm^{-1}$ was used as the extinction coefficient of the antibody at 280 nm) and Operation C of Example 5, and the antibody concentration after the medium exchange was 9.44 mg/mL. To 0.037 mL of an aqueous solution of the reference antibody (see SEQ ID NOs: 69-72, U1-59 in patent US20190151328A1 for the sequence of the antibody), 3.27 μL of a 5 mM TCEP solution (equivalent to 7 times the antibody content) was added, and 14 μL of 50 mM PBS 7.0 and 15.66 μL mL of deionized water were added thereto at the same time. The mixture was reacted at 37 °C for 2 h.

Conjugation of antibody to linker-payload: The mixture described above was incubated at 4 °C for 10 min. The linker-payload GGFG-DXd (purchased from DC Chemicals, DC50025) was dissolved in DMA and added to the mixture in a volume of 3.27 μL (equivalent to 14 times the antibody content), and the mixture was reacted at 22 °C for another 30 min.

Purification of antibody-drug conjugate: The reaction solution described above was purified by the method of Operation D in Example 5 to obtain the antibody-drug conjugate reference ADC.

Characterization of antibody-drug conjugate: The obtained antibody-drug conjugate was characterized using Operation E ($\varepsilon_{D,280}$ = 5178 and $\varepsilon_{D,370}$ = 20217), Operation F, and Operation G in Example 5.

The concentration of the antibody-drug conjugate was calculated to be 3.10 mg/mL by the Operation B measurement, and the number of payloads conjugated on average per antibody was calculated to be 6.63 by the Operation E measurement. FIG. 23A shows a detection profile of aggregates, with an aggregate content of 2.04% for antibody-drug conjugate reference ADC, as measured by Operation F. FIG. 23B shows a hydrophobic chromatography detection profile of the antibody-drug conjugate reference ADC, with a retention time of 8.215 min for the antibody-drug conjugate reference antibody-DXd, as measured by Operation G.

Also, FIG. 22 shows a comparison of hydrophobicity measurements for the L1H5 antibody prepared in Example 4, the antibody-drug conjugate L1H5-LP3 prepared in Example 6, the reference antibody, and the antibody-drug conjugate reference ADC, and Table 3 shows retention time of each test samples. The results show shorter retention time for the L1H5 antibody and the L1H5-LP3 compared to the positive control reference antibody and the drug-antibody conjugate reference ADC, indicating that both the reference antibody and the drug-antibody conjugate reference ADC are more hydrophobic than the L1H5 antibody and the antibody-drug conjugate L1H5-LP3. It is expected that the L1H5 antibody and L1H5-LP3 have relatively high stability *in vivo* due to their weak hydrophobicity.

Table 3 Retention time of L1H5, L1H5-LP3, reference antibody, and reference ADC

| Antibody and ADC | Retention time (min) |
|---|---|
| Hu4O3 (L1H5) | 3.789 |
| Hu4O3-LP3 (L1H5-LP3) | 6.169 |
| Reference antibody | 4.516 |
| Reference ADC | 8.215 |

**Comparative Example 2: Preparation of Antibody-Drug Conjugate Reference Antibody-LP3**

Reduction of antibody: The antibody was subjected to medium exchange using PBS7.0/EDTA according to Operation B (1.77 $mLmg^{-1}$ $cm^{-1}$ was used as the extinction coefficient of the antibody at 280 nm) and Operation C of Example 5, and the antibody concentration after the medium exchange was 15.5 mg/mL. To 1.858 mL of an aqueous solution of the reference antibody (the sequence is the same as that in Comparative Example 1), 268.80 μL of a 5 mM TCEP solution (equivalent to 7 times the antibody content) was added, and 360 μL of 100 mM PBS 7.0 and 1113.14 μL of deionized water were added thereto at the same time. The mixture was reacted at 37 °C for 2 h.

Conjugation of antibody to linker-payload: The mixture described above was incubated at 4 °C for 10 min. The linker-payload LP3 was dissolved in DMA and added to the mixture in a volume of 268.80 μL (equivalent to 14 times the antibody content), and the mixture was reacted at 22 °C for another 30 min.

Purification of antibody-drug conjugate: The reaction solution described above was purified by the method of Operation D in Example 5 to obtain the antibody-drug conjugate reference antibody-LP3.

Characterization of antibody-drug conjugate: The obtained antibody-drug conjugate was characterized using Operation E (εD,280 = 5186 and $\varepsilon_{D,370}$ = 13688), Operation F, and Operation G in Example 5.

The concentration of the antibody-drug conjugate was calculated to be 8.45 mg/mL by the Operation E measurement, and the number of payloads conjugated on average per antibody was calculated to be 7.60 by the Operation E measurement. FIG. 24A shows a detection profile of aggregates, with an aggregate content of 1.92% for antibody-drug conjugate reference antibody-LP3, as measured by Operation F. FIG. 24B shows a hydrophobic chromatography detection profile of the antibody-drug conjugate reference antibody-LP3, with a retention time of 6.262 min for the antibody-drug conjugate reference antibody-MCE8, as measured by Operation G.

**Comparative Example 3: Preparation of Antibody-Drug Conjugate LIH5-DXd**

Reduction of antibody: The antibody was subjected to medium exchange using PBS7.0/EDTA according to Operation B (1.49 $mLmg^{-1}$ $cm^{-1}$ was used as the extinction coefficient of the antibody at 280 nm) and Operation C of Example 5, and the antibody concentration after the medium exchange was 11.44 mg/mL. To 0.59 mL of an aqueous solution of the L1H5 antibody, 90 μL of a 5 mM TCEP solution (equivalent to 10 times the antibody content) was added, and 180 μL of 50 mM PBS 7.0 and 39.97 μL of deionized water were added thereto at the same time. The mixture was reacted at 37 °C for 2 h.

Conjugation of antibody to linker-payload: The mixture described above was incubated at 4 °C for 10 min. The linker-payload GGFG-DXd (purchased from DC Chemicals, DC50025) was dissolved in DMA and added to the mixture in a volume of 67.50 μL (equivalent to 15 times the antibody content), and the mixture was reacted at 22 °C for another 30 min.

Purification of antibody-drug conjugate: The reaction solution described above was purified by the method of Operation D in Example 5 to obtain the antibody-drug conjugate L1H5-DXd.

Characterization of antibody-drug conjugate: The obtained antibody-drug conjugate was characterized using Operation E ($\varepsilon_{D,280}$ = 5178 and $\varepsilon_{D,370}$ = 20217), Operation F, and Operation G in Example 5.

The concentration of the antibody-drug conjugate was calculated to be 5.17 mg/mL by the Operation E measurement, and the number of payloads conjugated on average per antibody was calculated to be 6.54 by the Operation E measurement. FIG. 25A shows a detection profile of aggregates, with an aggregate content of 2.02% for antibody-drug conjugate L1H5-DXd, as measured by Operation F. FIG. 25B shows a hydrophobic chromatography detection profile of antibody-drug conjugate L1H5-DXd, with a retention time of 7.807 min for the antibody-drug conjugate L1H5-DXd, as measured by Operation G.

**Comparative Example 4: Preparation of Antibody-Drug Conjugate Dxd Isotype Control ADC**

Reduction of antibody: The antibody was subjected to medium exchange using PBS7.0/EDTA according to Operation B (1.35 $mLmg^{-1}$ $cm^{-1}$ was used as the extinction coefficient of the antibody at 280 nm) and Operation C of Example 5, and the antibody concentration after the medium exchange was 10 mg/mL. To 2.45 mL of an aqueous solution of the isotype control antibody human IgG (purchased from Beijing Solarbio Science & Technology Co., Ltd., product model SP001), 326.67 μL of a 5 mM TCEP solution (equivalent to 10 times the antibody content) was added, and 700 μL of 50 mM PBS 7.0 and 23.33 μL of deionized water were added thereto at the same time. The mixture was reacted at 37 °C for 2 h.

Conjugation of antibody to linker-payload: The mixture described above was incubated at 4 °C for 10 min. The linker-payload GGFG-DXd was dissolved in DMA and added to the mixture in a volume of 294 μL (equivalent to 18 times the antibody content), and the mixture was reacted at 22 °C for another 30 min.

Purification of antibody-drug conjugate: The reaction solution described above was purified by the method of Operation D in Example 5 to obtain the antibody-drug conjugate Dxd isotype control ADC.

Characterization of antibody-drug conjugate: The obtained antibody-drug conjugate was characterized using Operation E ($\varepsilon_{D,280}$ = 5178 and $\varepsilon_{D,370}$ = 20217), Operation F, and Operation G in Example 5.

The concentration of the antibody-drug conjugate was calculated to be 8.20 mg/mL by the Operation E measurement, and the number of payloads conjugated on average per antibody was calculated to be 7.08 by the Operation E measurement. FIG. 26A shows a detection profile of aggregates, with an aggregate content of 4.25% for antibody-drug conjugate Dxd isotype control ADC, as measured by Operation F. FIG. 26B shows a hydrophobic chromatography detection profile of antibody-drug conjugate Dxd isotype control ADC, with a retention time of 8.222 min for the antibody-drug conjugate Dxd isotype control ADC, as measured by Operation G.

**Comparative Example 5: Preparation of Antibody-Drug Conjugate LP3 Isotype Control ADC**

Reduction of antibody: The antibody was subjected to medium exchange using PBS7.0/EDTA according to Operation B (1.35 $mLmg^{-1}$ $cm^{-1}$ was used as the extinction coefficient of the antibody at 280 nm) and Operation C of Example 5, and the antibody concentration after the medium exchange was 10 mg/mL. To 4 mL of an aqueous solution of the isotype control antibody human IgG, 672 μL of a 5 mM TCEP solution (equivalent to 7 times the antibody content) was

added, and 1440 μL of 50 mM PBS 7.0 and 1088 μL of deionized water were added thereto at the same time. The mixture was reacted at 37 °C for 2 h.

Conjugation of antibody to linker-payload: The mixture described above was incubated at 4 °C for 10 min. The linker-payload LP3 was dissolved in DMA and added to the mixture in a volume of 672 μL (equivalent to 14 times the antibody content), and the mixture was reacted at 22 °C for another 30 min.

Purification of antibody-drug conjugate: The reaction solution described above was purified by the method of Operation D in Example 5 to obtain the antibody-drug conjugate LP3 isotype control ADC.

Characterization of antibody-drug conjugate: The obtained antibody-drug conjugate was characterized using Operation E ($\varepsilon_{D,280}$ = 5186 and $\varepsilon_{D,370}$ = 13688), Operation F, and Operation G in Example 5.

The concentration of the antibody-drug conjugate was calculated to be 7.42 mg/mL by the Operation E measurement, and the number of payloads conjugated on average per antibody was calculated to be 7.28 by the Operation E measurement. FIG. 27A shows a detection profile of aggregates, with an aggregate content of 5.37% for antibody-drug conjugate LP3 isotype control ADC, as measured by Operation F. FIG. 27B shows a hydrophobic chromatography detection profile of antibody-drug conjugate LP3 isotype control ADC, with a retention time of 6.450 min for the antibody-drug conjugate LP3 isotype control ADC, as measured by Operation G.

### Assay Example 1: Flow Cytometry of Mu4O3 Antibody and Cells Expressing Different Amounts of HER3

On a culture dish, 4 cell lines, MCF-7 (human breast cancer cells, purchased from National Collection of Authenticated Cell Cultures of Chinese Academy of Sciences), SW620 (human colon cancer cells, purchased from Zhejiang Meisen Cell Technology Co., Ltd.), SK-BR-3 (human breast cancer cells, purchased from Shanghai Xunqing Biotechnology Co., Ltd.), and MDA-MB-231 (human breast cancer cells, purchased from Saibaikang (Shanghai) Biotechnology Co., Ltd.) were separately digested with trypsin, and the digested cells were centrifuged at 1000 rpm for 5 min and then blocked with 10% goat serum (purchased from Beijing Solarbio Science & Technology Co., Ltd., Catalog No. SL038) for 30 min. After blocking, the Mu4O3 antibody prepared in Example 4 was added at a final concentration of 20 μg/mL, incubated for 1 h, and washed 2 times with phosphate buffered saline (PBS, pH 7.2-7.4). FITC-labeled goat anti-mouse secondary antibody (purchased from Jackson ImmunoResarch, Catalog No. 111-545-003) was added at a ratio of 1:500, and the mixture was incubated for 1 h and washed 2 times with PBS. The results were analyzed using a flow cytometer.

The results are shown in FIG. 5. The Mu4O3 antibody was positive in all of the 3 HER3 positive cell lines (MCF-7, SW620, and SK-BR-3); in HER3 negative cells (MDA-MB-231), the assay result was negative and no fluorescence signal was detected. The results show that the Mu4O3 antibody is specific.

### Assay Example 2: Immunofluorescence Assay of Mu4O3 Antibody in HER3-GFP Tag Overexpressing 293T Cells

293T cells (purchased from National Collection of Authenticated Cell Cultures of Chinese Academy of Sciences) were inoculated onto a 24-well cell culture plate to achieve the cell density of 70%, and a human HER3 recombinant plasmid HER3-GFP Tag (purchased from Hunan Keai Medical Devices Co., Ltd., Catalog No. G109862) with a GFP label was transfected into the cells by a cationic polymer-polyethyleneimine (PEI) transfection method. The specific method was: 0.5 μg of the plasmid and 1.5 μg of PEI were mixed in 500 μL of Opti-MEM medium (purchased from Thermo Fisher Scientific (China) Co., Ltd., Catalog No. 31985070), and after stabilization at room temperature for 15 min, the mixture was slowly added to cell culture wells and returned to a 37 °C incubator. After 24 h, the cells were washed with PBS and fixed with 4% tissue cell fixation solution (PFA) for 10 min. After washing with PBS, the cells were blocked with 10% goat serum (purchased from Beijing Solarbio Science & Technology Co., Ltd., Catalog No. SL038) for 30 min. After blocking, the Mu4O3 antibody was added at a final concentration of 20 μg/mL, incubated for 1 h, and washed 2 times with PBS. APC-labeled goat anti-mouse secondary antibody (purchased from Jackson ImmunoResarch, Catalog No. 111-585-003) was added at a ratio of 1:500, and the mixture was incubated for 1 h and washed 2 times with PBS. DAPI (4',6-diamidino-2-phenylindole) solution was added, and the mixture was incubated for 5 min, washed 2 times with PBS, and subjected to fluorescence microscopy observation and image capture. FIG. 6 shows immunofluorescence images of the Mu4O3 antibody in HER3-GFP Tag overexpressing 293T cells. The results show significant co-localization features of HER3 protein and Mu4O3 antibody staining.

In FIG. 6, the picture labeled GFP only shows green fluorescence, indicating successful transfection of HER3 protein into 293T cells with expression both in the cytoplasm and in the cell membrane; the picture labeled Mu4O3 only shows red fluorescence, indicating that the Mu4O3 antibody successfully stained the overexpressing cells, with the staining localized to the membrane; the picture labeled DAPI only shows blue fluorescence, indicating staining for nuclei; the picture labeled MERGED is a merged image obtained by superimposing the 3 separate staining images described above, in which part of the membrane-localized green light can completely overlap with the membrane-localized red light,

and the fluorescence intensity remains the same, thus demonstrating that the Mu4O3 antibody specifically stains HER3-overexpressing protein. Moreover, no red fluorescence staining of Mu4O3 binding is found in the cells without overexpression (blue staining only in the cell nucleus, no green luminescence), indicating that the Mu4O3 antibody has no nonspecific staining.

**Assay Example 3: *In Vitro* Killing Assay of SW620 Cell by Mu4O3 Antibody**

SW620 cells (human colon cancer cells, purchased from Zhejiang Meisen Cell Technology Co., Ltd.) were cultured to reach a cell density of 80%. The cells were collected and plated in a 96-well plate, and the cell density was adjusted to 2-5 $\times$ $10^4$/mL. The cells were plated at 100 $\mu$L per well. The Mu4O3 antibody prepared in Example 4 or mouse anti-IgG (MouseIgG, purchased from Abmart Shanghai Co., Ltd.) was serially 3-fold diluted from 300 nM as the starting concentration and added to the cell culture after the dilution was complete, 2 $\mu$g/mL MMAE-conjugated goat anti-mouse secondary antibody (purchased from Abmart Shanghai Co., Ltd., Catalog No. B30008) was added thereto at the same time, and the mixture was cultured and left to stand for 5 days. During this period, apoptosis was observed periodically. After 5 days, 15 $\mu$L of CCK-8 stock solution was directly added to the 96-well plate, and the absorbance at 450 nm was measured after the mixture was reacted for 0.5 to 2 h in an incubator at 37 °C. A cell survival curve was plotted according to the assay results and the antibody dilution gradient.

The results are shown in FIG. 7, which shows that the Mu4O3 antibody has significant specific cell killing effect.

**Assay Example 4: Flow Affinity Assay of Hu4O3 Antibody Candidate Molecule in MDA-MB-453 and SW620 Cell Lines**

On a culture dish, SW620 (human colon cancer cells) and MDA-MB-453 (human breast cancer cells, purchased from Zhejiang Meisen Cell Technology Co., Ltd.) were separately digested with trypsin, and the digested cells were centrifuged at 1000 rpm for 5 min and then blocked with 10% goat serum (purchased from Beijing Solarbio Science & Technology Co., Ltd., Catalog No. SL038) for 30 min. The Hu4O3 antibody was serially 3-fold diluted from 500 nM as the starting concentration, and after completion, the serially diluted Hu4O3 antibody was added to the cells for incubation for 1 h. After the mixture was washed 2 times with PBS, FITC-labeled goat anti-human secondary antibody (purchased from Jackson ImmunoResarch, Catalog No. 109-545-008) was added at a ratio of 1:500, and the mixture was incubated for 1 h and washed 2 times with PBS. The results were analyzed using a flow cytometer.

Figs. 8 and 9 show flow affinity assay data of Hu4O3 antibody candidate molecules L1H4, L1H5, L2H4, L2H5, L4H4, L4H5, and L4H7 in MDA-MB-453 and SW620 cell lines. The results show that all candidate molecules bind specifically to both cell lines with varying binding affinities. L1H4 and L1H5 exhibited superior affinity for MDA-MB-453 cells with $EC_{50}$ at 71.5 nM and 121.7 nM, respectively. L1H4, L1H5, L4H4, and L4H7 exhibited superior affinity for SW620 cells, with $EC_{50}$ at 193.5 nM, 146.7 nM, 90.0 nM, and 21.9 nM, respectively, followed by L2H4 with $EC_{50}$ at 1203 nM.

**Assay Example 5: *In Vitro* Cell Proliferation Inhibition Assay of L1H5 Antibody and Reference Antibody on SK-BR-3 Cell Line**

SK-BR-3 (human breast cancer cells) were cultured to reach a cell density of 80%. The cells were collected and plated in a 96-well plate, and the cell density was adjusted to 2-5 $\times$ $10^4$/mL. The cells were plated at 100 $\mu$L per well. The antibody was added to the cell culture at a final concentration of 100 $\mu$g/mL, and the mixture was cultured and left to stand for 5 days. During this period, apoptosis was observed periodically. After 5 days, 15 $\mu$L of CCK-8 stock solution was added to the 96-well plate, and the absorbance at 450 nm was measured after the mixture was reacted for 0.5 to 2 h in an incubator at 37 °C. The survival rate of the cells was calculated according to the comparison of the assay results and blank control.

FIG. 10 shows the cell proliferation inhibition rate of the L1H5 antibody, the reference antibody, and the blank control prepared in Example 4 in the SK-BR-3 cell line. The results show that the *in vitro* cell proliferation inhibition effect of L1H5 on SK-BR-3 is better than that of the reference antibody.

**Assay Example 6: *In Vivo* Tumor Inhibition Assay in Mouse Breast Cancer CDX Model**

Balb/c nude mice, 6 weeks old, were purchased from Jiangsu Gempharmatech Co., Ltd. Five million BT-474 cells (human ductal breast cancer cells) were subcutaneously inoculated per mouse. When the average tumor volume was about 150 $mm^3$, L1H5 prepared in Example 4, the reference antibody (positive control), and a blank control PBS (negative control) were separately subcutaneously injected at a dose of 20 mg/kg. The treatment was performed once per week for two weeks in total. Tumor volume was measured twice a week using a vernier caliper after administration and calculated according to the following formula: TV = (length $\times$ width)$^2$/2.

FIG. 11 shows curves of changes of tumor volumes over time in the BT-474 breast cancer mouse model *in vivo.*

The results show that L1H5 is able to inhibit tumor growth, which is comparable to the positive reference antibody.

**Assay Example 7: *In Vitro* Killing Assay of SW620 Cell by Hu4O3 Antibody Candidate Molecule-Drug Conjugate**

SW620 (human colon cancer cells) were cultured to reach a cell density of 80%. The cells were collected and plated in a 96-well plate, and the cell density was adjusted to 2-5 $\times$ $10^4$/mL. The cells were plated at 100 $\mu$L per well. The ADC molecules were serially 3-fold diluted from 300 nM as the starting concentration and added to the cell culture after the dilution was complete, and the mixture was cultured and left to stand for 5 days. During this period, apoptosis was observed periodically. After 5 days, 15 $\mu$L of CCK-8 stock solution was added to the 96-well plate, and the absorbance at 450 nm was measured after the mixture was reacted for 0.5 to 2 h in an incubator at 37 °C. A cell survival curve was plotted according to the assay results and the ADC dilution gradient.

FIG. 12 shows cell survival curves of the antibody-drug conjugates L1H4-LP1, L1H5-LP1, and L4H4-LP1 prepared in Example 7 and the antibody-drug conjugate positive control reference ADC prepared in Comparative Example 1 in the SW620 cell line. The results show that the *in vitro* cell killing effect of L1H4-LP1, L1H5-LP1, and L4H4-LP1 on the SW620 cell line is better than that of the reference ADC, and the cell proliferation inhibition effect of L1H5-LP1 on the SW620 cell line is optimal.

**Assay Example 8: *In Vitro* Cell Killing Assay of L1H5 Antibody, L1H5-LP3, Reference Antibody, and Reference ADC on SK-BR-3 and BXPC-3 Cell Lines**

SK-BR-3 (human breast cancer cells) and BXPC-3 (human pancreatic cancer cells, purchased from National Collection of Authenticated Cell Cultures of Chinese Academy of Sciences) were cultured to reach a cell density of 80%. The cells were collected and plated in a 96-well plate, and the cell density was adjusted to 2-5 $\times$ $10^4$/mL. The cells were plated at 100 $\mu$L per well. The antibody and ADC molecules were both serially 3-fold diluted from 300 nM as the starting concentration and added to the cell culture after the dilution was complete, and the mixture was cultured and left to stand for 5 days. During this period, apoptosis was observed periodically. After 5 days, 15 $\mu$L of CCK-8 stock solution was added to the 96-well plate, and the absorbance at 450 nm was measured after the mixture was reacted for 0.5 to 2 h in an incubator at 37 °C. A cell survival curve was plotted according to the assay results and the ADC and antibody dilution gradient.

FIGs. 13 and 14 show cell survival curves of the L1H5 antibody prepared in Example 4, the antibody-drug conjugate L1H5-LP3 prepared in Example 6, the reference antibody (positive control), and the antibody-drug conjugate reference ADC prepared in Comparative Example 1 (positive control) in the SK-BR-3 cell line and the BXPC-3 cell line, respectively. The results show that the *in vitro* cell killing effect of the L1H5-LP3 on the two cell lines is significantly better than that of the reference ADC, the cell proliferation inhibition effect of the L1H5 antibody on the SK-BR-3 cell line is better than that of the reference antibody, and the L1H5 antibody and the reference antibody do not show an *in vitro* killing effect on the BXPC-3 cell line.

**Assay Example 9: *In Vitro* Killing Assay of HCT-15 Cell by L1H5-LP1 and L1H5-LP2 Antibodies**

HCT-15 (human colon cancer cells) were cultured to reach a cell density of 80%. The cells were collected and plated in a 96-well plate, and the cell density was adjusted to 2-5 $\times$ $10^4$/mL. The cells were plated at 100 $\mu$L per well. The ADC molecules were serially 3-fold diluted from 300 nM as the starting concentration and added to the cell culture after the dilution was complete, and the mixture was cultured and left to stand for 5 days. During this period, apoptosis was observed periodically. After 5 days, 15 $\mu$L of CCK-8 stock solution was added to the 96-well plate, and the absorbance at 450 nm was measured after the mixture was reacted for 0.5 to 2 h in an incubator at 37 °C. A cell survival curve was plotted according to the assay results and the ADC dilution gradient.

FIG. 15 shows cell survival curves of the antibody-drug conjugates L1H5-LP1 prepared in Example 7, L1H5-LP2 prepared in Example 8, and the antibody-drug conjugate positive control reference ADC prepared in Comparative Example 1 in the HCT-15 cell line. The results show that the *in vitro* cell killing effect of L1H5-LP1 and L1H5-LP2 on the HCT-15 cell line is significantly better than that of the reference ADC.

**Assay Example 10: *In Vivo* Tumor Inhibition Assay in Mouse Colon Cancer CDX Model**

Balb/c nude mice, 6 weeks old, were purchased from Jiangsu Gempharmatech Co., Ltd. Five million HCT-15 cells were subcutaneously inoculated per mouse. When the average tumor volume was about 150 $mm^3$, the reference ADC prepared in Comparative Example 1, L1H5-LP2 prepared in Example 8, and L1H5-LP1 prepared in Example 7 were separately intravenously injected at a dose of 10 mg/kg. Moreover, a blank control (negative control) group was set, in which an equal dose of PBS was intravenously injected. The administration was performed once per week for three times

in total. Tumor volume was measured twice a week using a vernier caliper and calculated according to the following formula: TV = (length × width)$^2$/2. FIG. 16 shows curves of changes of tumor volumes over time in the HCT-15 colon cancer mouse model *in vivo.* The results show that both L1H5-LP1 and L1H5-LP2 are able to inhibit tumor growth, which is slightly better than the positive reference ADC.

### Assay Example 11: *In Vivo* Tumor Inhibition Assay in Mouse Colon Cancer PDX Model

NU/NU mice, female, 6 weeks old, 18-21 g, were purchased from Beijing Vital River Laboratory Animal Technology Co., Ltd. The mice were inoculated subcutaneously with colon cancer tumor masses (specimen number of colon cancer tissue was 361795, from Xi'an Lide Biotechnology Co., Ltd.). When the average tumor volume reached 150 mm$^3$, the reference ADC prepared in Comparative Example 1, L1H5-LP3 prepared in Example 6, L1H5-LP1 prepared in Example 7, the Dxd isotype control ADC prepared in Comparative Example 4, and the LP3 isotype control ADC prepared in Comparative Example 5 were separately intravenously injected at a dose of 10 mg/kg. Moreover, a blank control (negative control) group was set, in which an equal dose of PBS was intravenously injected. The administration was performed once per week for 3 times in total. Tumor volume was measured twice a week using a vernier caliper after administration and calculated according to the following formula: TV = (length × width)$^2$/2.

FIG. 17 shows curves of changes of tumor volumes over time in the colon cancer mouse model *in vivo.* The results show that L1H5-LP3 and L1H5-LP1 significantly inhibit tumor growth compared to the positive control reference ADC.

### Assay Example 12: *In Vivo* Tumor Inhibition Assay in Mouse EGFR-TKI-Resistant Lung Adenocarcinoma PDX Model

NU/NU mice, female, 6 weeks old, 18-21 g, were purchased from Beijing Vital River Laboratory Animal Technology Co., Ltd. Each mouse was inoculated subcutaneously with EGFR-TKI-resistant lung adenocarcinoma tumor masses (specimen number of EGFR-TKI-resistant lung adenocarcinoma tissue was 0025-200717, from Xi'an Lide Biotechnology Co., Ltd.). When the average tumor volume reached 150 mm$^3$, the reference ADC prepared in Comparative Example 1, L1H5-LP3 prepared in Example 6, L1H5-LP1 prepared in Example 7, the Dxd isotype control ADC prepared in Comparative Example 4, and the LP3 isotype control ADC prepared in Comparative Example 5 were separately intravenously injected at a dose of 10 mg/kg. Moreover, a vehicle control group was set, in which an equal dose of PBS was intravenously injected. The administration was performed once per week for 2 times in total. Tumor volume was measured twice a week using a vernier caliper after administration and calculated according to the following formula: TV = (length × width)$^2$/2.

FIG. 18 shows curves of changes of tumor volumes over time in the EGFR-TKI-resistant lung adenocarcinoma mouse model *in vivo.* The results show that L1H5-LP3 and L1H5-LP1 significantly inhibit tumor growth compared to the positive control reference ADC.

### Assay Example 13: *In Vivo* Tumor Inhibition Assay in Mouse Colon Cancer CDX Model

Balb/c nude mice, 6 weeks old, were purchased from Jiangsu Gempharmatech Co., Ltd. Five million SW620 cells were subcutaneously inoculated per mouse. When the average tumor volume was about 150 mm$^3$, L1H5-DXd prepared in Comparative Example 3, the reference antibody-LP3 prepared in Comparative Example 2, L1H5-LP3 prepared in Example 6, the reference ADC prepared in Comparative Example 1, and negative control PBS were separately intravenously injected at a dose of 10 mg/kg. The treatment was performed once per week for three weeks in total. Tumor volume was measured twice a week using a vernier caliper after administration and calculated according to the following formula: TV = (length × width)$^2$/2.

FIG. 19 shows curves of changes of tumor volumes over time in the mouse colon cancer CDX model *in vivo.* It can be seen that L1H5-DXd, L1H5-LP3, and the reference antibody-LP3 are all superior to the positive control reference ADC.

FIG. 20 is a partially enlarged view of FIG. 19 with the negative control PBS group taken away. As is evident from the figure, the four active molecules are arranged from high to low according to the overall tumor inhibition effect *in vivo* as L1H5-LP3 > L1H5-DXd > reference antibody-LP3 > reference ADC.

### Assay Example 14: *In Vivo* Tumor Inhibition Assay in Mouse Pancreatic Cancer CDX Model

NU/NU mice, female, 6 weeks old, 18-21 g, purchased from Beijing Vital River Laboratory Animal Technology Co., Ltd. Each mouse was inoculated subcutaneously with pancreatic cancer tumor masses (specimen number of pancreatic cancer tissue was 0033-361319, from Xi'an Lide Biotechnology Co., Ltd.). When the average tumor volume reached 150 mm$^3$, the reference ADC prepared in Comparative Example 1, L1H5-LP3 prepared in Example 6, L1H5-LP1

prepared in Example 7, the Dxd isotype control ADC prepared in Comparative Example 4, and the LP3 isotype control ADC prepared in Comparative Example 5 were separately intravenously injected at a dose of 10 mg/kg. Moreover, a blank control group was set, in which an equal dose of PBS was intravenously injected. The administration was performed once per week for three times in total. Tumor volume was measured twice a week using a vernier caliper and calculated according to the following formula: TV = (length $\times$ width)$^2$/2.

FIG. 21 shows *in vivo* tumor inhibition curves in the pancreatic cancer mouse model. The results show that the positive control reference ADC, L1H5-LP3, and L1H5-LP1 all can significantly inhibit tumor growth at the initial stage of administration; the tumor inhibition effects of L1H5-LP3 and L1H5-LP1 are significantly better than those of the positive control reference ADC during the administration period; moreover, their tumor-inhibition duration was significantly longer than that of the reference ADC.

The antibody-drug conjugate prepared in the embodiments of the present disclosure has more excellent *in vivo* anti-tumor effect than the ADC conjugated with GGFG-DXd, and L1H5-LP3 shows *in vivo* efficacy better than that of the reference ADC in a plurality of tumor models; moreover, L1H5-LP3 shows a more lasting tumor inhibition effect in individual models. Also, L1H5-LP3 has a similar maximum tolerated dose (MTD) as GGFG-Dxd ADC, which is expected to have a higher therapeutic window than the ADC conjugated with GGFG-Dxd. The antibody-drug conjugate provided by the present disclosure has excellent safety.

## Claims

1. An anti-HER3 antibody or an antigen-binding fragment thereof, comprising at least one light chain variable region (VL) and heavy chain variable region (VH), wherein

   CDR1 of the VL comprises the amino acid sequence set forth in SEQ ID NO: 1, CDR2 of the VL comprises the amino acid sequence set forth in SEQ ID NO: 2, and CDR3 of the VL comprises the amino acid sequence set forth in SEQ ID NO: 3;
   CDR1 of the VH comprises the amino acid sequence set forth in SEQ ID NO: 4; CDR2 of the VH comprises an amino acid sequence having no more than 5, 4, 3, 2, 1, or 0 mutations compared to the amino acid sequence set forth in SEQ ID NO: 5; CDR3 of the VH comprises the amino acid sequence set forth in SEQ ID NO: 6.

2. The anti-HER3 antibody or the antigen-binding fragment thereof according to claim 1, wherein the CDR2 of the VH comprises one or more mutations selected from (a) and (b):

   (a) C52N, T62N, T66K, and G67S;
   (b) C52Y, T62N, T66K, and G67S.

3. The anti-HER3 antibody or the antigen-binding fragment thereof according to claim 1 or 2, wherein the CDR2 of the VH comprises mutations of (a) or (b):

   (a) C52N, T62N, T66K, and G67S;
   (b) C52Y, T62N, T66K, and G67S.

4. The anti-HER3 antibody or the antigen-binding fragment thereof according to any one of claims 1-3, wherein the combination of VH and VL contained in the anti-HER3 antibody or the antigen-binding fragment thereof is selected from one of groups (c)-(j):

   (c) the VL and VH respectively comprise amino acid sequences having at least 85%, 90%, 95%, 96%, 97%, 98%, or 99% identity to the amino acid sequences set forth in SEQ ID NO: 9 and SEQ ID NO: 10;
   (d) the VL and VH respectively comprise amino acid sequences having at least 85%, 90%, 95%, 96%, 97%, 98%, or 99% identity to the amino acid sequences set forth in SEQ ID NO: 11 and SEQ ID NO: 14;
   (e) the VL and VH respectively comprise amino acid sequences having at least 85%, 90%, 95%, 96%, 97%, 98%, or 99% identity to the amino acid sequences set forth in SEQ ID NO: 11 and SEQ ID NO: 15;
   (f) the VL and VH respectively comprise amino acid sequences having at least 85%, 90%, 95%, 96%, 97%, 98%, or 99% identity to the amino acid sequences set forth in SEQ ID NO: 12 and SEQ ID NO: 14;
   (g) the VL and VH respectively comprise amino acid sequences having at least 85%, 90%, 95%, 96%, 97%, 98%, or 99% identity to the amino acid sequences set forth in SEQ ID NO: 12 and SEQ ID NO: 15;
   (h) the VL and VH respectively comprise amino acid sequences having at least 85%, 90%, 95%, 96%, 97%, 98%, or 99% identity to the amino acid sequences set forth in SEQ ID NO: 13 and SEQ ID NO: 14;

(i) the VL and VH respectively comprise amino acid sequences having at least 85%, 90%, 95%, 96%, 97%, 98%, or 99% identity to the amino acid sequences set forth in SEQ ID NO: 13 and SEQ ID NO: 15; and
(j) the VL and VH respectively comprise amino acid sequences having at least 85%, 90%, 95%, 96%, 97%, 98%, or 99% identity to the amino acid sequences set forth in SEQ ID NO: 13 and SEQ ID NO: 16.

**5.** The anti-HER3 antibody or the antigen-binding fragment thereof according to any one of claims 1-4, wherein the combination of VH and VL contained in the anti-HER3 antibody or the antigen-binding fragment thereof is selected from one of groups (c)-(j):

(c) the VL comprises the amino acid sequence set forth in SEQ ID NO: 9 and conservatively substituted variants thereof, and the VH comprises the amino acid sequence set forth in SEQ ID NO: 10 and conservatively substituted variants thereof;
(d) the VL comprises the amino acid sequence set forth in SEQ ID NO: 11 and conservatively substituted variants thereof, and the VH comprises the amino acid sequence set forth in SEQ ID NO: 14 and conservatively substituted variants thereof;
(e) the VL comprises the amino acid sequence set forth in SEQ ID NO: 11 and conservatively substituted variants thereof, and the VH comprises the amino acid sequence set forth in SEQ ID NO: 15 and conservatively substituted variants thereof;
(f) the VL comprises the amino acid sequence set forth in SEQ ID NO: 12 and conservatively substituted variants thereof, and the VH comprises the amino acid sequence set forth in SEQ ID NO: 14 and conservatively substituted variants thereof;
(g) the VL comprises the amino acid sequence set forth in SEQ ID NO: 12 and conservatively substituted variants thereof, and the VH comprises the amino acid sequence set forth in SEQ ID NO: 15 and conservatively substituted variants thereof;
(h) the VL comprises the amino acid sequence set forth in SEQ ID NO: 13 and conservatively substituted variants thereof, and the VH comprises the amino acid sequence set forth in SEQ ID NO: 14 and conservatively substituted variants thereof;
(i) the VL comprises the amino acid sequence set forth in SEQ ID NO: 13 and conservatively substituted variants thereof, and the VH comprises the amino acid sequence set forth in SEQ ID NO: 15 and conservatively substituted variants thereof;
(j) the VL comprises the amino acid sequence set forth in SEQ ID NO: 13 and conservatively substituted variants thereof, and the VH comprises the amino acid sequence set forth in SEQ ID NO: 16 and conservatively substituted variants thereof.

**6.** The anti-HER3 antibody or the antigen-binding fragment thereof according to any one of claims 1-5, wherein the anti-HER3 antibody or the antigen-binding fragment thereof specifically binds to human HER3.

**7.** A nucleic acid, encoding the anti-HER3 antibody or the antigen-binding fragment thereof according to any one of claims 1-6.

**8.** A vector, comprising the nucleic acid according to claim 7.

**9.** A host cell, comprising the nucleic acid according to claim 7 and/or the vector according to claim 8.

**10.** An antibody-drug conjugate having the structure of formula I,

Ab-(L-D)n (I)

or an isomer, an isotopic variant, a pharmaceutically acceptable salt, a prodrug, or a solvate thereof, or a combination thereof,
wherein Ab is the anti-HER3 antibody or the antigen-binding fragment thereof according to any one of claims 1-6;
L is a linker covalently linked to Ab and D;
D is a payload;
n is a number between 1 and 10.

**11.** The antibody-drug conjugate according to claim 10, wherein the linker is a cleavable linker and a non-cleavable linker.

12. The antibody-drug conjugate according to claim 10 or 11, wherein the linker comprises a cleavable peptide.

13. The antibody-drug conjugate according to claim 12, wherein the cleavable peptide is cleavable by an enzyme.

14. The antibody-drug conjugate according to claim 13, wherein the enzyme comprises cathepsin B.

15. The antibody-drug conjugate according to any one of claims 10-14, wherein the cleavable peptide or L comprises an amino acid unit.

16. The antibody-drug conjugate according to claim 15, wherein the amino acid unit comprises a dipeptide, tripeptide, tetrapeptide, or pentapeptide.

17. The antibody-drug conjugate according to claim 16, wherein the amino acid unit is selected from: any or a combination of Val-Cit, Val-Ala, Glu-Val-Cit, Ala-Ala-Asn, Gly-Val-Cit, Gly-Gly-Gly, and Gly-Gly-Phe-Gly.

18. The antibody-drug conjugate according to any one of claims 10-17, wherein the linker comprises a self-immolative spacer.

19. The antibody-drug conjugate according to claim 18, wherein the self-immolative spacer comprises *p*-aminobenzoxy carbonyl (PABC) or *p*-aminobenzyl (PAB).

20. The antibody-drug conjugate according to any one of claims 12-19, wherein the cleavable peptide is spliced directly to the self-immolative spacer.

21. The antibody-drug conjugate according to any one of claims 10-20, wherein the linker comprises the structure of $-L_1-L_2-L_3-$, wherein

$L_1$ represents -(succinimid-3-yl-N)-$(CH_2)m^1$-C(=O)-, $-CH_2$-C(=O)-NH-$(CH_2)m^2$-C(=O)-, or -C(=O)-$(CH_2)m^3$-C(=O)-, wherein $m^1$ represents an integer of 2-8, $m^2$ represents an integer of 1-8, and $m^3$ represents an integer of 1-8;
$L_2$ represents an amino acid unit;
$L_3$ represents a self-immolative spacer.

22. The antibody-drug conjugate according to any one of claims 10-21, wherein L is selected from:

-(succinimid-3-yl-N)-$CH_2CH_2$-C(=O)-GGFG-PABC-;
-(succinimid-3-yl-N)-$CH_2CH_2CH_2CH_2CH_2$-C(=O)-GGFG-PABC-;
-(succinimid-3-yl-N)-$CH_2CH_2CH_2CH_2CH_2$-C(=O)-GGFG-NH-PABC-;
-(succinimid-3-yl-N)-$CH_2CH_2$-C(=O)-NH-$CH_2CH_2O$-$CH_2CH_2O$-$CH_2CH_2$-C(=O)-GGFG-PABC-;
-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-GGFG-PABC-;
$-CH_2$-C(=O)-NH-$CH_2CH_2$-C(=O)-GGFG-PABC-;
-C(=O)-$CH_2CH_2CH_2CH_2CH_2CH_2$-C(=O)-GGFG-PABC-;
-(succinimid-3-yl-N)-$CH_2CH_2$-C(=O)-GGFG-NH-$CH_2CH_2$-C(=O)-;
-(succinimid-3-yl-N)-$CH_2CH_2$-C(=O)-GGFG-NH-$CH_2CH_2CH_2$-C(=O)-;
-(succinimid-3-yl-N)-$CH_2CH_2CH_2CH_2CH_2$-C(=O)-GGFG-NH-$CH_2CH_2$-C(=O)-;
-(succinimid-3-yl-N)-$CH_2CH_2CH_2CH_2CH_2$-C(=O)-GGFG-NH-$CH_2CH_2CH_2$-C(=O)-;
-(succinimid-3-yl-N)-$CH_2CH_2CH_2CH_2CH_2$-C(=O)-GGFG-NH-$CH_2CH_2CH_2CH_2CH_2$-C(=O)-;
-(succinimid-3-yl-N)-$CH_2CH_2CH_2CH_2CH_2$-C(=O)-GGFG-NH-$CH_2$-O-$CH_2$-C(=O)-;
-(succinimid-3-yl-N)-$CH_2CH_2CH_2CH_2CH_2$-C(=O)-GGFG-NH-$CH_2CH_2$-O-$CH_2$-C(=O)-;
-(succinimid-3-yl-N)-$CH_2CH_2$-C(=O)-NH-$CH_2CH_2O$-$CH_2CH_2O$-$CH_2CH_2$-C(=O)-GGFG-NH-$CH_2CH_2CH_2$-C(=O)-;
-(succinimid-3-yl-N)-$CH_2CH_2$-C(=O)-NH-$CH_2CH_2O$-$CH_2CH_2O$-$CH_2CH_2$-C(=O)-GGFG-NH-$CH_2CH_2$-C(=O)-;
-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-GGFG-NH-$CH_2CH_2CH_2$-C(=O)-;
-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-GGFG-NH-$CH_2CH_2$-C(=O)-;
$-CH_2$-C(=O)-NH-$CH_2CH_2$-C(=O)-GGFG-NH-$CH_2CH_2CH_2$-C(=O)-;

$-C(=O)-CH_2CH_2CH_2CH_2CH_2CH_2-C(=O)-GGFG-NH-CH_2CH_2CH_2-C(=O)-$;
-(succinimid-3-yl-N)-$CH_2CH_2$-C(=O)-VA-PABC-;
-(succinimid-3-yl-N)-CH2CH2CH2CH2CH2-C(=O)-VA-PABC-;
-(succinimid-3-yl-N)-$CH_2CH_2CH_2CH_2CH_2$-C(=O)-VA-NH-PABC-;
-(succinimid-3-yl-N)-$CH_2CH_2$-C(=O)-NH-$CH_2CH_2O$-$CH_2CH_2O$-$CH_2CH_2$-C(=O)-VA-PABC-;
-(succinimid-3-yl-N)-CH2CH2-C(=O)-NH-$CH_2CH_2O$-$CH_2CH_2O$-$CH_2CH_2O$-$CH_2CH_2O$-$CH_2CH_2$-C(=O)-VA-PABC-;
-$CH_2$-C(=O)-NH-$CH_2CH_2$-C(=O)-VA-PABC-;
-C(=O)-$CH_2CH_2CH_2CH_2CH_2CH_2$-C(=O)-VA-PABC-;
-(succinimid-3-yl-N)-$CH_2CH_2$-C(=O)-VA-NH-$CH_2CH_2$-C(=O)-;
-(succinimid-3-yl-N)-$CH_2CH_2$-C(=O)-VA-NH-$CH_2CH_2CH_2$-C(=O)-;
-(succinimid-3-yl-N)-$CH_2CH_2CH_2CH_2CH_2$-C(=O)-VA-NH-$CH_2CH_2$-C(=O)-;
-(succinimid-3-yl-N)-$CH_2CH_2CH_2CH_2CH_2$-C(=O)-VA-NH-$CH_2CH_2CH_2$-C(=O)-;
-(succinimid-3-yl-N)-$CH_2CH_2CH_2CH_2CH_2$-C(=O)-VA-NH-$CH_2CH_2CH_2CH_2CH_2$-C(=O)-;
-(succinimid-3-yl-N)-$CH_2CH_2CH_2CH_2CH_2$-C(=O)-VA-NH-$CH_2$-O-$CH_2$-C(=O)-;
-(succinimid-3-yl-N)-$CH_2CH_2CH_2CH_2CH_2$-C(=O)-VA-NH-$CH_2CH_2$-O-$CH_2$-C(=O)-;
-(succinimid-3-yl-N)-$CH_2CH_2$-C(=O)-NH-$CH_2CH_2O$-$CH_2CH_2O$-$CH_2CH_2$-C(=O)-VA-NH-$CH_2CH_2CH_2$-C(=O)-;
-(succinimid-3-yl-N)-$CH_2CH_2$-C(=O)-NH-$CH_2CH_2O$-$CH_2CH_2O$-$CH_2CH_2$-C(=O)-VA-NH-$CH_2CH_2$-C(=O)-;
-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-VA-NH-$CH_2CH_2CH_2$-C(=O)-;
-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-VA-NH-$CH_2CH_2$-C(=O)-;
-$CH_2$-C(=O)-NH-$CH_2CH_2$-C(=O)-VA-NH-$CH_2CH_2CH_2$-C(=O)-; and
-C(=O)-$CH_2CH_2CH_2CH_2CH_2CH_2$-C(=O)-VA-NH-$CH_2CH_2CH_2$-C(=O)-.

**23.** The antibody-drug conjugate according to any one of claims 19-22, wherein the *p*-aminobenzyloxycarbonyl (PABC) or p-aminobenzyl (PAB) is linked to a poly-sarcosine (poly-N-methylglycine) residue or methylamino.

**24.** The antibody-drug conjugate according to any one of claims 10-23, wherein the linker is shown in formula II;

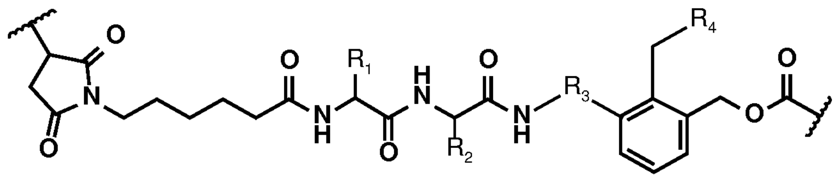

formula II

the linker of formula II is bonded with sulfhydryl reduced from an interchain disulfide chain of the antibody through a thioether bond via a succinimidyl group;
the carbonyl in the ester group of the linker of formula II is linked to the amino group in the payload; $R_1$ and $R_2$ are independently selected from hydrogen, methyl, and isopropyl groups;
$R_3$ represents $-(CR_5HCONH)n^1-(CH_2CONH)n^2-$ or a single bond, $R_5$ is selected from hydrogen and benzyl, $n^1$ represents an integer of 0-2, and $n^2$ represents an integer of 0-2;
$R_4$ represents methylamino or $-(NCH_3COCH_2)n^3-NCH_3COCH_3$, and $n^3$ represents an integer of 1-20.

**25.** The antibody-drug conjugate according to claim 24, wherein in the linker of formula II, $R_4$ represents $-(NCH_3COCH_2)n^3-NCH_3COCH_3$, and $n^3$ represents an integer of 8-15.

**26.** The antibody-drug conjugate according to claim 24 or 25, wherein in the linker of formula II, $R_4$ represents $-(NCH_3COCH_2)n^3-NCH_3COCH_3$, and $n^3$ represents an integer of 10-12.

**27.** The antibody-drug conjugate according to any one of claims 24-26, wherein in the linker of formula II, $R_3$ represents $-(CR_5HCONH)n^1-(CH_2CONH)n^2-$ or a single bond, $R_5$ is selected from benzyl, $n^1$ represents 1 or 2, and $n^2$ represents 1 or 2;
$R_4$ represents $-(NCH_3COCH_2)n^3-NCH_3COCH_3$, and $n^3$ represents an integer of 8-15.

**28.** The antibody-drug conjugate according to any one of claims 24-27, wherein in the linker of formula II, $R_3$ represents a single bond; $R_4$ represents $-(NCH_3COCH_2)n^3-NCH_3COCH_3$, and $n^3$ represents an integer of 8-15.

**29.** The antibody-drug conjugate according to any one of claims 24-28, wherein in the linker of formula II, $R_3$ represents a single bond; $R_4$ represents methylamino.

**30.** The antibody-drug conjugate according to any one of claims 10-29, wherein the linker is selected from one or more of the following groups,

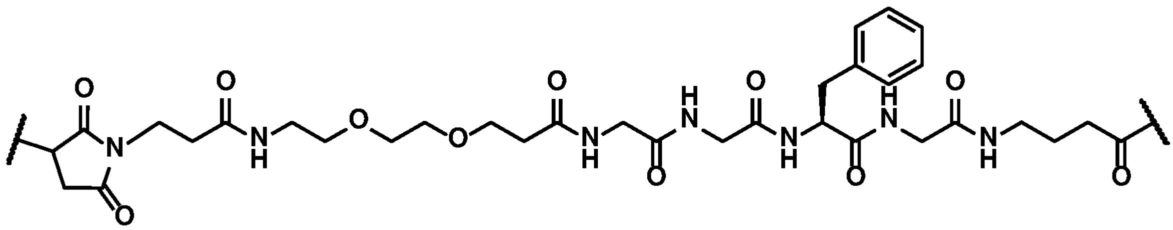

;

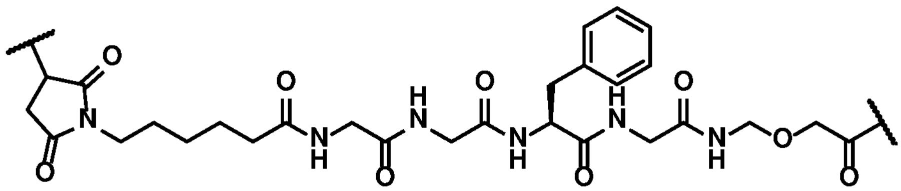

;

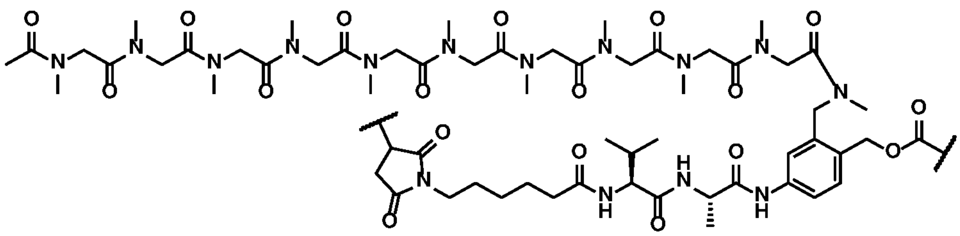

;

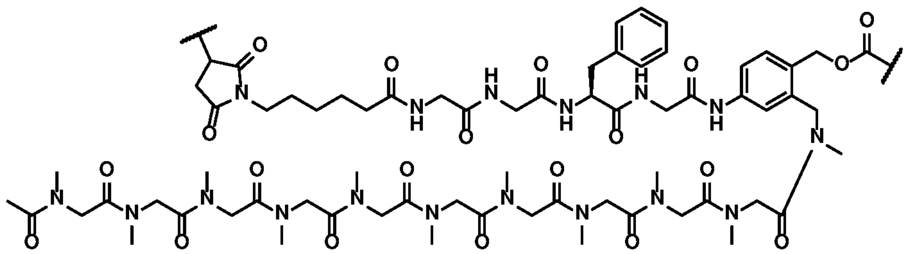

;

and

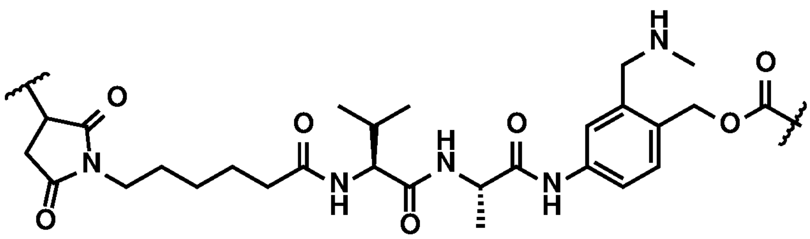

.

**31.** The antibody-drug conjugate according to any one of claims 10-30, wherein the payload is selected from at least one of the group comprising cytotoxic agents, markers, nucleic acids, radionuclides, hormones, immunomodulators, prodrug-converting enzymes, ribonucleases, agonistic antibodies, antagonistic antibodies, and fragments, fusion

proteins or derivatives thereof.

**32.** The antibody-drug conjugate according to claim 31, wherein the cytotoxic agent comprises a tubulin inhibitor and/or a topoisomerase inhibitor;
the tubulin inhibitor comprises auristatin or a derivative thereof, or maytansine or a derivative thereof; the topoisomerase inhibitor comprises camptothecin or a derivative thereof.

**33.** The antibody-drug conjugate according to any one of claims 10-32, wherein the payload is exatecan of formula III linked to the linker through the nitrogen atom of the amino group on a cyclohexane ring thereof,

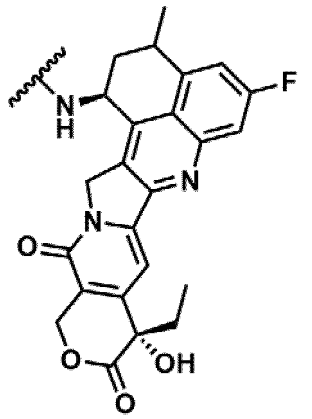

formula III.

**34.** The antibody-drug conjugate according to any one of claims 10-33, wherein the antibody-drug conjugate is selected from one of the following groups

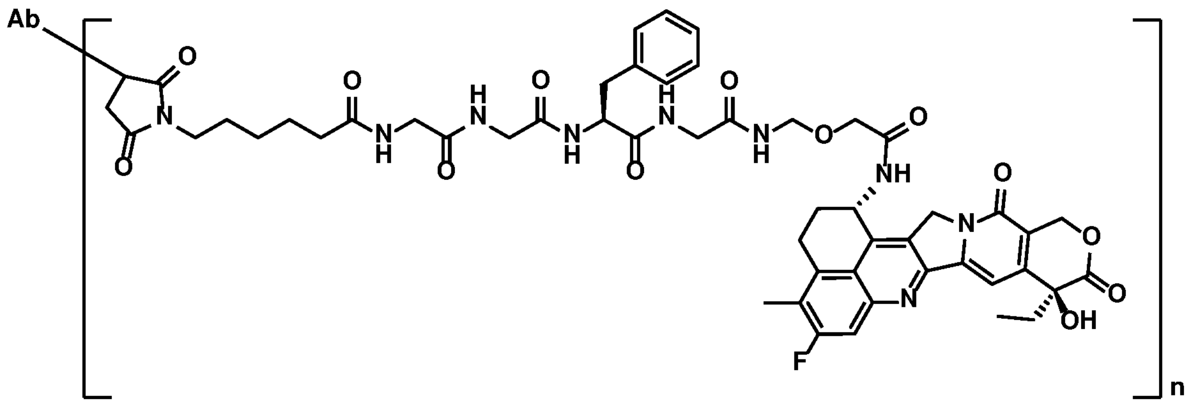

,

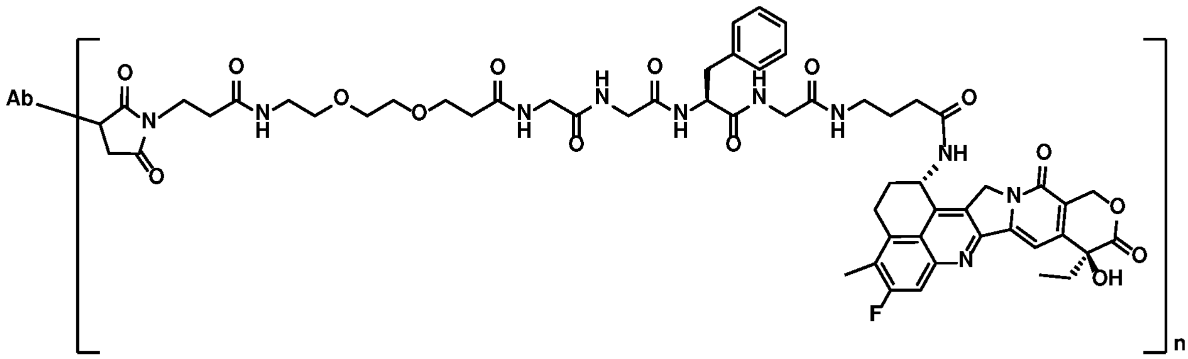

,

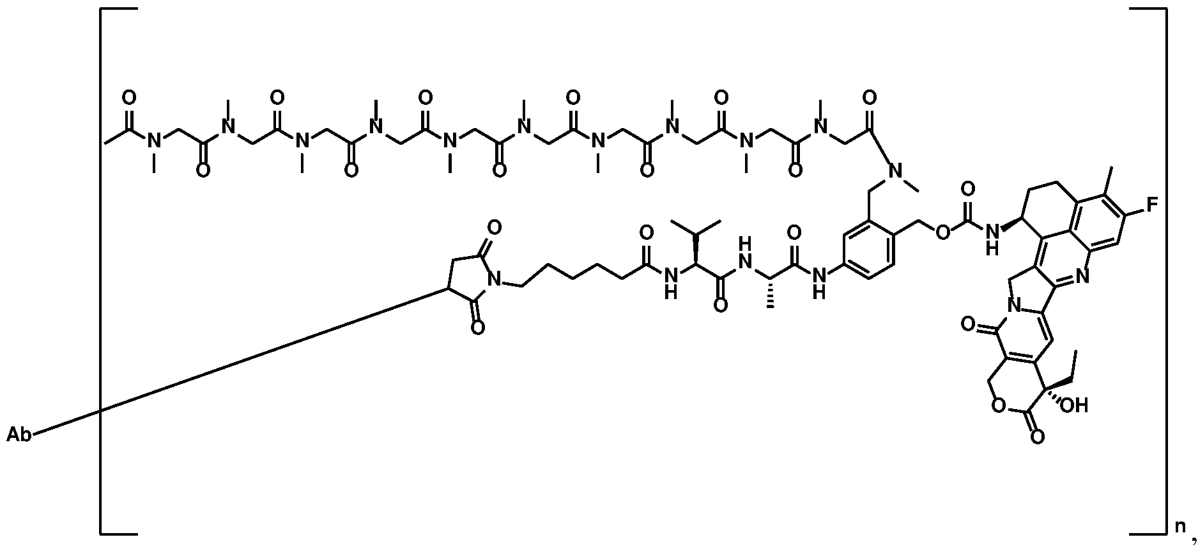

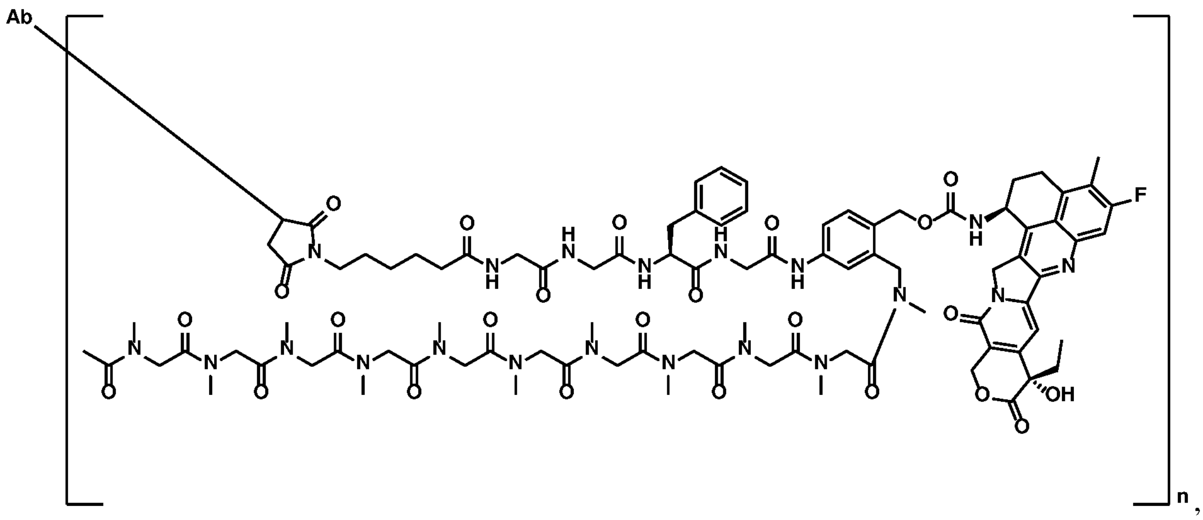

and

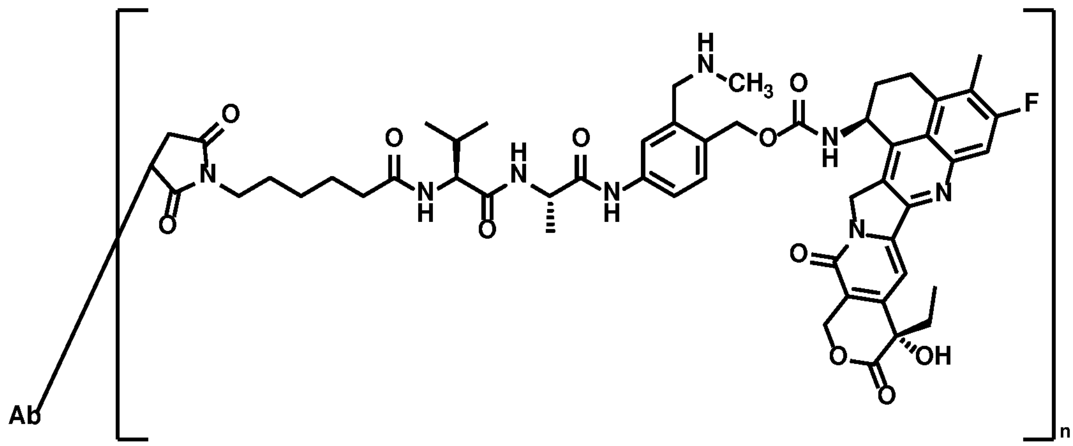

n is 1-10 or 4-10.

**35.** A preparation method for the antibody-drug conjugate according to any one of claims 10-34, comprising the following steps:

reducing at least part of interchain disulfide bonds of the antibody or the antigen-binding fragment thereof through reduction treatment, and reacting with a reactive group of the linker in the linker-payload to obtain the antibody-drug conjugate having the structure of formula I,

Ab-(L-D)n (I)

or an isomer, an isotopic variant, a pharmaceutically acceptable salt, a prodrug, or a solvate thereof, or a combination thereof,

wherein Ab is the anti-HER3 antibody or the antigen-binding fragment thereof according to any one of claims 1-6;

L is a linker covalently linked to Ab and D;

D is a payload;

n is a number between 1 and 10.

**36.** The preparation method according to claim 35, wherein the linker is a cleavable linker and a non-cleavable linker.

**37.** The preparation method according to claim 35 or 36, wherein the linker comprises a cleavable peptide.

**38.** The preparation method according to claim 37, wherein the cleavable peptide is cleavable by an enzyme.

**39.** The preparation method according to claim 38, wherein the enzyme comprises cathepsin B.

**40.** The preparation method according to any one of claims 35-39, wherein the cleavable peptide or L comprises an amino acid unit.

**41.** The preparation method according to claim 40, wherein the amino acid unit comprises a dipeptide, tripeptide, tetrapeptide, or pentapeptide.

**42.** The preparation method according to claim 41, wherein the amino acid unit is selected from: any or a combination of Val-Cit, Val-Ala, Glu-Val-Cit, Ala-Ala-Asn, Gly-Val-Cit, Gly-Gly-Gly, and Gly-Gly-Phe-Gly.

**43.** The preparation method according to any one of claims 35-42, wherein the linker comprises a self-immolative spacer.

**44.** The preparation method according to claim 43, wherein the self-immolative spacer comprises p-aminobenzyloxycarbonyl (PABC) or *p*-aminobenzyl (PAB).

**45.** The preparation method according to any one of claims 37-44, wherein the cleavable peptide is spliced directly to the self-immolative spacer.

**46.** The preparation method according to any one of claims 35-45, wherein the linker comprises the structure of $-L_1-L_2-L_3-$, wherein

$L_1$ represents -(succinimid-3-yl-N)-$(CH_2)m^1$-C(=O)-, $-CH_2$-C(=O)-NH-$(CH_2)m^2$-C(=O)-, or -C(=O)-$(CH_2)m^3$-C(=O)-, wherein $m^1$ represents an integer of 2-8, $m^2$ represents an integer of 1-8, and $m^3$ represents an integer of 1-8;

$L_2$ represents an amino acid unit;

$L_3$ represents a self-immolative spacer.

**47.** The preparation method according to any one of claims 35-49, wherein L is selected from:

-(succinimid-3-yl-N)-$CH_2CH_2$-C(=O)-GGFG-PABC-;

-(succinimid-3-yl-N)-$CH_2CH_2CH_2CH_2CH_2$-C(=O)-GGFG-PABC-;

-(succinimid-3-yl-N)-$CH_2CH_2CH_2CH_2CH_2$-C(=O)-GGFG-NH-PABC-;

-(succinimid-3-yl-N)-$CH_2CH_2$-C(=O)-NH-$CH_2CH_2O$-$CH_2CH_2O$-$CH_2CH_2$-C(=O)-GGFG-PABC-;

-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-GGFG-PABC-;

$-CH_2$-C(=O)-NH-$CH_2CH_2$-C(=O)-GGFG-PABC-;

-C(=O)-$CH_2CH_2CH_2CH_2CH_2CH_2$-C(=O)-GGFG-PABC-;

-(succinimid-3-yl-N)-$CH_2CH_2$-C(=O)-GGFG-NH-$CH_2CH_2$-C(=O)-;

-(succinimid-3-yl-N)-$CH_2CH_2$-C(=O)-GGFG-NH-$CH_2CH_2CH_2$-C(=O)-;
-(succinimid-3-yl-N)-$CH_2CH_2CH_2CH_2CH_2$-C(=O)-GGFG-NH-$CH_2CH_2$-C(=O)-;
-(succinimid-3-yl-N)-$CH_2CH_2CH_2CH_2CH_2$-C(=O)-GGFG-NH-$CH_2CH_2CH_2$-C(=O)-;
-(succinimid-3-yl-N)-$CH_2CH_2CH_2CH_2CH_2$-C(=O)-GGFG-NH-$CH_2CH_2CH_2CH_2CH_2$-C(=O)-;
-(succinimid-3-yl-N)-$CH_2CH_2CH_2CH_2CH_2$-C(=O)-GGFG-NH-$CH_2$-O-$CH_2$-C(=O)-;
-(succinimid-3-yl-N)-$CH_2CH_2CH_2CH_2CH_2$-C(=O)-GGFG-NH-$CH_2CH_2$-O-$CH_2$-C(=O)-;
-(succinimid-3-yl-N)-$CH_2CH_2$-C(=O)-NH-$CH_2CH_2O$-$CH_2CH_2O$-$CH_2CH_2$-C(=O)-GGFG-NH-$CH_2CH_2CH_2$-C(=O)-;
-(succinimid-3-yl-N)-$CH_2CH_2$-C(=O)-NH-$CH_2CH_2O$-$CH_2CH_2O$-$CH_2CH_2$-C(=O)-GGFG-NH-$CH_2CH_2$-C(=O)-;
-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-GGFG-NH-$CH_2CH_2CH_2$-C(=O)-;
-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-GGFG-NH-$CH_2CH_2$-C(=O)-;
-$CH_2$-C(=O)-NH-$CH_2CH_2$-C(=O)-GGFG-NH-$CH_2CH_2CH_2$-C(=O)-;
-C(=O)-$CH_2CH_2CH_2CH_2CH_2CH_2$-C(=O)-GGFG-NH-$CH_2CH_2CH_2$-C(=O)-;
-(succinimid-3-yl-N)-$CH_2CH_2$-C(=O)-VA-PABC-;
-(succinimid-3-yl-N)-CH2CH2CH2CH2CH2-C(=O)-VA-PABC-;
-(succinimid-3-yl-N)-$CH_2CH_2CH_2CH_2CH_2$-C(=O)-VA-NH-PABC-;
-(succinimid-3-yl-N)-$CH_2CH_2$-C(=O)-NH-$CH_2CH_2O$-$CH_2CH_2O$-$CH_2CH_2$-C(=O)-VA-PABC-;
-(succinimid-3-yl-N)-CH2CH2-C(=O)-NH-$CH_2CH_2O$-$CH_2CH_2O$-$CH_2CH_2O$-$CH_2CH_2O$-$CH_2CH_2$-C(=O)-VA-PABC-;
-$CH_2$-C(=O)-NH-$CH_2CH_2$-C(=O)-VA-PABC-;
-C(=O)-$CH_2CH_2CH_2CH_2CH_2CH_2$-C(=O)-VA-PABC-;
-(succinimid-3-yl-N)-$CH_2CH_2$-C(=O)-VA-NH-$CH_2CH_2$-C(=O)-;
-(succinimid-3-yl-N)-$CH_2CH_2$-C(=O)-VA-NH-$CH_2CH_2CH_2$-C(=O)-;
-(succinimid-3-yl-N)-$CH_2CH_2CH_2CH_2CH_2$-C(=O)-VA-NH-$CH_2CH_2$-C(=O)-;
-(succinimid-3-yl-N)-$CH_2CH_2CH_2CH_2CH_2$-C(=O)-VA-NH-$CH_2CH_2CH_2$-C(=O)-;
-(succinimid-3-yl-N)-$CH_2CH_2CH_2CH_2CH_2$-C(=O)-VA-NH-$CH_2CH_2CH_2CH_2CH_2$-C(=O)-;
-(succinimid-3-yl-N)-$CH_2CH_2CH_2CH_2CH_2$-C(=O)-VA-NH-$CH_2$-O-$CH_2$-C(=O)-;
-(succinimid-3-yl-N)-$CH_2CH_2CH_2CH_2CH_2$-C(=O)-VA-NH-$CH_2CH_2$-O-$CH_2$-C(=O)-;
-(succinimid-3-yl-N)-$CH_2CH_2$-C(=O)-NH-$CH_2CH_2O$-$CH_2CH_2O$-$CH_2CH_2$-C(=O)-VA-NH-$CH_2CH_2CH_2$-C(=O)-;
-(succinimid-3-yl-N)-$CH_2CH_2$-C(=O)-NH-$CH_2CH_2O$-$CH_2CH_2O$-$CH_2CH_2$-C(=O)-VA-NH-$CH_2CH_2$-C(=O)-;
-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-VA-NH-$CH_2CH_2CH_2$-C(=O)-;
-(succinimid-3-yl-N)-$CH_2CH_2$-C(=O)-NH-$CH_2CH_2O$-$CH_2CH_2O$-$CH_2CH_2O$-$CH_2CH_2O$-$CH_2CH_2$-C(=O)-VA-NH-$CH_2CH_2$-C(=O)-;
-$CH_2$-C(=O)-NH-$CH_2CH_2$-C(=O)-VA-NH-$CH_2CH_2CH_2$-C(=O)-; and
-C(=O)-$CH_2CH_2CH_2CH_2CH_2CH_2$-C(=O)-VA-NH-$CH_2CH_2CH_2$-C(=O)-.

**48.** The preparation method according to any one of claims 44-47, wherein the *p*-aminobenzyloxycarbonyl (PABC) or p-aminobenzyl (PAB) is linked to a poly-sarcosine (poly-N-methylglycine) residue or methylamino.

**49.** The preparation method according to any one of claims 35-48, wherein the preparation method comprises the following steps: reducing at least part of interchain disulfide bonds of the anti-HER3 antibody or the antigen-binding fragment thereof through reduction treatment, and reacting with the carbon atom at position 3 of the maleimide-N-yl of the linker part of formula IV in the linker-payload;

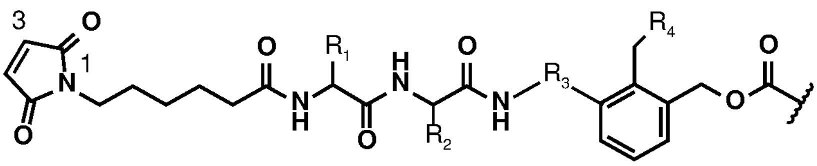

formula IV

in the linker-payload, the carbonyl in the ester group of the linker of formula IV is linked to the amino group in the payload;
in formula IV, $R_1$ and $R_2$ are independently selected from hydrogen, methyl, and isopropyl groups; $R_3$ represents

$-(CR_5HCONH)n^1-(CH_2CONH)n^2-$ or a single bond, $R_5$ is selected from hydrogen and benzyl, $n^1$ represents an integer of 0-2, and $n^2$ represents an integer of 0-2;
$R_4$ represents a methylamino group or $-(NCH_3COCH_2)n^3-NCH_3COCH_3$, and $n^3$ represents an integer of 1-20.

**50.** The preparation method according to claim 49, wherein the method further comprises: reacting the anti-HER3 antibody or the antigen-binding fragment thereof with a reducing agent in a buffer solution containing a chelating agent, adding a linker-payload solution, and adjusting the pH.

**51.** The preparation method according to claim 49 or 50, wherein the method further comprises: linking the nitrogen atom of the amino group on a cyclohexane ring of the payload, which is exatecan of formula III, to the carbonyl of the ester of formula IV,

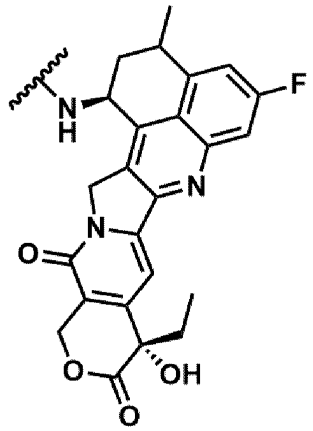

formula III.

**52.** A pharmaceutical composition, comprising the anti-HER3 antibody or the antigen-binding fragment thereof according to any one of claims 1-6 or the antibody-drug conjugate according to any one of claims 10-34, or an isomer, an isotopic variant, a pharmaceutically acceptable salt, a prodrug, or a solvate thereof, or a combination thereof, and a pharmaceutically acceptable excipient.

**53.** A kit, comprising the anti-HER3 antibody or the antigen-binding fragment thereof according to any one of 1-6 or the antibody-drug conjugate according to any one of claims 10-34, or an isomer, an isotopic variant, a pharmaceutically acceptable salt, a prodrug, or a solvate thereof, or a combination thereof.

**54.** Use of the anti-HER3 antibody or the antigen-binding fragment thereof according to any one of claims 1-6, the antibody-drug conjugate according to any one of claims 10-34, the antibody-drug conjugate prepared by the method according to any one of claims 35-51, or the pharmaceutical composition according to claim 52, or the kit according to claim 53 in preparing a therapeutic agent for diagnosing, preventing and treating tumor diseases.

**55.** The use according to claim 54, wherein the use comprises use in preparing a medicament targeting HER3.

**56.** The use according to claim 54 or 55, wherein the tumor comprises a HER3-expressing solid tumor.

**57.** The use according to any one of claims 54-56, wherein the tumor disease includes lung cancer, kidney cancer, urothelial cancer, colorectal cancer, prostate cancer, glioblastoma multiforme, ovarian cancer, pancreatic cancer, breast cancer, metastatic breast cancer, luminal breast cancer, melanoma, liver cancer, bladder cancer, gastric cancer (stomach cancer), gastrointestinal stromal tumor, cervical cancer, head and neck cancer, esophageal cancer, epidermoid cancer, peritoneal cancer, adult glioblastoma multiforme, hepatic cancer, hepatocellular cancer, colon cancer, rectal cancer, colon and rectal cancer, endometrial cancer, uterine cancer, salivary gland cancer, renal cancer, vulval cancer, thyroid cancer, hepatic carcinoma, anal cancer, penile cancer, and lung adenocarcinoma.

**58.** A method for diagnosing, preventing and treating tumor diseases, comprising administering to a subject a therapeutic dose of a therapeutic agent comprising the anti-HER3 antibody or the antigen-binding fragment thereof according to any one of claims 1-6, the antibody-drug conjugate according to any one of claims 10-34, the antibody-drug conjugate prepared by the method according to any one of claims 35-51, the pharmaceutical composition according to claim 52, or the kit according to claim 53.

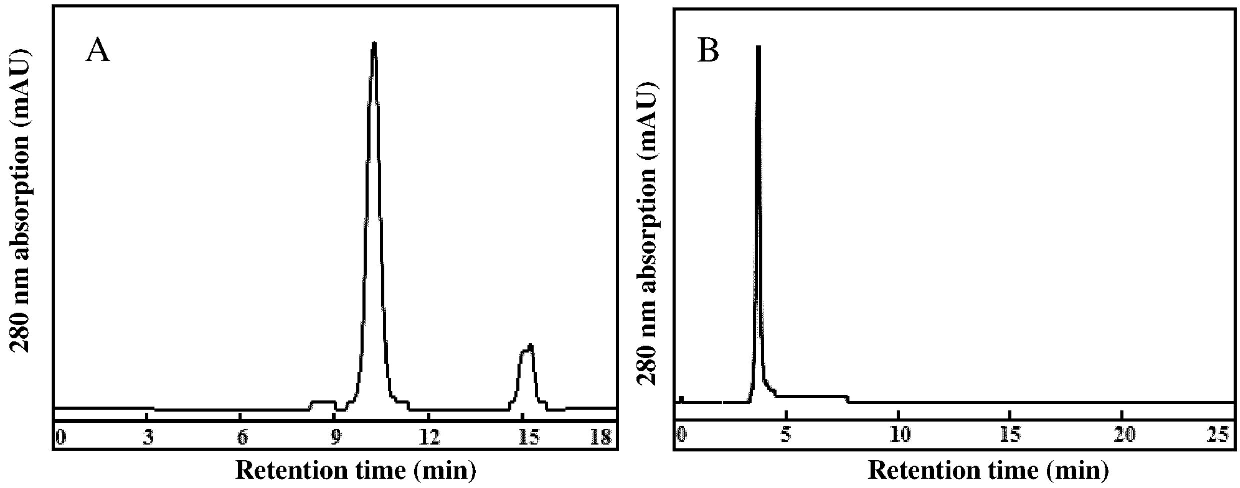


FIG. 1

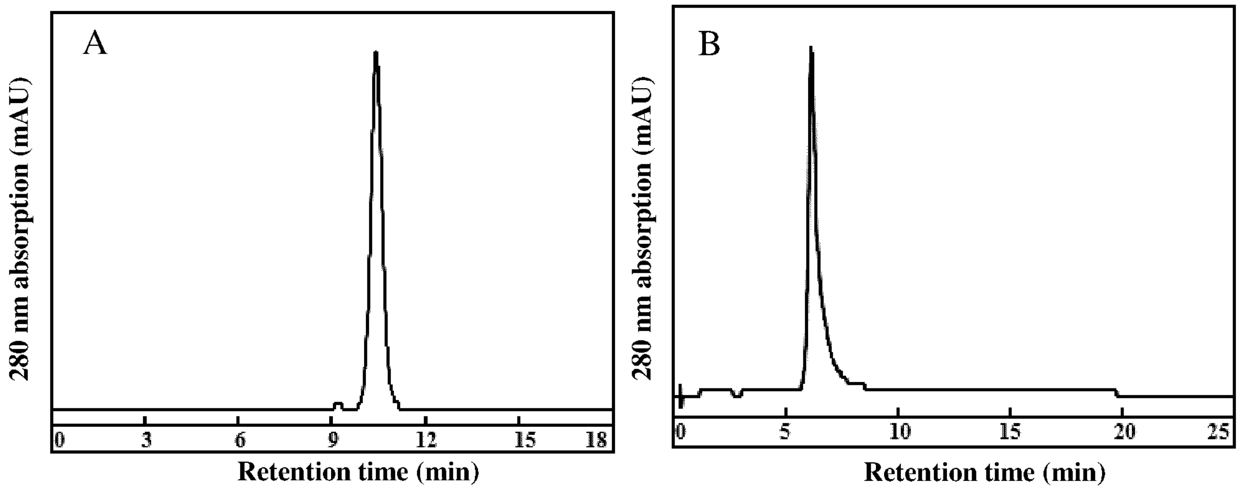


FIG. 2

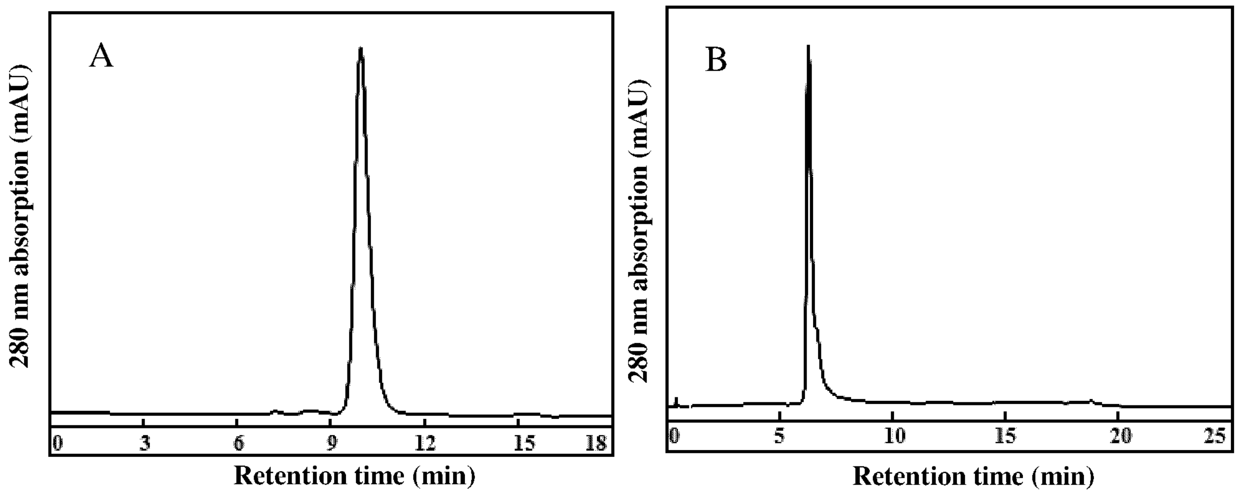


FIG. 3

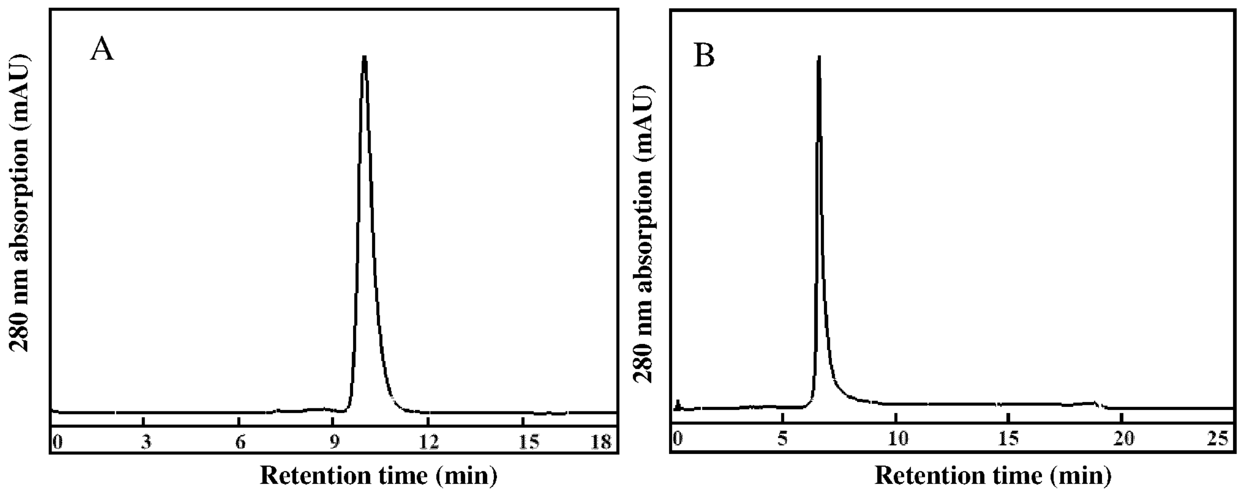
A
280 nm absorption (mAU)
0
3
6
9
12
15
18
Retention time (min)
B
280 nm absorption (mAU)
0
5
10
15
20
25
Retention time (min)

FIG. 4

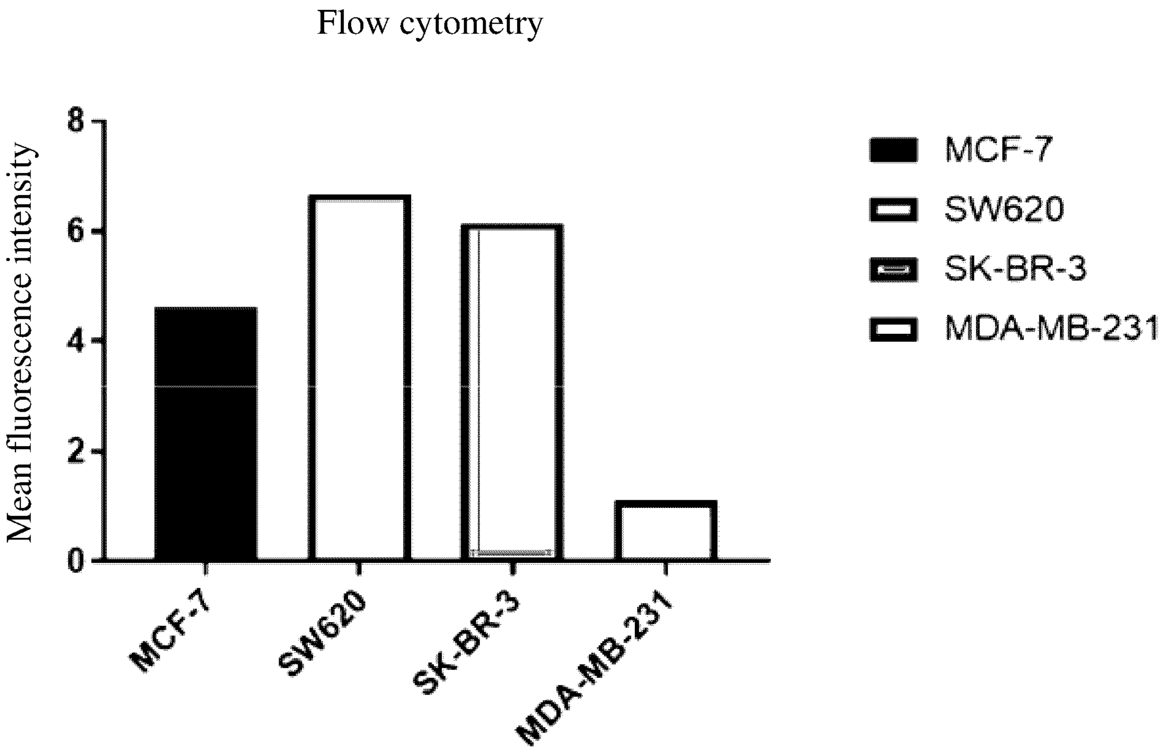
Flow cytometry
Mean fluorescence intensity
8
6
4
2
0
MCF-7
SW620
SK-BR-3
MDA-MB-231
MCF-7
SW620
SK-BR-3
MDA-MB-231

FIG. 5

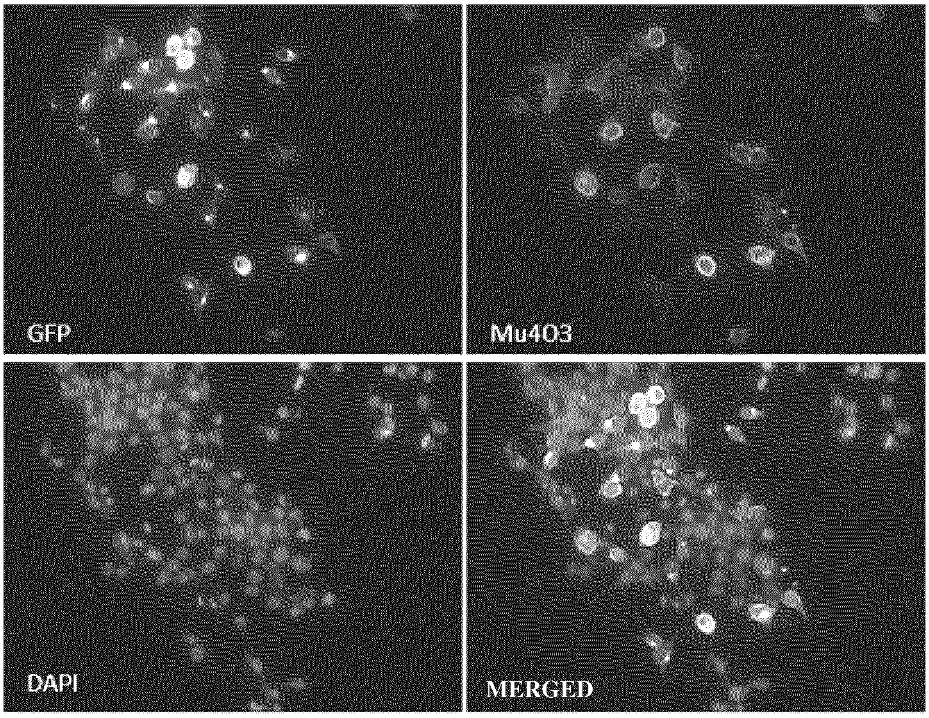
GFP
Mu4O3
DAPI
MERGED

FIG. 6

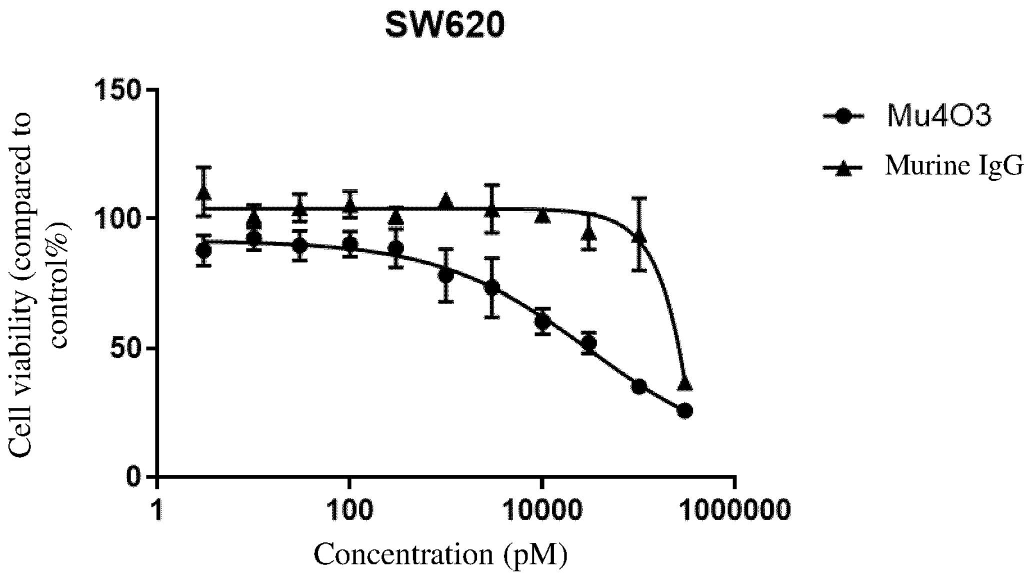
SW620
Cell viability (compared to control%)
150
100
50
0
1
100
10000
1000000
Concentration (pM)
Mu4O3
Murine IgG

FIG. 7

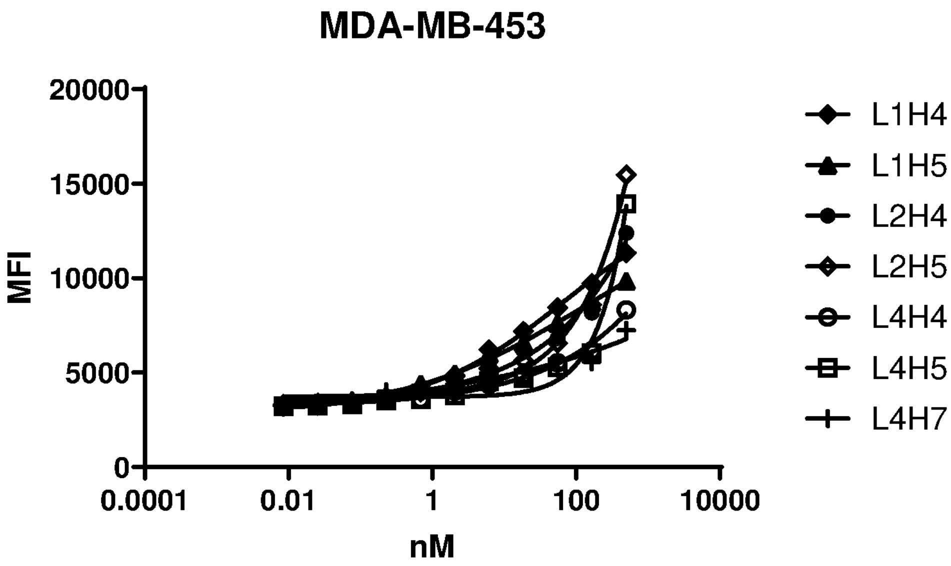


FIG. 8

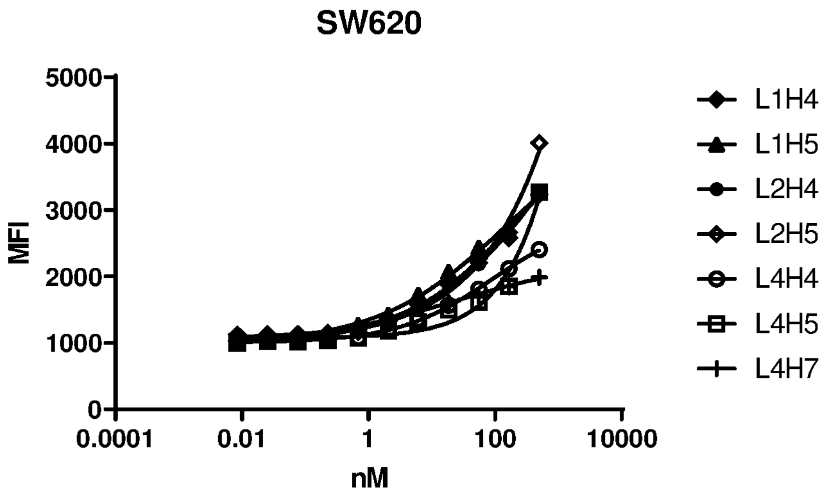


FIG. 9

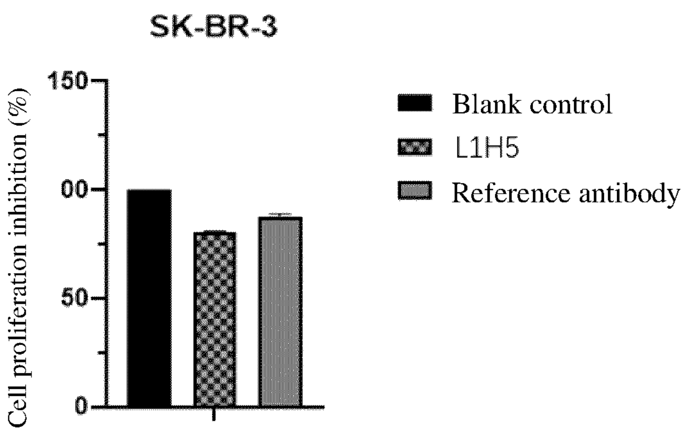


FIG. 10

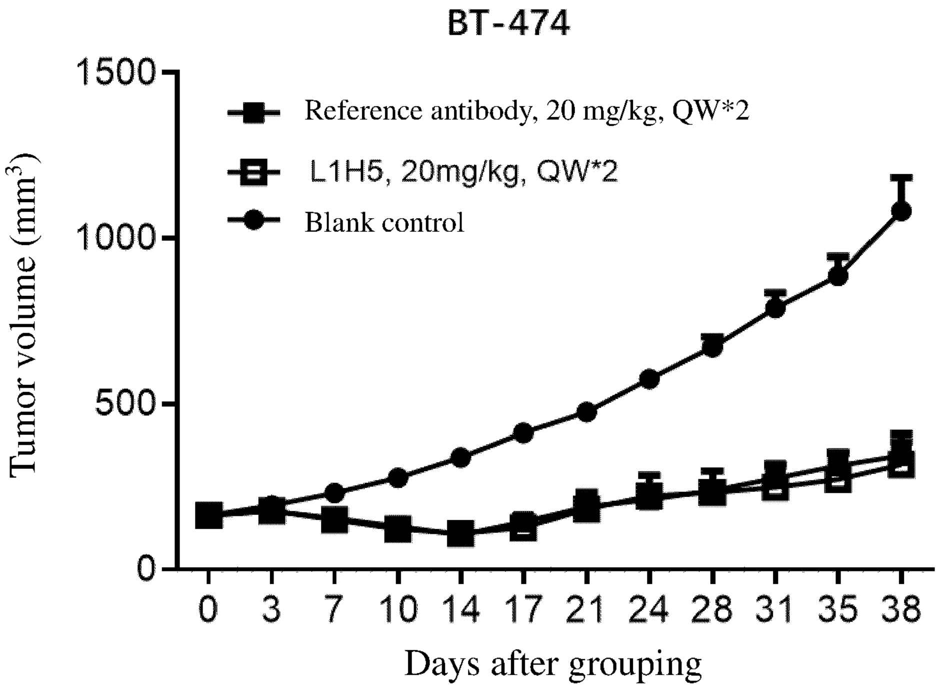
BT-474
Tumor volume (mm³)
1500
1000
500
0
Reference antibody, 20 mg/kg, QW*2
L1H5, 20mg/kg, QW*2
Blank control
0 3 7 10 14 17 21 24 28 31 35 38
Days after grouping

FIG. 11

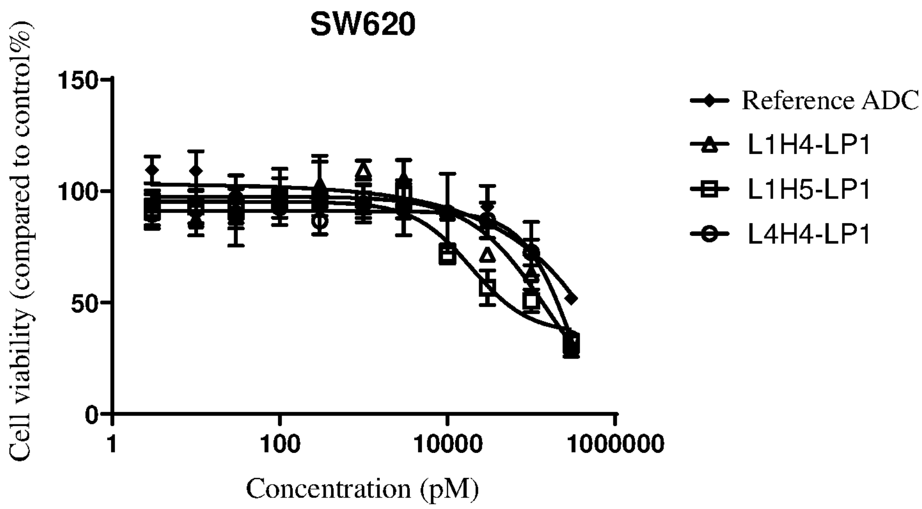
SW620
Cell viability (compared to control%)
150
100
50
0
1
100
10000
1000000
Concentration (pM)
Reference ADC
L1H4-LP1
L1H5-LP1
L4H4-LP1

FIG. 12

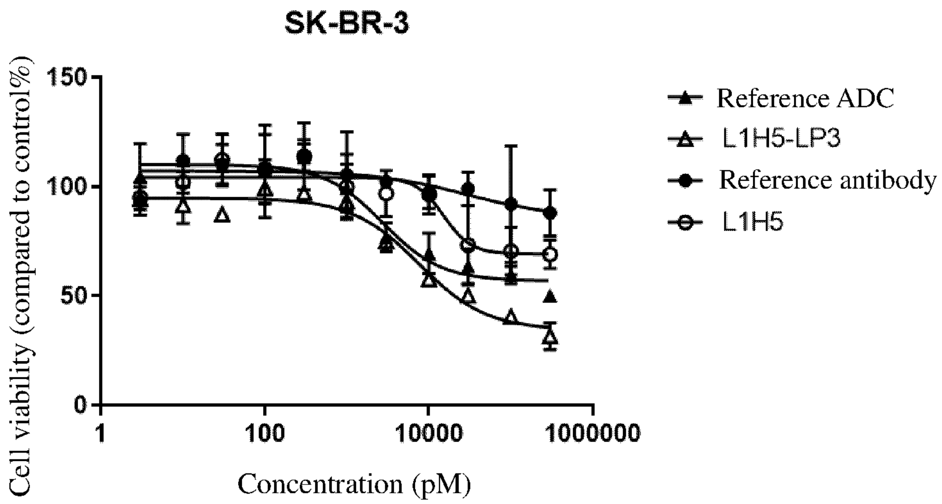


FIG. 13

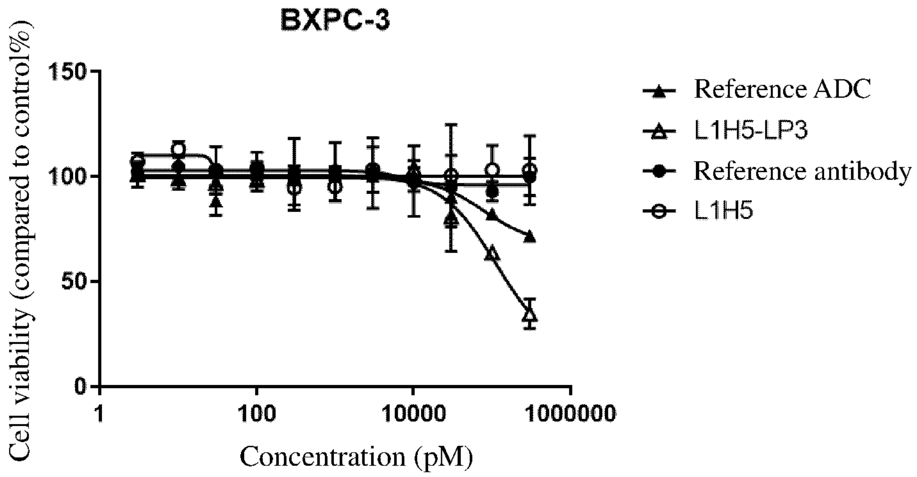


FIG. 14

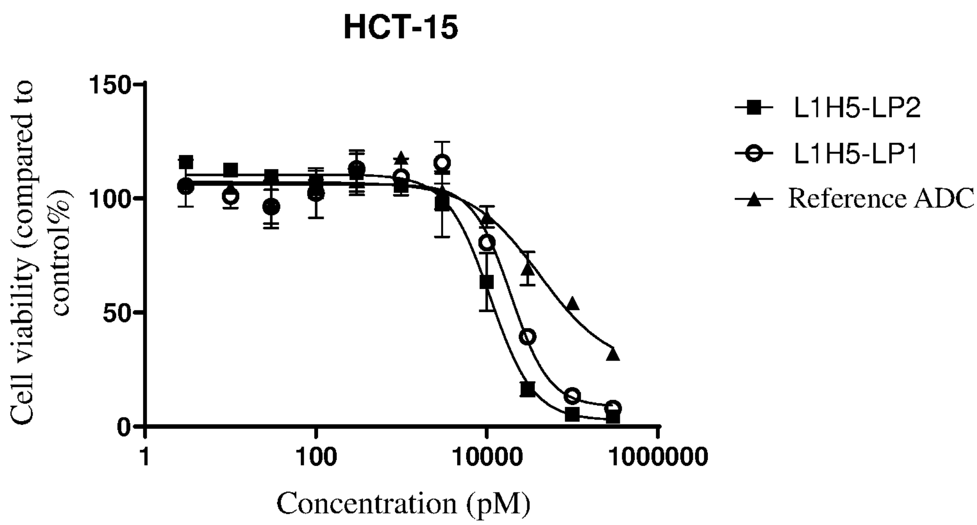


FIG. 15

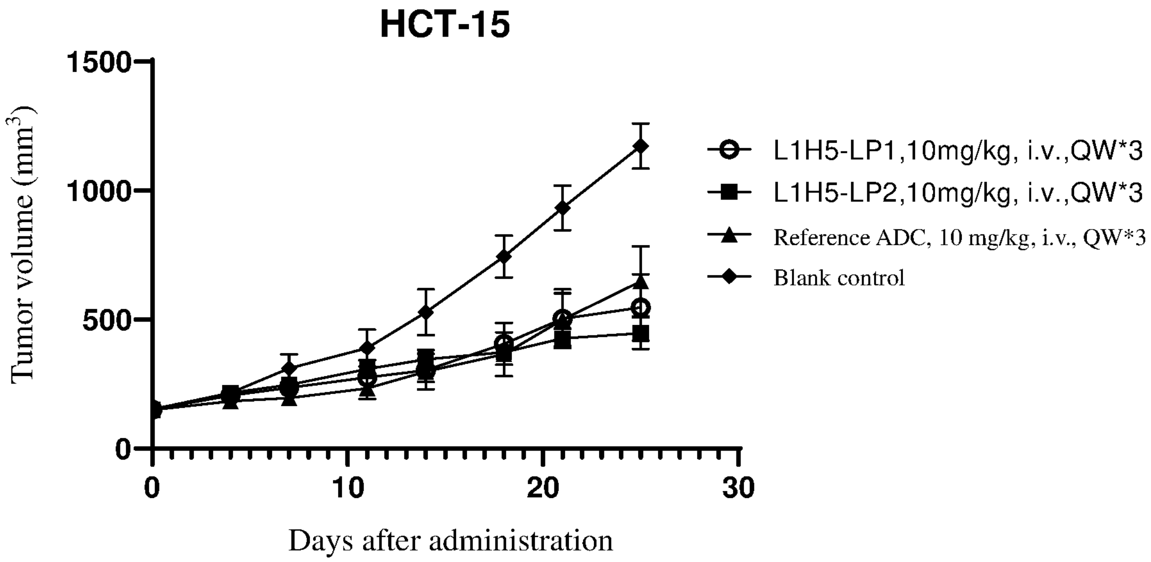
HCT-15
Tumor volume (mm3)
1500
1000
500
0
0
10
20
30
Days after administration
L1H5-LP1,10mg/kg, i.v.,QW*3
L1H5-LP2,10mg/kg, i.v.,QW*3
Reference ADC, 10 mg/kg, i.v., QW*3
Blank control

FIG. 16

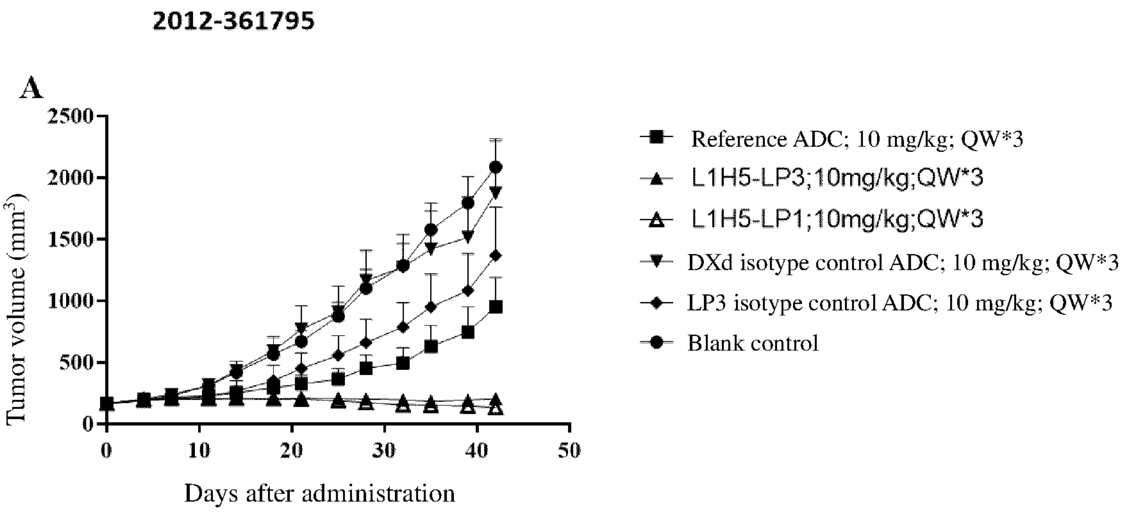
2012-361795
A
Tumor volume (mm3)
2500
2000
1500
1000
500
0
0
10
20
30
40
50
Days after administration
Reference ADC; 10 mg/kg; QW*3
L1H5-LP3;10mg/kg;QW*3
L1H5-LP1;10mg/kg;QW*3
DXd isotype control ADC; 10 mg/kg; QW*3
LP3 isotype control ADC; 10 mg/kg; QW*3
Blank control

FIG. 17

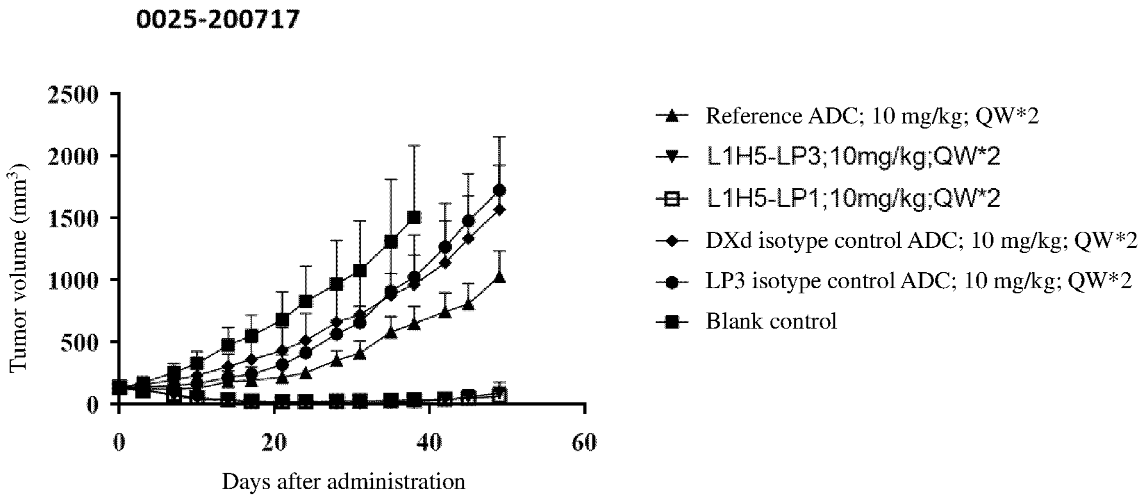


FIG. 18

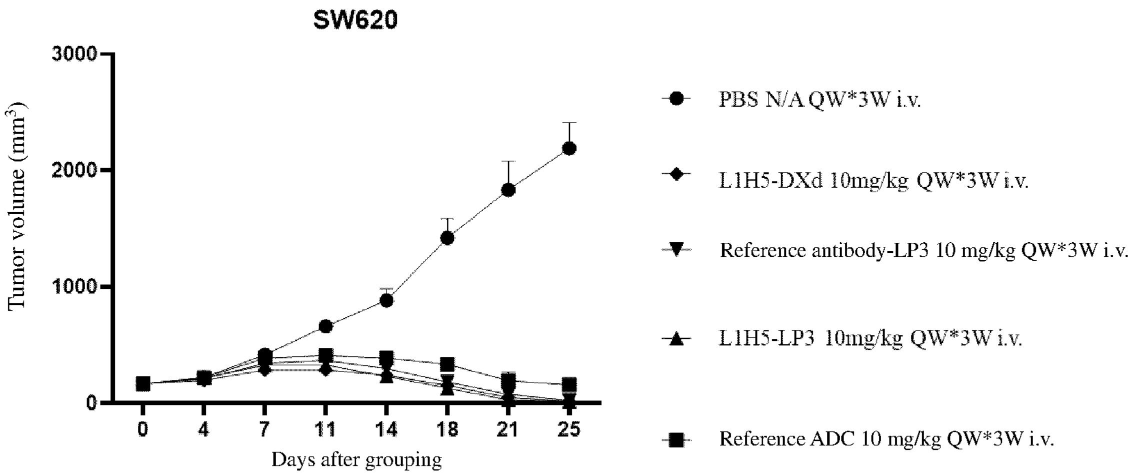


FIG. 19

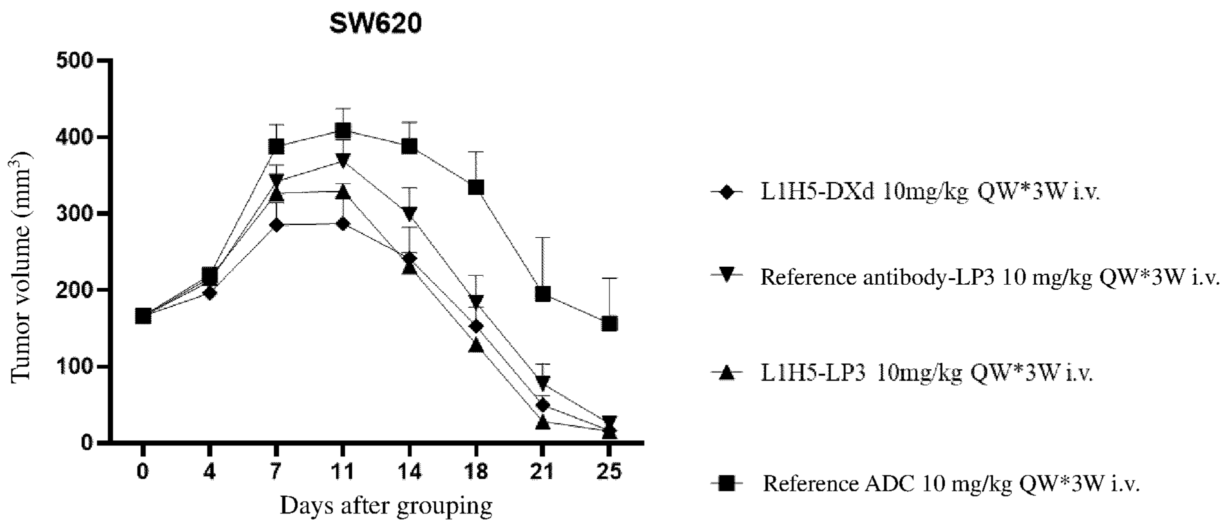


FIG. 20

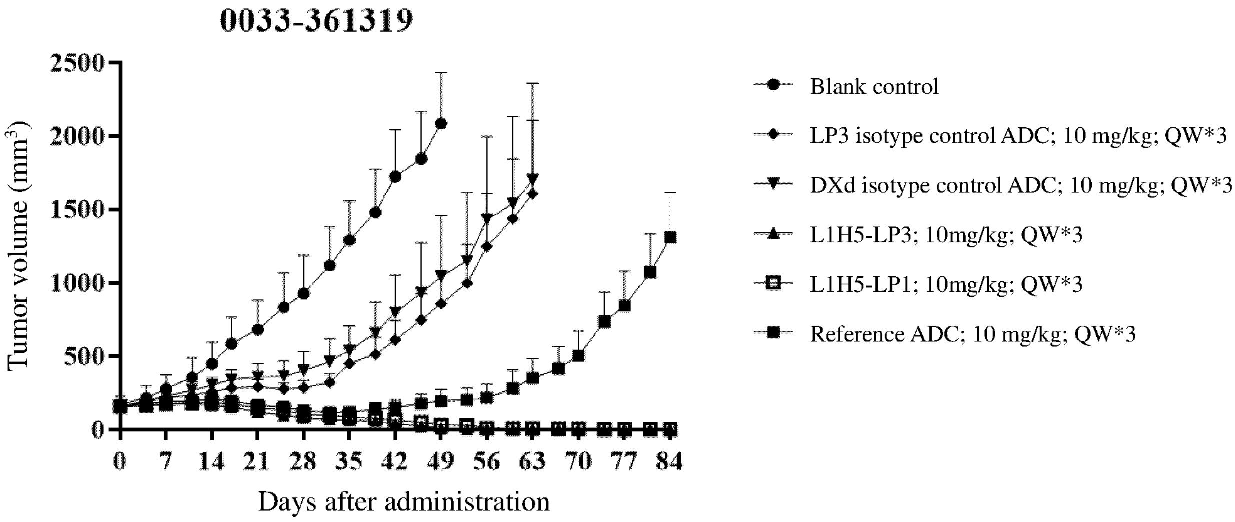
0033-361319
Tumor volume (mm3)
2500
2000
1500
1000
500
0
0 7 14 21 28 35 42 49 56 63 70 77 84
Days after administration
Blank control
LP3 isotype control ADC; 10 mg/kg; QW*3
DXd isotype control ADC; 10 mg/kg; QW*3
L1H5-LP3; 10mg/kg; QW*3
L1H5-LP1; 10mg/kg; QW*3
Reference ADC; 10 mg/kg; QW*3

FIG. 21

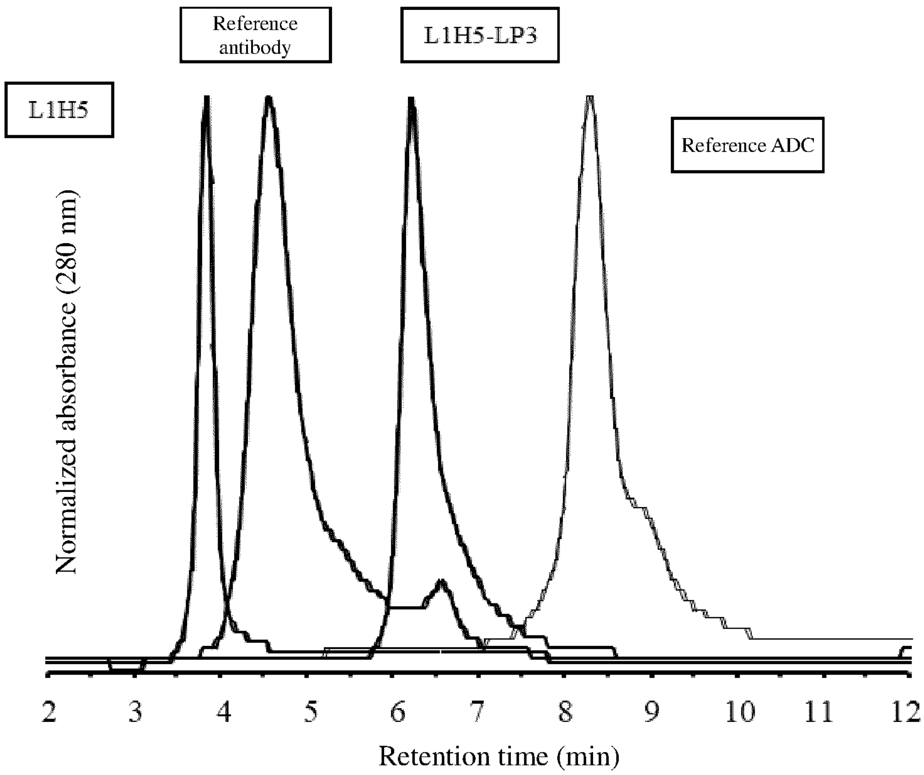
Reference antibody
L1H5-LP3
L1H5
Reference ADC
Normalized absorbance (280 nm)
2 3 4 5 6 7 8 9 10 11 12
Retention time (min)

FIG. 22

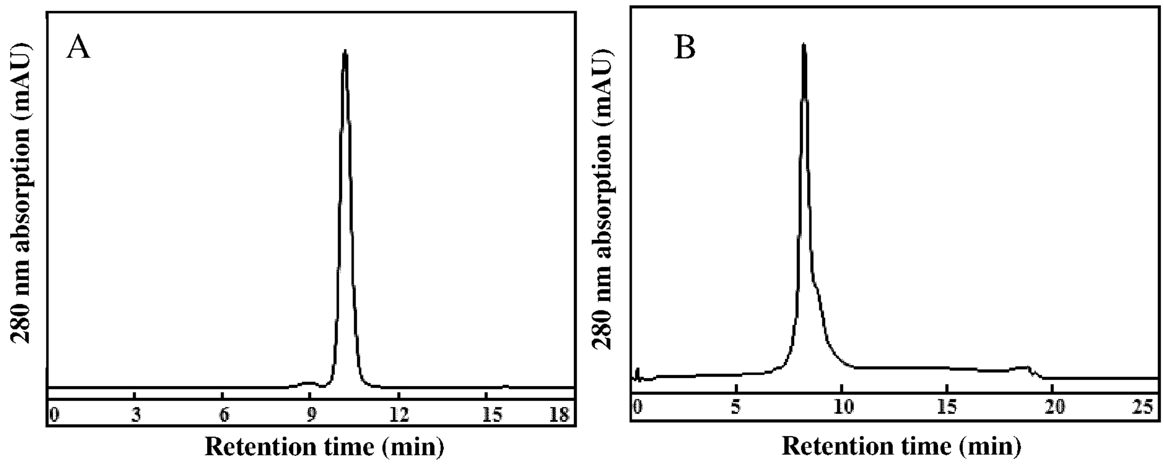


FIG. 23

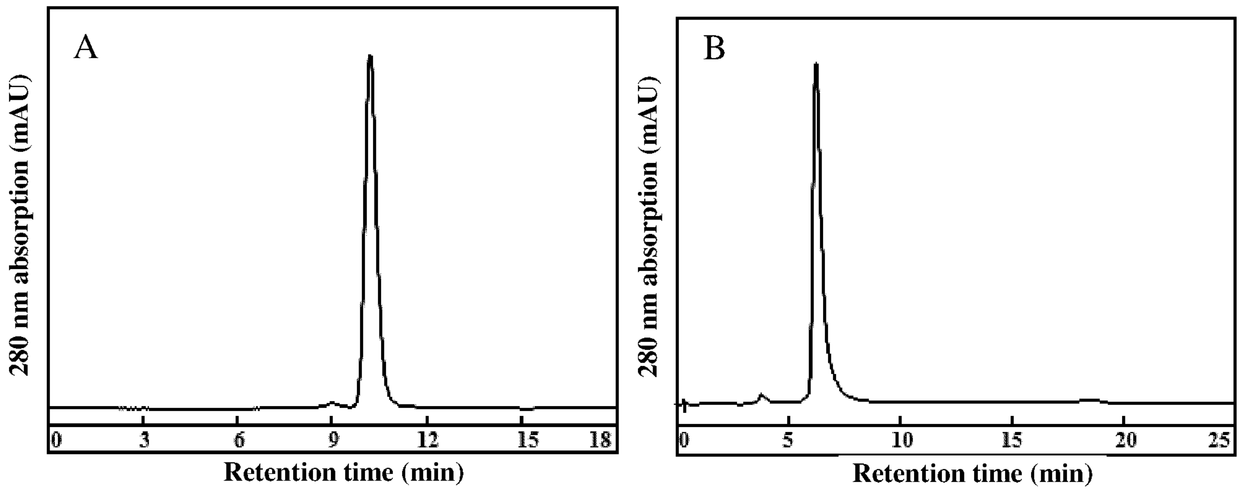


FIG. 24

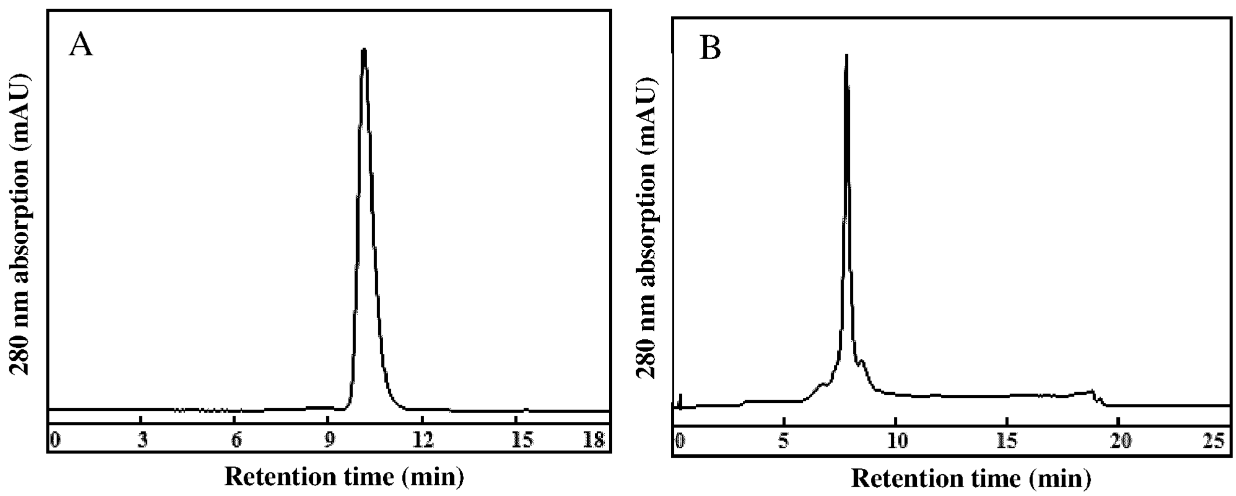


FIG. 25

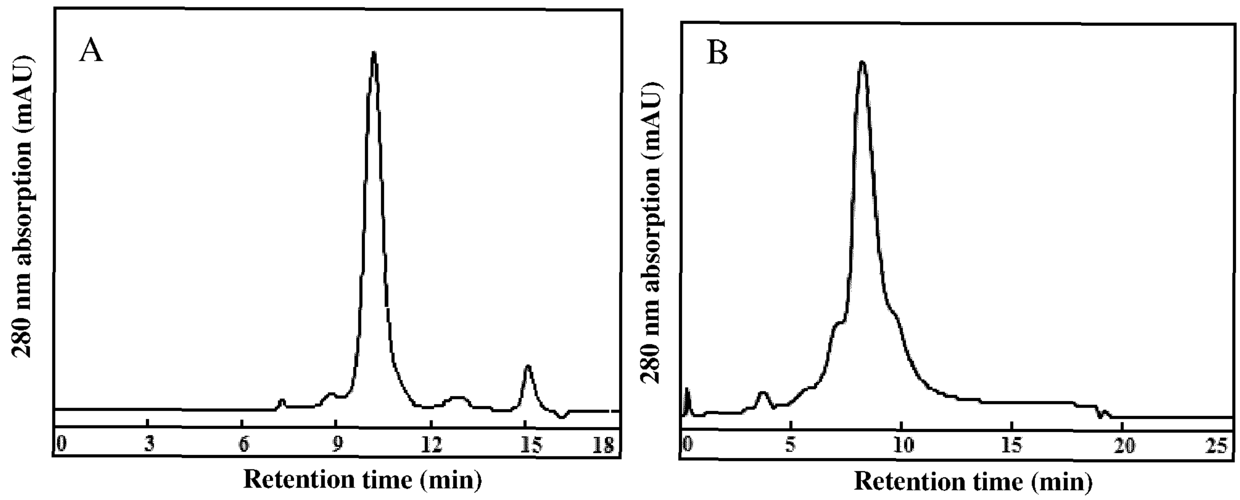
A
280 nm absorption (mAU)
0 3 6 9 12 15 18
Retention time (min)
B
280 nm absorption (mAU)
0 5 10 15 20 25
Retention time (min)

FIG. 26

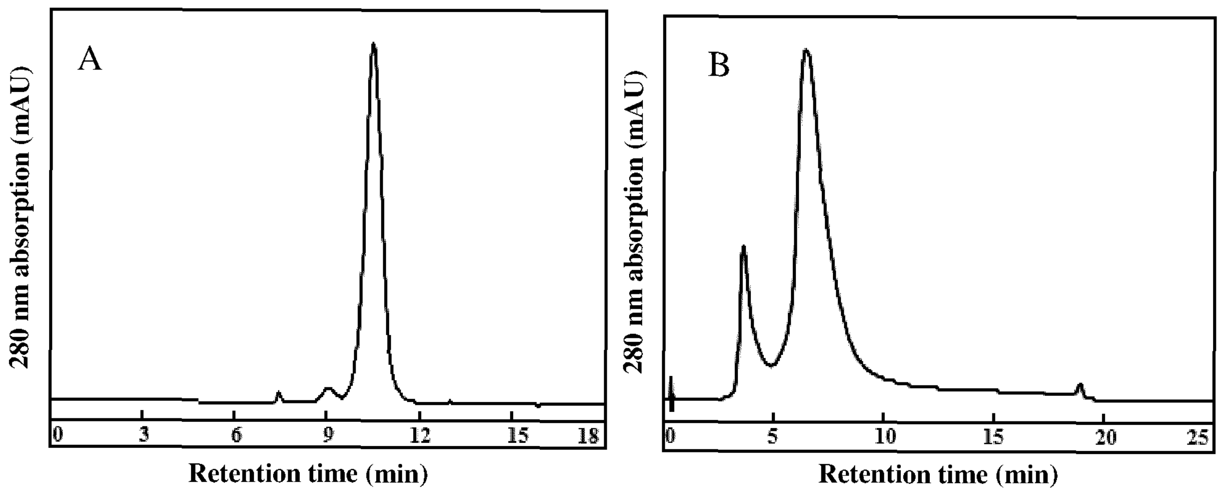
A
280 nm absorption (mAU)
0 3 6 9 12 15 18
Retention time (min)
B
280 nm absorption (mAU)
0 5 10 15 20 25
Retention time (min)

FIG. 27

**INTERNATIONAL SEARCH REPORT**

International application No.
**PCT/CN2023/125269**

**A. CLASSIFICATION OF SUBJECT MATTER**

C07K16/32(2006.01)i; C07K16/30(2006.01)i; C07K16/28(2006.01)i; A61K47/68(2017.01)i; A61K39/395(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07K A61K A61P

Documentation searched other than minimum documentation to the extent that such documents are included in the fields searched

Electronic data base consulted during the international search (name of data base and, where practicable, search terms used)

CNABS, CNTXT, VEN, ENTXT, ENTXTC, WOTXT, USTXT, EPTXT, CNKI, 万方, WANFANG, Pubmed, ISI web of knowledge, BING, Genbank, STN, HER3, ErbB-3, 偶联物, 缀合物, conjugate, ADC, 连接子, linker, 接头, 肽, GGFG, PABC, PAB, 依喜替康, 依沙替康, exatecan, 肿瘤, 癌, cancer, tumor, SEQ ID NOs: 1-16

**C. DOCUMENTS CONSIDERED TO BE RELEVANT**

| Category* | Citation of document, with indication, where appropriate, of the relevant passages | Relevant to claim No. |
|---|---|---|
| A | CN 106163559 A (DAICHI SANKYO COMPANY, LTD. et al.) 23 November 2016 (2016-11-23)<br>claims 1-20, and description, paragraphs 46-300 and 1040-1045 | 1-58 |
| A | WO 2022078425 A1 (JIANGSU HENGRUI MEDICINE CO., LTD. et al.) 21 April 2022 (2022-04-21)<br>claims 1-19, and description, page, 2 line 1 to page 12, line 34 | 1-58 |
| A | WO 2012019024 A2 (IMMUNOGEN, INC.) 09 February 2012 (2012-02-09)<br>claims 1-61, and description, paragraphs 8-32 | 1-58 |
| A | WO 2019241893 A2 (CRD PHARMACEUTICALS INC.) 26 December 2019 (2019-12-26)<br>claims 1-40, and description, page 4, last paragraph to page 11, paragraph 1, and page 35, paragraph 2 to page 38, paragraph 3 | 1-58 |
| A | CN 104093743 A (MEDIMMUNE LLC) 08 October 2014 (2014-10-08)<br>claims 1-119 | 1-58 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **09 December 2023** | **18 December 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2023/125269

**C. DOCUMENTS CONSIDERED TO BE RELEVANT**

| Category* | Citation of document, with indication, where appropriate, of the relevant passages | Relevant to claim No. |
|---|---|---|
| A | KOYAMA, K. et al. "Patritumab Deruxtecan (HER3-DXd), a Novel HER3 Directed Antibody Drug Conjugate, Exhibits In Vitro Activity Against Breast Cancer Cells Expressing HER3 Mutations with and without HER2 Overexpression" *PLoS One,* Vol. 17, No. (5), 03 May 2022 (2022-05-03), e0267027, pages 1-10 | 1-58 |
| A | KOGANEMARU, S. et al. "U3-1402, a Novel HER3-Targeting Antibody–Drug Conjugate, for the Treatment of Colorectal Cancer" *Molecular Cancer Therapeutics,* Vol. 18, No. (11), 08 August 2019 (2019-08-08), pp. 2043-2050 | 1-58 |

Form PCT/ISA/210 (second sheet) (July 2022)

**INTERNATIONAL SEARCH REPORT**

International application No.
**PCT/CN2023/125269**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2023/125269**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **58**
because they relate to subject matter not required to be searched by this Authority, namely:

Claim 58 relates to a method for diagnosing, preventing and treating tumor diseases, that is, claim 58 relates to subject matter for which no search is required by the International Searching Authority as defined in PCT Rule 39.1: (4) methods for treatment of the human or animal body by surgery or therapy, as well as diagnostic methods. The international search is made on the basis of the use of an effective dose of a therapeutic agent in the preparation of a drug for diagnosing, preventing or treating tumor diseases.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2023/125269**

| Patent document cited in search report | Publication date (day/month/year) | Patent family member(s) | Publication date (day/month/year) |
|---|---|---|---|
| CN 106163559 A | 23 November 2016 | MX 2016010533 A | 12 December 2016 |
| | | KR 20200077620 A | 30 June 2020 |
| | | KR 102186027 B1 | 03 December 2020 |
| | | KR 20220068270 A | 25 May 2022 |
| | | KR 102445502 B1 | 21 September 2022 |
| | | CA 2939802 A1 | 15 October 2015 |
| | | CA 2939802 C | 01 November 2022 |
| | | KR 20190004837 A | 14 January 2019 |
| | | KR 102127623 B1 | 29 June 2020 |
| | | JP 2022180416 A | 06 December 2022 |
| | | JP 7383772 B2 | 20 November 2023 |
| | | JP 6148422 B1 | 14 June 2017 |
| | | JP 2017197515 A | 02 November 2017 |
| | | RS 61711 B1 | 31 May 2021 |
| | | TW 201542230 A | 16 November 2015 |
| | | TWI 704928 B | 21 September 2020 |
| | | LT 3129063 T | 25 June 2021 |
| | | US 2019151328 A1 | 23 May 2019 |
| | | US 11298359 B2 | 12 April 2022 |
| | | RU 2019143766 A | 20 February 2020 |
| | | HRP 20210593 T1 | 14 May 2021 |
| | | KR 20200136052 A | 04 December 2020 |
| | | KR 102239413 B1 | 12 April 2021 |
| | | JP 2017503784 A | 02 February 2017 |
| | | JP 6105171 B2 | 29 March 2017 |
| | | KR 20210115056 A | 24 September 2021 |
| | | KR 102351755 B1 | 14 January 2022 |
| | | JP 2019135248 A | 15 August 2019 |
| | | JP 6707696 B2 | 10 June 2020 |
| | | IL 300540 A | 01 April 2023 |
| | | KR 20230021173 A | 13 February 2023 |
| | | IL 248239 A0 | 30 November 2016 |
| | | IL 248239 B | 01 July 2022 |
| | | TW 202108176 A | 01 March 2021 |
| | | TWI 745021 B | 01 November 2021 |
| | | SI 3129063 T1 | 31 August 2021 |
| | | ES 2859648 T3 | 04 October 2021 |
| | | US 2017021031 A1 | 26 January 2017 |
| | | US 10383878 B2 | 20 August 2019 |
| | | JP 2021169515 A | 28 October 2021 |
| | | JP 7138750 B2 | 16 September 2022 |
| | | AU 2020200227 A1 | 06 February 2020 |
| | | AU 2020200227 B2 | 07 October 2021 |
| | | KR 20210041126 A | 14 April 2021 |
| | | BR 112016017893 A2 | 17 October 2017 |
| | | BR 112016017893 B1 | 23 August 2022 |
| | | SG 10201907807 XA | 27 September 2019 |
| | | CY 1124071 T1 | 27 May 2022 |
| | | RU 2016143351 A | 15 May 2018 |
| | | RU 2016143351 A3 | 05 March 2019 |

Form PCT/ISA/210 (patent family annex) (July 2022)

**INTERNATIONAL SEARCH REPORT**
**Information on patent family members**

International application No.
**PCT/CN2023/125269**

| Patent document cited in search report | Publication date (day/month/year) | Patent family member(s) | Publication date (day/month/year) |
|---|---|---|---|
| | | RU 2711640 C2 | 17 January 2020 |
| | | EP 3129063 A1 | 15 February 2017 |
| | | EP 3129063 B1 | 27 January 2021 |
| | | IL 293177 A | 01 July 2022 |
| | | IL 293177 B1 | 01 March 2023 |
| | | IL 293177 B2 | 01 July 2023 |
| | | DK 3129063 T3 | 06 April 2021 |
| | | PL 3129063 T3 | 12 July 2021 |
| | | EP 3789042 A1 | 10 March 2021 |
| | | JP 2020143114 A | 10 September 2020 |
| | | JP 6918182 B2 | 11 August 2021 |
| | | KR 20160144396 A | 16 December 2016 |
| | | KR 101937549 B1 | 10 January 2019 |
| | | SG 11201608309 PA | 29 November 2016 |
| | | KR 20220012402 A | 03 February 2022 |
| | | KR 102399277 B1 | 18 May 2022 |
| | | AU 2021286320 A1 | 20 January 2022 |
| | | HUE 054411 T2 | 28 September 2021 |
| | | AU 2015245122 A1 | 18 August 2016 |
| | | AU 2015245122 B2 | 24 October 2019 |
| | | TW 202218685 A | 16 May 2022 |
| | | MX 2021004884 A | 15 June 2021 |
| | | PH 12016501711 A1 | 19 December 2016 |
| | | WO 2015155998 A1 | 15 October 2015 |
| | | KR 20220132025 A | 29 September 2022 |
| | | KR 102495426 B1 | 06 February 2023 |
| | | JP 2017222638 A | 21 December 2017 |
| | | JP 6513128 B2 | 15 May 2019 |
| | | MY 195180 A | 11 January 2023 |
| | | PT 3129063 T | 01 April 2021 |
| WO 2022078425 A1 | 21 April 2022 | CA 3196940 A1 | 21 April 2022 |
| | | JP 2023545925 A | 01 November 2023 |
| | | AU 2021360625 A1 | 01 June 2023 |
| | | KR 20230086702 A | 15 June 2023 |
| | | TW 202304983 A | 01 February 2023 |
| | | EP 4230653 A1 | 23 August 2023 |
| WO 2012019024 A2 | 09 February 2012 | WO 2012019024 A3 | 03 May 2012 |
| WO 2019241893 A2 | 26 December 2019 | JP 2021534811 A | 16 December 2021 |
| | | US 2021246223 A1 | 12 August 2021 |
| | | WO 2019241893 A3 | 13 February 2020 |
| CN 104093743 A | 08 October 2014 | IL 232726 A0 | 31 July 2014 |
| | | IL 232726 B | 01 November 2022 |
| | | IL 232726 B2 | 01 March 2023 |
| | | CO 7051008 A2 | 10 September 2014 |
| | | JP 2015506912 A | 05 March 2015 |
| | | JP 6180425 B2 | 23 August 2017 |
| | | NZ 625046 A | 30 September 2016 |
| | | RU 2014120757 A | 27 December 2015 |
| | | RU 2620068 C2 | 22 May 2017 |
| | | ES 2745684 T3 | 03 March 2020 |

Form PCT/ISA/210 (patent family annex) (July 2022)

**INTERNATIONAL SEARCH REPORT**
**Information on patent family members**

International application No.
**PCT/CN2023/125269**

| Patent document cited in search report | Publication date (day/month/year) | Patent family member(s) | | | Publication date (day/month/year) |
|---|---|---|---|---|---|
| | | CA | 2856297 | A1 | 30 May 2013 |
| | | CA | 2856297 | C | 17 October 2023 |
| | | US | 2016159913 | A1 | 09 June 2016 |
| | | US | 10040857 | B2 | 07 August 2018 |
| | | DK | 2797957 | T3 | 23 September 2019 |
| | | WO | 2013078191 | A1 | 30 May 2013 |
| | | SG | 11201402536 | PA | 27 June 2014 |
| | | BR | 112014012539 | A2 | 14 February 2018 |
| | | BR | 112014012539 | B1 | 20 December 2022 |
| | | ZA | 201403570 | B | 21 December 2022 |
| | | US | 2014363429 | A1 | 11 December 2014 |
| | | US | 9220775 | B2 | 29 December 2015 |
| | | HK | 1203207 | A1 | 23 October 2015 |
| | | KR | 20140113912 | A | 25 September 2014 |
| | | KR | 102080535 | B1 | 24 February 2020 |
| | | MX | 2014006324 | A | 22 September 2014 |
| | | MX | 350957 | B | 27 September 2017 |
| | | US | 2019040143 | A1 | 07 February 2019 |
| | | US | 11091554 | B2 | 17 August 2021 |
| | | AU | 2012340766 | A1 | 12 June 2014 |
| | | AU | 2012340766 | B2 | 10 May 2018 |
| | | EP | 2797957 | A1 | 05 November 2014 |
| | | EP | 2797957 | A4 | 01 July 2015 |
| | | EP | 2797957 | B1 | 19 June 2019 |
| | | EP | 3608340 | A1 | 12 February 2020 |
| | | JP | 2017149720 | A | 31 August 2017 |
| | | JP | 6513728 | B2 | 15 May 2019 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 13948732 B
- WO 9007861 A
- US 5244903 A
- US 2016297890 A
- US 20190151328 A1


### Non-patent literature cited in the description


- **NICOLAS JOUBERT et al.** Antibody-Drug Conjugates: The Last Decade. *Pharmaceuticals*, 14 September 2020, vol. 13 (9), 245
- **YUSUKE OGITANI et al.** *Clin Cancer Res*, 2016, vol. 22 (20), 5097-5108
- *Clin Cancer Res*, 15 March 2014, vol. 20 (6)
- **CREIGHTON**. *Proteins*, 1984
- **ALTSCHUL et al.** *Nuc. Acids Res.*, 1997, vol. 25, 3389-3402
- **ALTSCHUL et al.** *J. Mol. Biol.*, 1990, vol. 215, 403-410
- **KOHLER** ; **MILSTEIN**. *Nature*, 1975, vol. 256, 495-497
- Monoclonal Antibodies. Plenum Press, 1980, 365-367
- **GODING J.W.** Monoclonal Antibodies: Principles and Practice. Academic Press, 1986
- *Proc. Natl. Acad. Sci. U.S.A.*, 1984, vol. 81, 6851-6855
- *Nature*, 1986, vol. 321, 522-525
- **TOMIZUKA, K. et al.** *Nature Genetics*, 1997, vol. 16, 133-143
- **KUROIWA, Y. et al.** *Nucl. Acids Res.*, 1998, vol. 26, 3447-3448
- **YOSHIDA, H. et al.** Animal Cell Technology: Basic and Applied Aspects. Kluwer Academic Publishers, 1999, vol. 10, 69-73
- Proc. Natl. Acad. Sci. USA. 2000, vol. 97, 722-727
- **WINTER** ; **MILSTEIN**. *Nature*, 1991, vol. 349, 293-299
- **RADER et al.** *Proc. Nat. Acad. Sci. U.S.A.*, 1998, vol. 95, 8910-8915
- **STEINBERGER et al.** *J. Biol. Chem.*, 2000, vol. 275, 36073-36078
- **QUEEN et al.** *Proc. Natl. Acad. Sci. U.S.A.*, 1989, vol. 86, 10029-10033
- **KYOJI TSUCHIKAMA et al.** Antibody-drug conjugates: recent advances in conjugation and linker chemistrie. *Protein Cell*, January 2018, vol. 9 (1), 33-46
- *Bioorg. Med. Chem. Lett.*, 2016, vol. 26, 1542-1545
- *Angew. Chem. Int. Ed.*, 2015, vol. 54, 7492-7509
- **HERMANSON, G. T**. Bioconjugate Techniques. Academic Press, 1996, 56-136, 456-493
- **ANSEL**. Introduction to Pharmaceutical Dosage Forms. 1999
- *Protein Science*, 1995, vol. 4, 2411-2423